# EUROPEAN PATENT APPLICATION

(11) **EP 1 505 072 A2**
(43) Date of publication of application: **09.02.2005**
(21) Application number: 04020121.2
(22) Date of filing: 04.12.2000
(51) Int. Cl.: C07J 71/00, A61K 31/58

(54) **Eplerenone crystalline form exhiniting enhanced dissolution rate**

(30) Priority: 08.12.1999 US 169682 P; 08.12.1999 US 169608 P; 08.12.1999 US 169556 P; 08.12.1999 US 169690 P
(62) Divisional of application: 00983781.6
(71) Applicant: Pharmacia Corporation, Chicago, IL 60680 (US)
(72) Inventor: The designation of the inventor has not yet been filed
(74) Representative: Bannerman, David Gardner

(57) **Abstract**

A novel crystalline form (Form H) of the aldosterone receptor antagonist drug eplerenone is provided having a relatively rapid dissolution rate in aqueous media. Also provided are novel solvated crystalline forms of eplerenone that, when desolvated, can yield Form H eplerenone. Also provided is amorphous eplerenone. Pharmaceutical compositions are provided comprising Form H eplerenone, optionally accompanied by one or more other solid state forms of eplerenone, in a total unit dosage amount of eplerenone of about 10 to about 1000 mg, and further comprising one or more pharmaceutically acceptable excipients. Processes are provided for preparing Form H eplerenone and for preparing compositions comprising Form H eplerenone. A method for prophylaxis and/or treatment of an aldosterone-mediated condition or disorder is also provided, comprising administering to a subject a therapeutically effective amount of eplerenone, wherein at least a fraction of the eplerenone present is Form H eplerenone.

## Description

### FIELD OF THE INVENTION

This invention is in the field of pharmaceutical agents active as aldosterone receptor antagonists, more particularly to the aldosterone receptor antagonist eplerenone. Specifically, the invention relates to a novel crystalline form of eplerenone, to methods of preparing this crystalline form, to pharmaceutical compositions comprising this crystalline form, to methods for treatment and/or prophylaxis of aldosterone-mediated conditions and/or disorders, including conditions and disorders associated with hyperaldosteronism such as hypertension, using this crystalline form, and to use of this crystalline form in manufacture of medicaments.

### BACKGROUND OF THE INVENTION

The compound methyl hydrogen 9,11-epoxy-17-hydroxy-3-oxopregn-4-ene-7,21-dicarboxylate, γ-lactone having the structure (I) and known as eplerenone was first reported in U.S. Patent No. 4,559,332 to Grob *et al*., which discloses a class of 9,11-epoxy steroid compounds and their salts. Eplerenone is an aldosterone receptor antagonist and can be administered in a therapeutically effective amount where use of an aldosterone receptor antagonist is indicated, such as in treatment of pathological conditions associated with hyperaldosteronism including hypertension, heart failure including cardiac insufficiency, and cirrhosis of the liver.

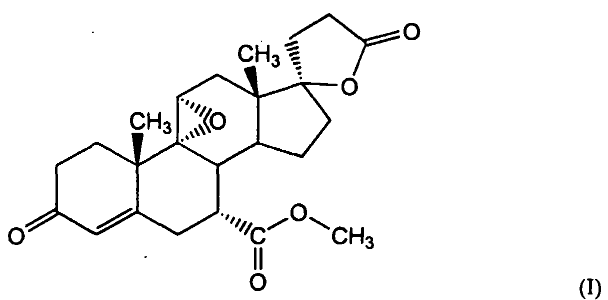

Above-cited U.S. Patent No. 4,559,332, which is incorporated herein by reference, generally discloses preparation of eplerenone and preparation of pharmaceutical compositions comprising eplerenone. Additional processes for the preparation of 9,11-epoxy steroid compounds and their salts, including eplerenone, are disclosed in International Patent Publications No. WO 97/21720 and No. WO 98/25948.

Grob *et al*. (1997), "Steroidal aldosterone antagonists: increased selectivity of 9α,11-epoxy derivatives", Helvetica Chimica Acta, 80, 566-585, discloses an X-ray crystal structure analysis of a solvate of eplerenone prepared by crystallizing eplerenone from a methylene chloride/diethyl ether solvent system.

De Gasparo *et al*. (1989), "Antialdosterones: incidence and prevention of sexual side effects", Journal of Steroid Biochemistry, 32(13), 223-227, discloses use of non-formulated eplerenone having a 20 µm particle size in a single dose study of eplerenone.

Spironolactone a 20-spiroxane-steroid of structure (II) having activity as an aldosterone receptor antagonist, is commercially available for treatment of hypertension. Spironolactone, however, has antiandrogenic activity that can result in gynecomastia and impotence in men. It also has weak progestational activity that can produce menstrual irregularities in women. Accordingly, there is interest in development of additional active aldosterone receptor antagonists such as eplerenone that do not interact with other steroid receptor systems such as glucocorticoid, progestin and androgen steroid receptor systems and/or that provide for a broader range of treatment.

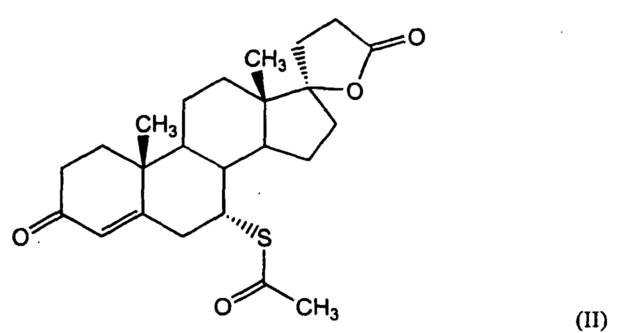

Agafonov *et al*. (1991), "Polymorphism of spironolactone", Journal of Pharmaceutical Sciences, 80(2), 181-185, discloses an acetonitrile solvate, an ethanol solvate, an ethyl acetate solvate, a methanol solvate and two non-solvated polymorphic crystalline forms of spironolactone. Brittan (1999), Polymorphism in Pharmaceutical Solids, pp. 114-116, 207, 235 and 261 (Marcel Dekker), likewise discloses these solid state forms of spironolactone.

Eplerenone has very low solubility in aqueous media and release of the drug in the gastrointestinal tract from oral dosage forms is often a limiting factor to bioavailability of the drug, and more particularly to speed of onset of therapeutic effect following oral administration.

### SUMMARY OF THE INVENTION

There is now provided a novel crystalline form of eplerenone having a relatively rapid dissolution rate in aqueous media and having other unique properties relative to other solid state forms of eplerenone. This crystalline form is fully characterized hereinbelow but is referred to for convenience as "Form H".

The invention provides, in a first aspect, this novel crystalline Form H of eplerenone *per se.* Among the properties distinguishing Form H from another crystalline form, referred to as "Form L", Form H exhibits an orthorhombic crystal system, an X-ray powder diffraction pattern with a peak at 12.0 ± 0.2 degrees 2θ and a melting point in a range from about 247°C to about 251°C.

In a second aspect, the invention provides an eplerenone drug substance comprising Form H eplerenone in at least a detectable amount.

In a third aspect, the invention provides an eplerenone drug substance that is substantially phase pure Form H eplerenone. The term "phase pure" herein refers to purity with respect to other solid state forms of eplerenone and does not necessarily imply a high degree of chemical purity with respect to other compounds.

In a fourth aspect, the invention provides solvated crystalline forms of eplerenone that, when desolvated, can yield Form H eplerenone.

In a fifth aspect, the invention provides pharmaceutical compositions comprising Form H eplerenone, optionally accompanied by one or more other solid state forms of eplerenone, in a total unit dosage amount of eplerenone of about 10 to about 1000 mg, and further comprising one or more pharmaceutically acceptable excipients.

In a sixth aspect, the invention provides processes for preparing Form H eplerenone and for preparing compositions comprising Form H eplerenone.

In a seventh aspect, the invention provides a method for prophylaxis and/or treatment of an aldosterone-mediated condition or disorder comprising administering to a subject a therapeutically effective amount of eplerenone, wherein at least a fraction of the eplerenone present is Form H eplerenone.

Additional aspects of the invention are discussed throughout the specification of this application.

### BRIEF DESCRIPTION OF THE DRAWINGS

Fig. 1-A shows X-ray powder diffraction patterns of Form H eplerenone.
Fig. 1-B shows X-ray powder diffraction patterns of Form L eplerenone.
Fig. 1-C shows X-ray powder diffraction patterns of the methyl ethyl ketone solvate of eplerenone.
Figs. 1-D through 1-0 show X-ray powder diffraction patterns of the following eplerenone solvates: n-propyl alcohol, tetrahydrofuran, ethyl propionate, acetic acid, acetone, toluene, isopropanol, ethanol, isobutyl acetate, butyl acetate, methyl acetate and propyl acetate solvates respectively.
Fig. 2-A shows a differential scanning calorimetry (DSC) thermogram of non-milled Form L eplerenone directly crystallized from methyl ethyl ketone.
Fig. 2-B shows a DSC thermogram of non-milled Form L eplerenone prepared by desolvation of a solvate obtained by crystallization of a high purity eplerenone from methyl ethyl ketone.
Fig. 2-C shows a DSC thermogram of Form L eplerenone prepared by milling the product of desolvation of a solvate obtained by crystallization of a high purity eplerenone from methyl ethyl ketone.
Fig. 2-D shows a DSC thermogram of non-milled Form H eplerenone prepared by desolvation of a solvate obtained by digestion of low purity eplerenone from appropriate solvents.
Figs. 2-E through 2-T show DSC thermograms for the following eplerenone solvates: n-propyl alcohol, tetrahydrofuran, ethyl propionate, acetic acid, chloroform, acetone, toluene, isopropanol, t-butyl acetate, ethanol, isobutyl acetate, butyl acetate, methyl acetate, propyl acetate, n-butanol and n-octanol solvates respectively.
Fig. 3-A shows infrared (IR) spectra (diffuse reflectance, DRIFT) of Form H eplerenone.
Fig. 3-B shows IR spectra (diffuse reflectance, DRIFT) of Form L eplerenone.
Fig. 3-C shows IR spectra (diffuse reflectance, DRIFT) of the methyl ethyl ketone solvate of eplerenone.
Fig. 3-D shows IR spectra (diffuse reflectance, DRIFT) of eplerenone in chloroform solution.
Figs. 3-E through 3-Q show IR spectra for the following eplerenone solvates: n-propyl alcohol, tetrahydrofuran, ethyl propionate, acetic acid, acetone, toluene, isopropanol, ethanol, isobutyl acetate, butyl acetate, propyl acetate, methyl acetate, propylene glycol and t-butyl acetate solvates respectively.
Fig. 4 shows ¹³C NMR spectra of Form H eplerenone.
Fig. 5 shows ¹³C NMR spectra of Form L eplerenone.
Figs. 6-A through 6-R show thermogravimetric analysis profiles of the following eplerenone solvates: methyl ethyl ketone, n-propyl alcohol, tetrahydrofuran, ethyl propionate, acetic acid, chloroform, acetone, toluene, isopropanol, ethanol, isobutyl acetate, butyl acetate, methyl acetate, propyl acetate, propylene glycol, n-butanol, n-octanol and t-butyl acetate solvates respectively.
Fig. 7 is a scanning electron micrograph of Form L eplerenone prepared by desolvation of the methyl ethyl ketone solvate of eplerenone.
Fig. 8 is a scanning electron micrograph of Form L eplerenone prepared by direct crystallization from ethyl acetate.
Fig. 9 shows an X-ray powder diffraction pattern of a crystalline form of 7-methyl hydrogen 4α,5α;9α,11α-diepoxy-17 hydroxy-3-oxo-17α-pregnane-7α,21-dicarboxylate, γ-lactone (the "diepoxide") isolated from methyl ethyl ketone.
Fig. 10 shows an X-ray powder diffraction pattern of a crystalline form of 7-methyl hydrogen 11α,12α-epoxy-17-hydroxy-3-oxo-17α-pregn-4-ene-7α,21-dicarboxylate, γ-lactone (the "11,12-epoxide") isolated from isopropanol.
Fig. 11 shows an X-ray powder diffraction pattern of a crystalline form of 7-methyl hydrogen 17-hydroxy-3-oxo-17α-pregna-4,9(11)-diene-7α,21-dicarboxylate, γ-lactone (the "9,11-olefin") isolated from n-butanol.
Fig. 12 illustrates the relationship between Gibbs free energy and temperature for enantiotropically related polymorphs.
Fig. 13 shows X-ray powder diffraction patterns of methyl ethyl ketone solvate wet cake obtained from (a) 0%, (b) 1%, (c) 3% and (d) 5% diepoxide-doped methyl ethyl ketone crystallizations.
Fig. 14 shows X-ray powder diffraction patterns for dried solids obtained from (a) 0%, (b) 1%, (c) 3% and (d) 5% diepoxide-doped methyl ethyl ketone crystallizations.
Fig. 15 shows X-ray powder diffraction patterns for dried solids from a methyl ethyl ketone crystallization with 3% doping of diepoxide, (a) without and (b) with grinding of the solvate prior to drying.
Fig. 16 shows X-ray powder diffraction patterns of methyl ethyl ketone solvate wet cake obtained from (a) 0%, (b) 1%, (c) 5% and (d) 10% 11,12-epoxide-doped methyl ethyl ketone crystallizations.
Fig. 17 shows X-ray powder diffraction patterns of dried solids obtained from
(a) 0%, (b) 1%, (c) 5% and (d) 10% 11,12-epoxide-doped methyl ethyl ketone crystallizations.
Fig. 18 shows a cube plot of product purity, starting material purity, cooling rate and end-point temperature based on data reported in Table 7A of Example 7 herein.
Fig. 19 shows a half-normal plot prepared using the cube plot of Fig. 18 to determine those variables having a statistically significant effect on purity of final material.
Fig. 20 is an interaction graph based on data reported in Table 7A of Example 7 herein, showing an interaction between starting material purity and cooling rate in effect on purity of final material.
Fig. 21 shows a cube plot of Form H weight fraction, starting material purity, cooling rate and end-point temperature based on data reported in Table 7A of Example 7 herein.
Fig. 22 shows a half-normal plot prepared using the cube plot of Fig. 21 to determine those variables having a statistically significant effect on purity of final material.
Fig. 23 is an interaction graph based on data reported in Table 7A of Example 7 herein, showing an interaction between starting material purity and end-point temperature in effect on purity of final material.
Fig. 24 shows an X-ray diffraction pattern of amorphous eplerenone.
Fig. 25 shows a DSC thermogram of amorphous eplerenone.
Fig. 26 shows dissolution rates measured for four eplerenone polymorph samples.

### DETAILED DESCRIPTION OF THE INVENTION

As with all pharmaceutical compounds and compositions, chemical and physical properties of eplerenone are important in its commercial development. These properties include, but are not limited to: (1) packing properties such as molar volume, density and hygroscopicity, (2) thermodynamic properties such as melting temperature, vapor pressure and solubility, (3) kinetic properties such as dissolution rate and stability (including stability at ambient conditions, especially to moisture, and under storage conditions), (4) surface properties such as surface area, wettability, interfacial tension and shape, (5) mechanical properties such as hardness, tensile strength, compactibility, handling, flow and blend; and (6) filtration properties. These properties can affect, for example, processing and storage of pharmaceutical compositions comprising eplerenone. Solid state forms of eplerenone that provide an improvement in one or more of these properties relative to other solid state forms of eplerenone are desirable.

According to the present invention, novel solid state forms of eplerenone are provided. Specifically, these include various solvated crystalline forms, at least two non-solvated and non-hydrated crystalline forms (designated "Form H" and "Form L"), and an amorphous form of eplerenone. Each solid state form of eplerenone described in the present application possesses one or more of the above-described advantageous chemical and/or physical properties relative to other solid state forms described herein or otherwise disclosed in the literature. Form H and Form L are referred to in priority documents claimed herein as "Form I" and "Form II" respectively, and are sometimes described as the "high melting point polymorph" and the "low melting point polymorph" respectively.

The present invention relates to Form H eplerenone. Form H exhibits a faster dissolution rate (approximately 30% faster) in an aqueous medium than, for example, Form L eplerenone at temperatures below the enantiotropic transition temperature (as discussed hereinbelow). Where dissolution of eplerenone in the gastrointestinal tract is the rate-controlling step for delivery of the eplerenone to target cells or tissues, faster dissolution generally results in improved bioavailability. Form H, therefore, can provide an improved bioavailability profile relative to Form L. In addition, selection of a solid state form of eplerenone having a faster dissolution rate likewise provides greater flexibility in selection of excipients for, and in formulation of, pharmaceutical compositions, particularly those intended to exhibit immediate release of eplerenone, relative to other solid state forms having a slower dissolution rate.

Form L eplerenone also has advantages over other solid state forms. In particular, it possesses greater physical stability at temperatures below the enantiotropic transition temperature (as discussed hereinbelow) than, for example, Form H. Solid state forms of eplerenone such as Form L that do not require special processing or storage conditions, and that avoid need for frequent inventory replacement, are desirable. For example, selection of a solid state form of eplerenone that is physically stable during a manufacturing process (such as during milling of eplerenone to obtain a material with reduced particle size and increased surface area) can avoid need for special processing conditions and the increased costs generally associated with such special processing conditions. Similarly, selection of a solid state form of eplerenone that is physically stable over a wide range of storage conditions (especially considering the different possible storage conditions that can occur during the lifetime of an eplerenone product) can help avoid polymorphic or other degradative changes in the eplerenone that can lead to product loss or deterioration of product efficacy. Therefore, the selection of a solid state form of eplerenone such as Form L having greater physical stability provides a meaningful benefit over less stable eplerenone forms.

The invention also relates to solvated crystalline forms of eplerenone. These solvated forms are useful as intermediates in preparation of Form H and Form L eplerenone; of particular interest in the context of the present invention are solvated crystalline forms of eplerenone that, when desolvated, can yield Form H eplerenone. A particular benefit from use of a solvated crystalline form as an intermediate is "intrinsic micronizing" of the crystal that results upon desolvation as discussed later in this application. Such "intrinsic micronizing" can reduce or eliminate milling requirements. Further, where additional milling is still required, it is easier to mill certain solvates before the desolvation step than to mill Form H or Form L after desolvation of the solvated crystalline form.

Pharmaceutically acceptable solvated crystalline forms of eplerenone also can be used directly in pharmaceutical compositions. In one embodiment, solvated crystalline forms useful in directly preparing such compositions do not comprise methylene chloride, isopropanol or ethyl ether; in another embodiment, do not comprise methylene chloride, isopropanol, ethyl ether, methyl ethyl ketone or ethanol; and, in yet another embodiment, do not comprise methylene chloride, isopropanol, ethyl ether, methyl ethyl ketone, ethanol, ethyl acetate or acetone. Most preferably for this use, the solvated crystalline forms of eplerenone are substantially exclusive of solvents that are not pharmaceutically acceptable solvents.

Solvated crystalline forms used in pharmaceutical compositions generally and preferably comprise a pharmaceutically acceptable higher boiling point and/or hydrogen-bonding solvent such as, but not limited to, butanol. It is believed that the solvated crystalline forms collectively can offer a range of different dissolution rates and, where dissolution of eplerenone in the gastrointestinal tract is the rate-controlling step for delivery of the eplerenone to the target cells or tissues, a range of different bioavailabilities relative to Form H and Form L.

The invention also relates to an amorphous form of eplerenone. Amorphous eplerenone is useful as an intermediate in the preparation of Form H and Form L eplerenone. In addition, it is believed that amorphous eplerenone possesses a different dissolution rate and, where amorphous eplerenone is present in a pharmaceutical composition and where dissolution of eplerenone in the gastrointestinal tract is the rate-controlling step for delivery of the eplerenone to the target cells, such amorphous eplerenone can provide different bioavailability relative to Form H and Form L.

Also of interest are combinations of solid state forms selected from the group consisting of Form H eplerenone, Form L eplerenone, solvated crystalline forms of eplerenone and amorphous eplerenone. Such combinations are useful, for example, in preparation of pharmaceutical compositions having a variety of dissolution profiles, including controlled-release compositions. In an embodiment of the present invention, a combination of solid state forms is provided comprising Form H eplerenone in at least a detectable amount, with the balance being one or more solid state forms selected from the group consisting of Form L eplerenone, solvated crystalline forms of eplerenone and amorphous eplerenone.

Depending upon the intended use of the solid state form of eplerenone, processing considerations may favor selection of a specific solid state form or a specific combination of such solid state forms. Phase pure Form L, for example, generally is more easily prepared than phase pure Form H. A mixture of Form H and Form L, however, generally is more easily prepared than phase pure Form L and allows for the use of an eplerenone starting material of relatively low chemical purity. Use of a solvated crystalline form instead of Form H or Form L in a composition eliminates a processing step, namely desolvation, for those processes that otherwise would proceed by desolvation of a solvated crystalline form. Alternatively, the desolvation step can be eliminated, for example, if Form L is directly crystallized from an appropriate solvent without intervening preparation and desolvation of an intermediate solvated crystalline form. Such processes are described in greater detail hereinbelow.

### Definitions

The term "amorphous" as applied to eplerenone herein refers to a solid state wherein the eplerenone molecules are present in a disordered arrangement and do not form a distinguishable crystal lattice or unit cell. When subjected to X-ray powder diffraction, amorphous eplerenone does not produce any characteristic crystalline peaks.

Where reference is made herein to the boiling point of a substance or solution, the term "boiling point" means the boiling point of the substance or solution under the applicable process conditions.

The term "crystalline form" as applied to eplerenone herein refers to a solid state form wherein the eplerenone molecules are arranged to form a distinguishable crystal lattice (i) comprising distinguishable unit cells, and (ii) yielding diffraction peaks when subjected to X-ray radiation.

The term "crystallization" as used herein can refer to crystallization and/or recrystallization depending upon the applicable circumstances relating to preparation of eplerenone starting material.

The term "digestion" herein means a process in which a slurry of solid eplerenone in a solvent or mixture of solvents is heated at the boiling point of the solvent or mixture of solvents under the applicable process conditions.

The term "direct crystallization" as used herein refers to crystallization of eplerenone directly from a suitable solvent without formation and desolvation of an intermediate solvated crystalline solid state form of eplerenone.

The term "eplerenone drug substance" as used herein means eplerenone *per se* as qualified by the context in which the term is used, and can refer to unformulated eplerenone or to eplerenone present as an ingredient of a pharmaceutical composition.

The term "particle size" as used herein refers to particle size as measured by conventional particle size measuring techniques well known in the art, such as laser light scattering, sedimentation field flow fractionation, photon correlation spectroscopy or disk centrifugation. The "D₉₀ particle size" is a particle size such that 90% by weight of the particles are smaller than the D₉₀ particle size as measured by such conventional particle size measuring techniques.

The term "DSC" means differential scanning calorimetry.

The term "HPLC" means high pressure liquid chromatography.

The term "IR" means infrared.

The term "purity" herein, unless otherwise qualified, means the chemical purity of eplerenone according to conventional HPLC assay. As used herein, "low purity eplerenone" generally means eplerenone that contains an effective amount of a Form H crystal growth promoter and/or a Form L crystal growth inhibitor. As used herein, "high purity eplerenone" generally means eplerenone that does not contain, or contains less than an effective amount of, a Form H crystal growth promoter and/or a Form L crystal growth inhibitor.

The term "phase purity" herein means the solid state purity of eplerenone with regard to a particular crystalline or amorphous form of the eplerenone as determined by the infrared spectroscopy analytical methods described herein.

The term "XRPD" means X-ray powder diffraction.

The term "rpm" means revolutions per minute.

The term "TGA" means thermogravimetric analysis.

The term "Tₘ" means melting temperature.

### Characterization of crystalline form

### 1. Molecular conformation

Single crystal X-ray analysis indicates that the molecular conformation of eplerenone differs between Form H and Form L, particularly with respect to orientation of the ester group at the 7-position of the steroid ring. The orientation of the ester group can be defined by the C8-C7-C23-O1 torsion angle.

In the Form H crystal lattice, the eplerenone molecule adopts a conformation in which the methoxy group of the ester is approximately aligned with the C-H bond at the 7-position and the carbonyl group is approximately positioned over the center of the B-steroid ring. The C8-C7-C23-O1 torsion angle is approximately -73.0° in this conformation. In this orientation, the carbonyl oxygen atom of the ester group (O1) is in close contact with the oxygen atom of the 9,11-epoxide ring (04). The O1-O4 distance is about 2.97 Å, which is just below the van der Waals contact distance of 3.0 Å (assuming van der Waals radii of 1.5Å for the oxygen atoms).

In the Form L crystal lattice, the eplerenone molecule adopts a conformation in which the ester group is rotated approximately 150° relative to that of Form H and has a C8-C7-C23-O1 torsion angle of approximately +76.9°. In this orientation, the methoxy group of the ester is directed toward the 4,5-alkene segment of the A-steroid ring. In this orientation, the distance between either oxygen atom of the ester group (01,02) and the oxygen atom of the 9,11-epoxide ring (04) is increased relative to the distance determined for Form H. The 02-04 distance is approximately 3.04 Å, falling just above the van der Waals contact distance. The O1-O4 distance is about 3.45 Å.

In the solvated crystalline forms analyzed by single crystal X-ray diffraction to date, the eplerenone molecule appears to adopt a conformation characteristic of Form L.

### 2. X-ray powder diffraction

The various crystalline forms of eplerenone were analyzed with either a Siemens D5000 powder diffractometer or an Inel Multipurpose diffractometer. For the Siemens D5000 powder diffractometer, the raw data were measured for 2θ (two theta) values from 2 to 50, with steps of 0.020 and step periods of two seconds. For the Inel Multipurpose diffractometer, samples were placed in an aluminum sample holder and raw data were collected for 30 minutes at all 2θ values simultaneously.

Tables 1A, 1B and 1C set out the significant parameters of the main peaks in terms of 2θ values and intensities for Form H (prepared by desolvation of an ethanol solvate obtained by digestion of low purity eplerenone), Form L (prepared by desolvation of a methyl ethyl ketone solvate obtained by recrystallization of high purity eplerenone), and methyl ethyl ketone solvate (prepared by room temperature slurry conversion of high purity eplerenone in methyl ethyl ketone) crystalline forms of eplerenone, respectively (X-ray radiation at a wavelength of 1.54056 Å).

Minor shifts in peak positioning may be present in the diffraction patterns of Form H and Form L as a result of imperfections in the spacing of the crystal diffraction planes associated with the route of manufacture of Form H and Form L (i.e., desolvation of a solvate). In addition, Form H is isolated from a solvate prepared by digestion of crude eplerenone. This method results in a lower overall chemical purity (approximately 90%) of the Form H. Finally, the solvated forms of eplerenone are expected to show some shifting in the positioning of the diffraction peaks due to increased mobility of solvent molecules within solvent channels in the crystal lattice.

**Table 1A:**

| **X-ray diffraction data, Form H** | | | |
|---|---|---|---|
| Angle 2θ | d-spacing Å | Intensity Cps | Intensity % |
| 6.994 | 12.628 | 1188 | 7.2 |
| 8.291 | 10.655 | 2137 | 13.0 |
| 10.012 | 8.827 | 577 | 3.5 |
| 11.264 | 7.849 | 1854 | 11.3 |
| 12.040 | 7.344 | 7707 | 46.8 |
| 14.115 | 6.269 | 3121 | 19.0 |
| 14.438 | 6.130 | 15935 | 96.8 |
| 15.524 | 5.703 | 637 | 3.9 |
| 16.169 | 5.477 | 1349 | 8.2 |
| 16.699 | 5.305 | 1663 | 10.1 |
| 16.940 | 5.230 | 1692 | 10.3 |
| 17.147 | 5.167 | 2139 | 13.0 |
| 17.660 | 5.018 | 6883 | 41.8 |
| 17.910 | 4.949 | 16455 | 100.0 |
| 18.379 | 4.823 | 3106 | 18.9 |
| 18.658 | 4.752 | 1216 | 7.4 |
| 19.799 | 4.480 | 1499 | 9.1 |
| 20.235 | 4.385 | 383 | 2.3 |
| 21.707 | 4.091 | 1267 | 7.7 |
| 21.800 | 4.073 | 1260 | 7.7 |
| 21.959 | 4.044 | 1279 | 7.8 |
| 22.461 | 3.955 | 4264 | 25.9 |
| 23.191 | 3.832 | 1026 | 6.2 |
| 23.879 | 3.723 | 1000 | 6.1 |
| 24.599 | 3.616 | 1688 | 10.3 |
| 25.837 | 3.445 | 931 | 5.7 |
| 26.034 | 3.420 | 686 | 4.2 |
| 26.868 | 3.316 | 912 | 5.5 |
| 27.093 | 3.288 | 1322 | 8.0 |
| 27.782 | 3.209 | 1236 | 7.5 |
| 28.340 | 3.147 | 1845 | 11.2 |
| 28.861 | 3.091 | 957 | 5.8 |
| 29.866 | 2.9892 | 745 | 4.5 |
| 30.627 | 2.9166 | 992 | 6.0 |
| 31.108 | 2.8726 | 1205 | 7.3 |
| 33.215 | 2.6951 | 1287 | 7.8 |
| 33.718 | 2.6560 | 802 | 4.9 |
| 34.434 | 2.6024 | 914 | 5.6 |

**Table 1B:**

| **X-ray diffraction data, Form L** | | | |
|---|---|---|---|
| Angle 2θ | d-spacing Å | Intensity Cps | Intensity % |
| 7.992 | 11.054 | 11596 | 26.6 |
| 10.044 | 8.799 | 12048 | 27.6 |
| 11.206 | 7.889 | 4929 | 11.3 |
| 12.441 | 7.109 | 1747 | 4.0 |
| 12.752 | 6.936 | 4340 | 9.9 |
| 13.257 | 6.673 | 2444 | 5.6 |
| 14.705 | 6.019 | 43646 | 100 |
| 15.460 | 5.727 | 2670 | 6.1 |
| 15.727 | 5.630 | 7982 | 18.3 |
| 16.016 | 5.529 | 3519 | 8.1 |
| 17.671 | 5.015 | 8897 | 20.4 |
| 17.900 | 4.951 | 2873 | 6.6 |
| 18.352 | 4.830 | 612 | 1.4 |
| 18.703 | 4.740 | 689 | 1.6 |
| 19.524 | 4.543 | 1126 | 2.6 |
| 20.103 | 4.413 | 3753 | 8.6 |
| 20.630 | 4.302 | 1451 | 3.3 |
| 21.067 | 4.214 | 876 | 2.0 |
| 21.675 | 4.097 | 2760 | 6.3 |
| 22.232 | 3.995 | 1951 | 4.5 |
| 22.652 | 3.922 | 1657 | 3.8 |
| 23.624 | 3.763 | 827 | 1.9 |
| 24.279 | 3.663 | 1242 | 2.8 |
| 25.021 | 3.556 | 5144 | 11.8 |
| 25.485 | 3.492 | 1702 | 3.9 |
| 25.707 | 3.463 | 2493 | 5.7 |
| 26.251 | 3.392 | 1371 | 3.1 |
| 26.850 | 3.318 | 1970 | 4.5 |
| 27.319 | 3.262 | 1029 | 2.4 |
| 27.931 | 3.192 | 440 | 1.0 |
| 27.969 | 3.187 | 440 | 1.0 |
| 28.937 | 3.083 | 1128 | 2.6 |
| 29.703 | 3.005 | 1211 | 2.8 |
| 30.173 | 2.9594 | 1506 | 3.5 |
| 30.584 | 2.9206 | 1602 | 3.7 |
| 30.885 | 2.8928 | 1550 | 3.6 |
| 31.217 | 2.8628 | 1068 | 2.4 |
| 31.605 | 2.8285 | 1038 | 2.4 |
| 32.059 | 2.7895 | 1211 | 2.8 |
| 32.640 | 2.7412 | 684 | 1.6 |
| 32.747 | 2.7324 | 758 | 1.7 |
| 33.460 | 2.6759 | 506 | 1.2 |
| 34.194 | 2.6201 | 1085 | 2.5 |
| 34.545 | 2.5943 | 915 | 2.1 |

**Table 1C:**

| **X-ray diffraction data, methyl ethyl ketone solvate** | | | |
|---|---|---|---|
| Angle 2θ | d-spacing Å | Intensity Cps | Intensity % |
| 7.584 | 11.648 | 5629 | 32.6 |
| 7.753 | 11.393 | 15929 | 92.3 |
| 10.151 | 8.707 | 2877 | 16.7 |
| 11.310 | 7.817 | 701 | 4.1 |
| 12.646 | 6.994 | 1027 | 5.9 |
| 13.193 | 6.705 | 15188 | 88.0 |
| 13.556 | 6.526 | 14225 | 82.4 |
| 14.074 | 6.287 | 1966 | 11.4 |
| 14.746 | 6.002 | 2759 | 16.0 |
| 15.165 | 5.837 | 801 | 4.6 |
| 15.548 | 5.694 | 1896 | 11.0 |
| 17.031 | 5.202 | 7980 | 46.2 |
| 17.280 | 5.127 | 17267 | 100.0 |
| 17.706 | 5.005 | 6873 | 39.8 |
| 18.555 | 4.778 | 545 | 3.2 |
| 18.871 | 4.699 | 1112 | 6.4 |
| 19.766 | 4.488 | 1704 | 9.9 |
| 20.158 | 4.401 | 1396 | 8.1 |
| 20.725 | 4.282 | 2644 | 15.3 |
| 21.787 | 4.076 | 1127 | 6.5 |
| 22.060 | 4.026 | 451 | 2.6 |
| 22.864 | 3.886 | 1542 | 8.9 |
| 23.412 | 3.796 | 14185 | 82.2 |
| 23.750 | 3.743 | 1154 | 6.7 |
| 24.288 | 3.662 | 3063 | 17.7 |
| 25.253 | 3.524 | 1318 | 7.6 |
| 25.503 | 3.490 | 1736 | 10.1 |
| 25.761 | 3.455 | 1225 | 7.1 |
| 26.176 | 3.402 | 1346 | 7.8 |
| 26.548 | 3.355 | 1098 | 6.4 |
| 27.357 | 3.257 | 1944 | 11.3 |
| 27.605 | 3.229 | 2116 | 12.3 |
| 27.900 | 3.195 | 858 | 5.0 |
| 28.378 | 3.142 | 583 | 3.4 |
| 28.749 | 3.103 | 763 | 4.4 |
| 29.300 | 3.046 | 1182 | 6.8 |
| 29.679 | 3.008 | 2606 | 15.1 |
| 30.402 | 2.9377 | 2184 | 12.6 |
| 30.739 | 2.9063 | 648 | 3.8 |

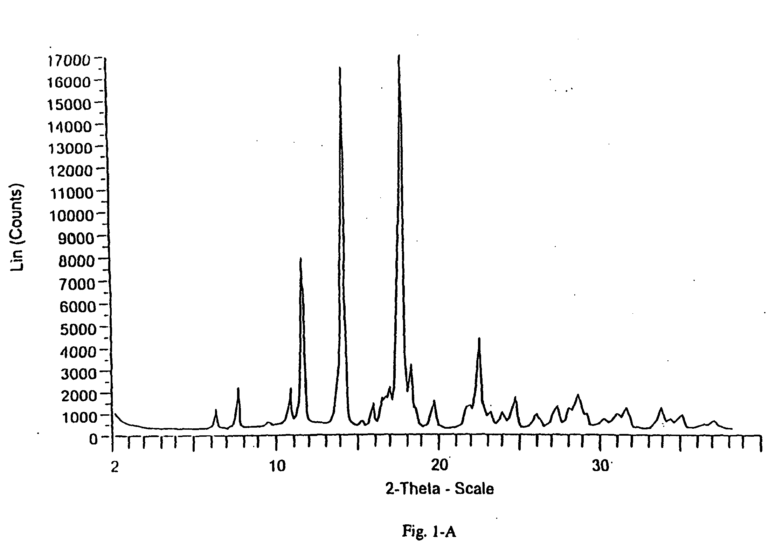

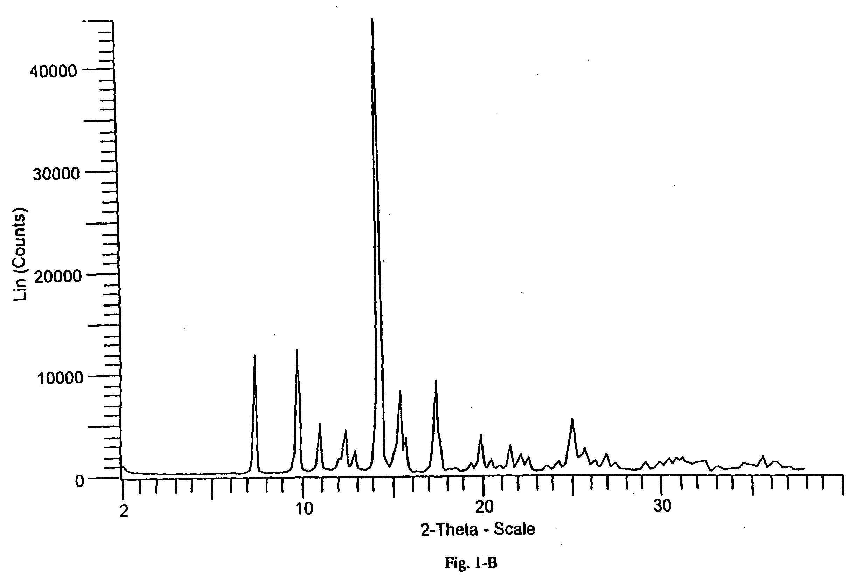

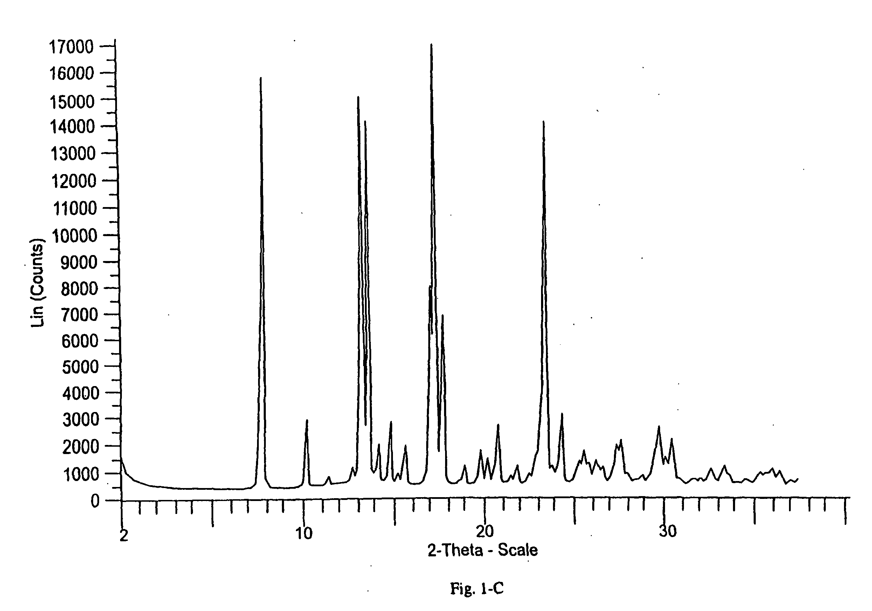

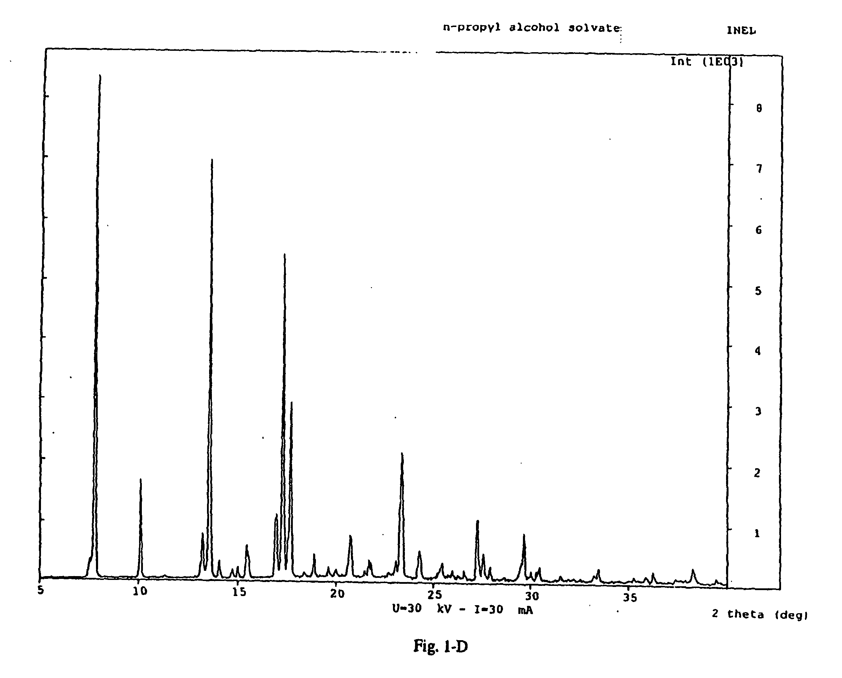

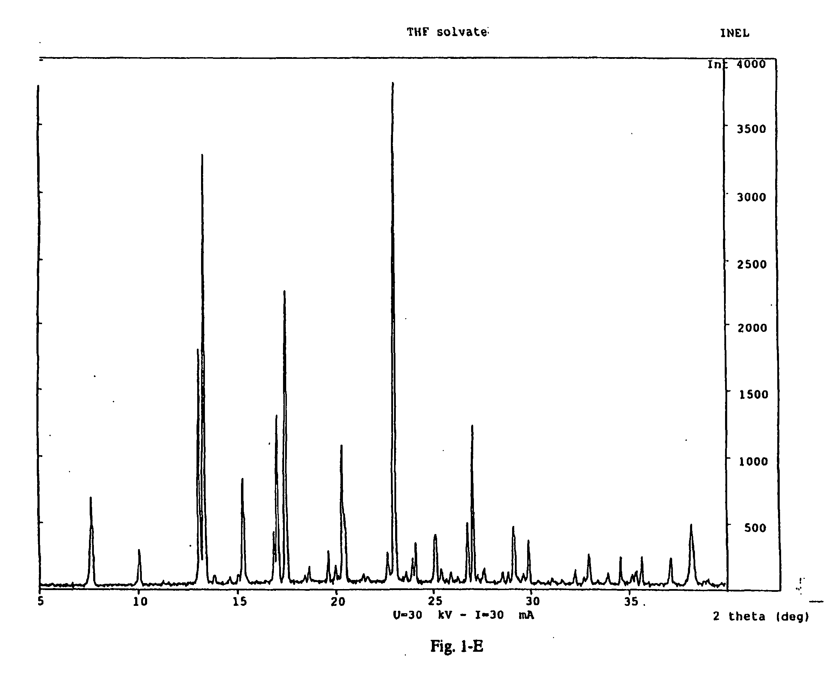

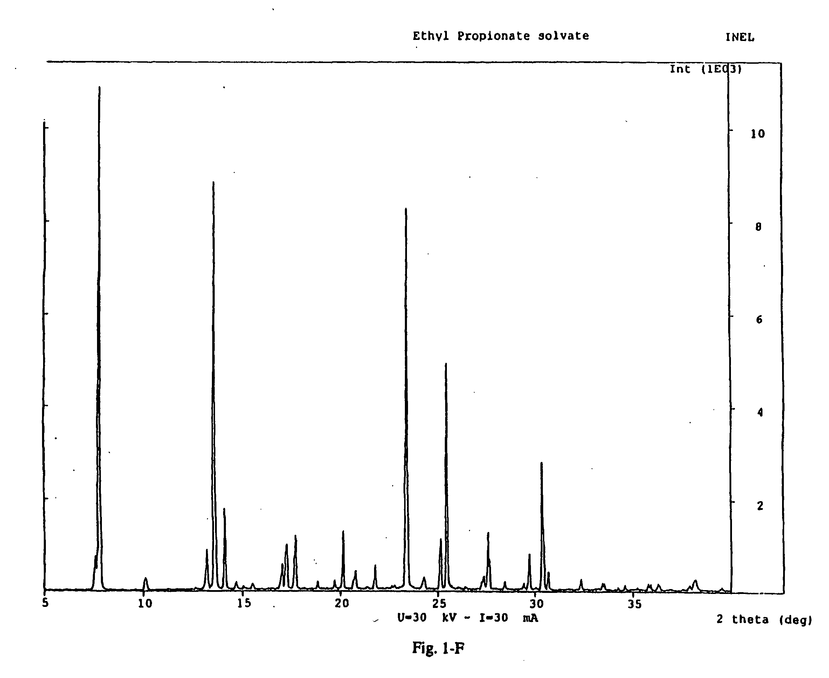

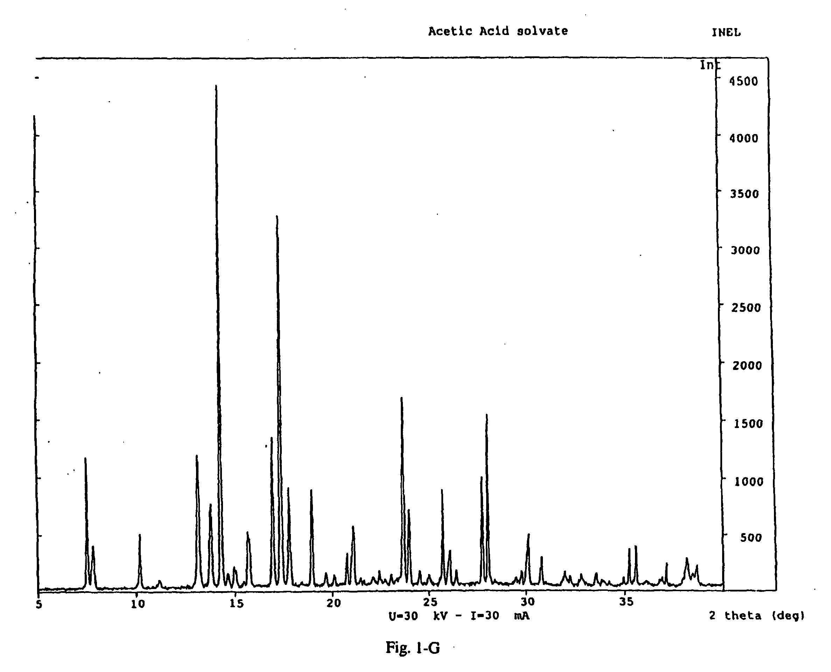

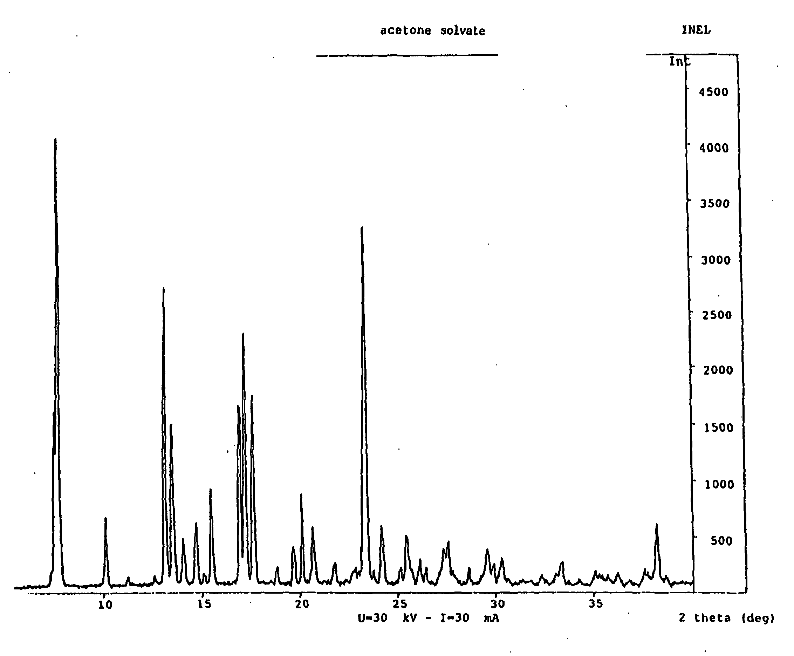

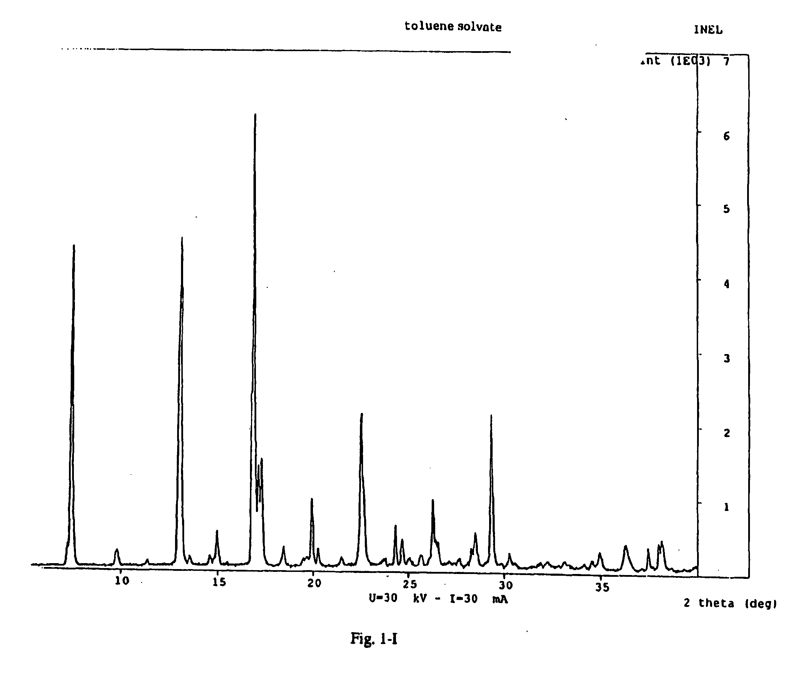

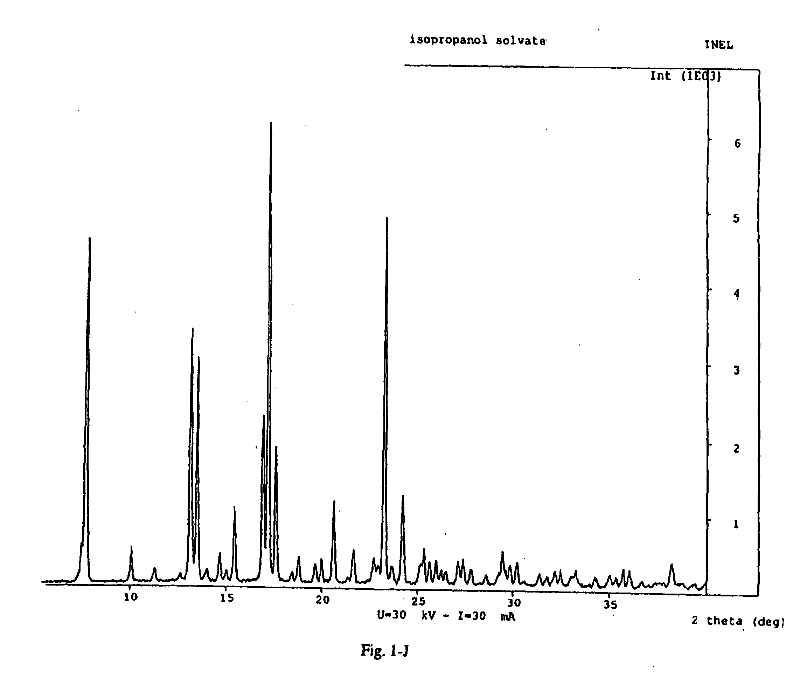

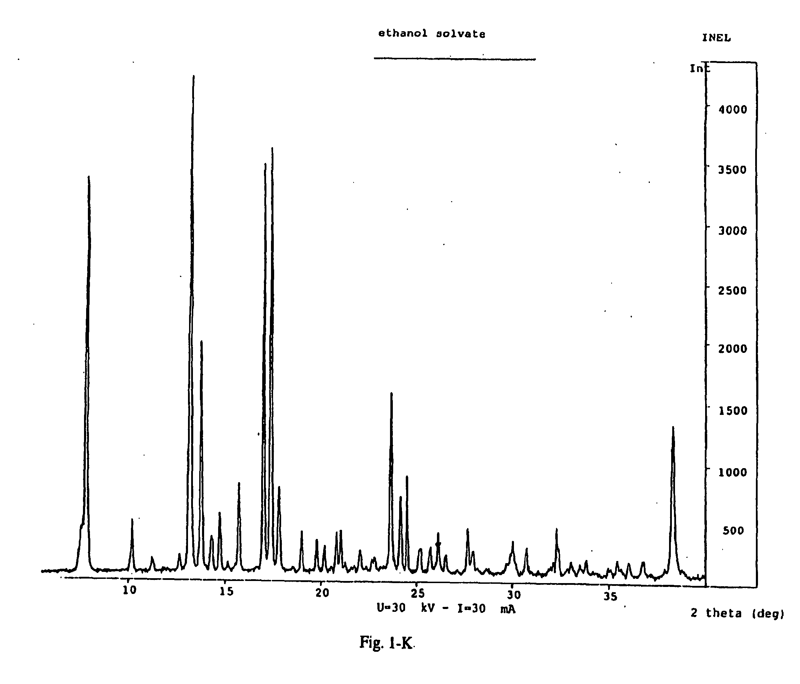

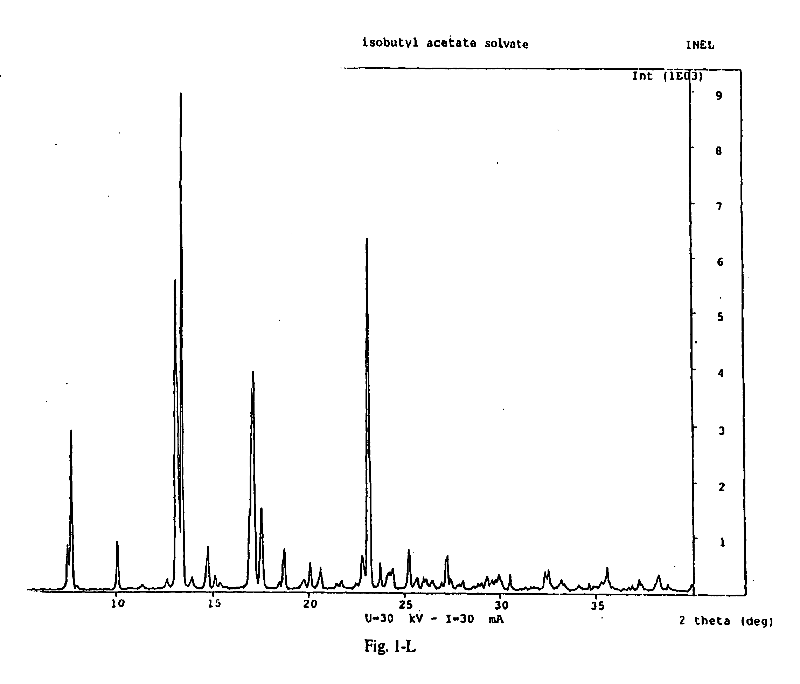

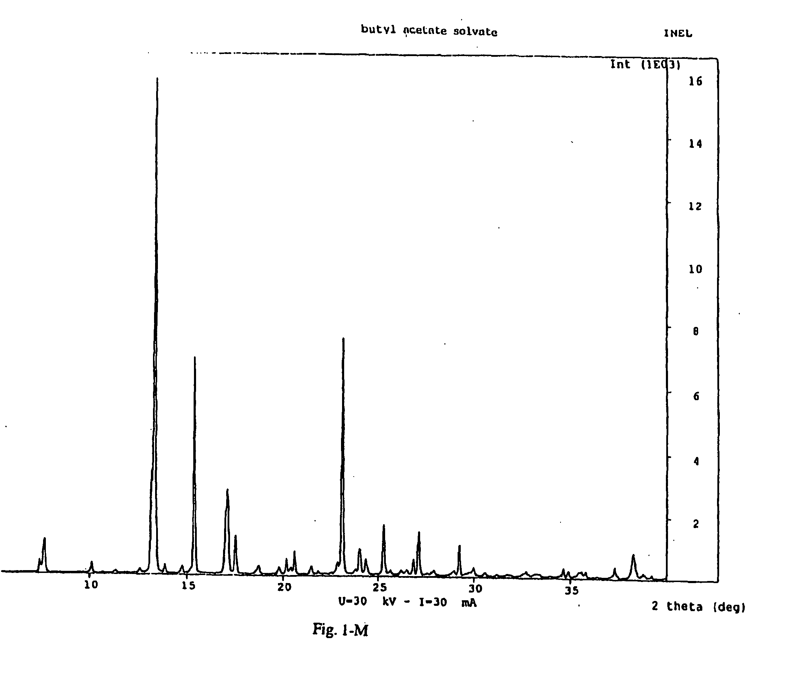

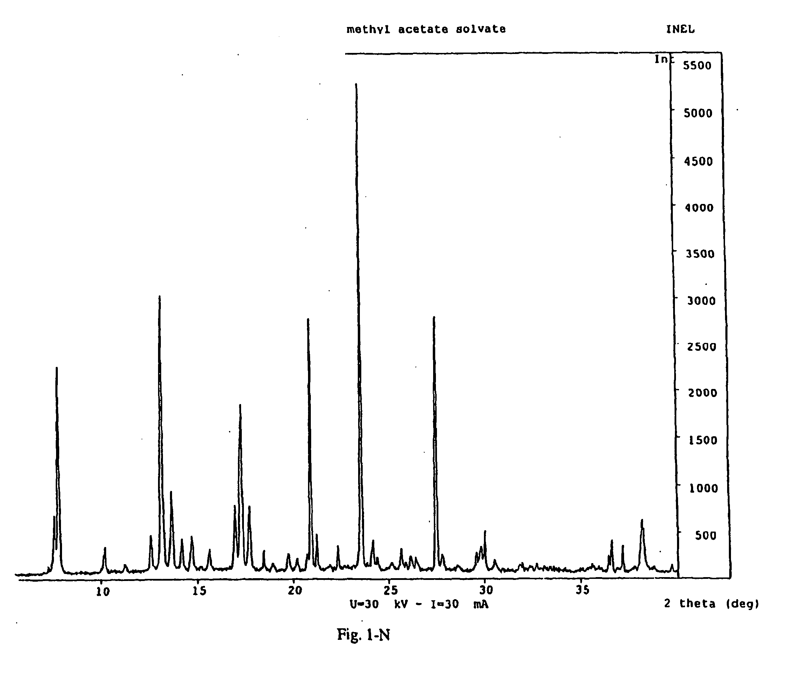

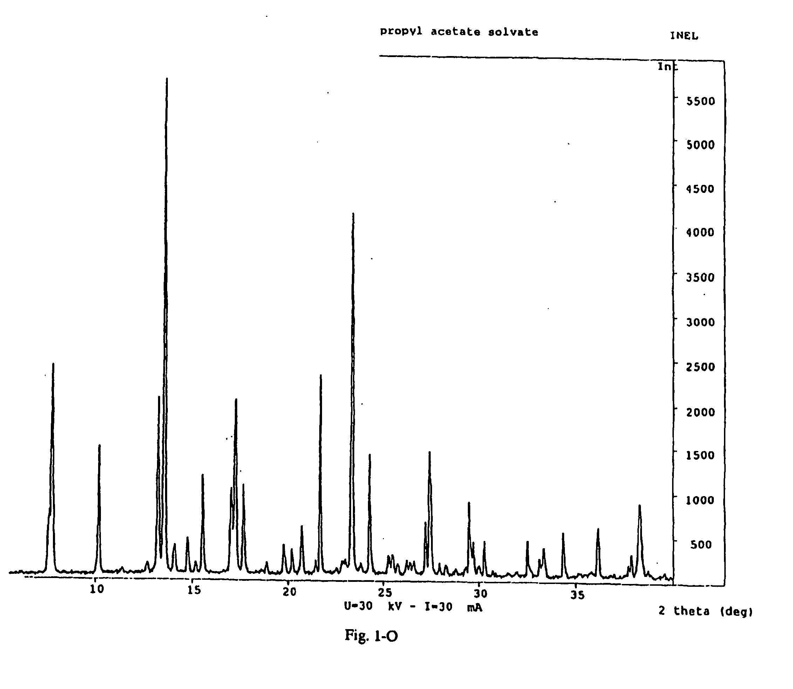

Graphical examples of the X-ray diffraction patterns for Form H, Form L, and the methyl ethyl ketone solvate crystalline forms of eplerenone are shown in Figs. 1-A, 1-B and 1-C, respectively. Form H shows distinguishing peaks at 7.0 ± 0.2, 8.3 ± 0.2, and 12.0 ± 0.2 degrees 2θ. Form L shows distinguishing peaks at 8.0 ± 0.2, 12.4 ± 0.2, 12.8 ± 0.2, and 13.3 ± 0.2 degrees 2θ. The methyl ethyl ketone solvated crystalline form shows distinguishing peaks at 7.6 ± 0.2, 7.8 ± 0.2, and 13.6 ± 0.2 degrees 2θ.

Examples of the X-ray diffraction patterns are shown in Figs. 1-D through 1-O for the following solvate crystalline forms of eplerenone: n-propyl alcohol solvate, tetrahydrofuran solvate, ethyl propionate solvate, acetic acid solvate, acetone solvate, toluene solvate, isopropanol solvate, ethanol solvate, isobutyl acetate solvate, butyl acetate solvate, methyl acetate solvate, and propyl acetate solvate, respectively.

### 3. Melting/decomposition temperature

The temperatures of melting and/or decomposition of non-solvated eplerenone crystalline forms were determined using a TA Instruments 2920 differential scanning calorimeter. Each sample, in an amount of 1-2 mg, was placed in either a sealed or unsealed aluminum pan and heated to provide a rate of temperature increase of about 10°C/minute. Melting/decomposition temperature ranges were defined from the extrapolated onset to the maximum of the melting/decomposition endotherm.

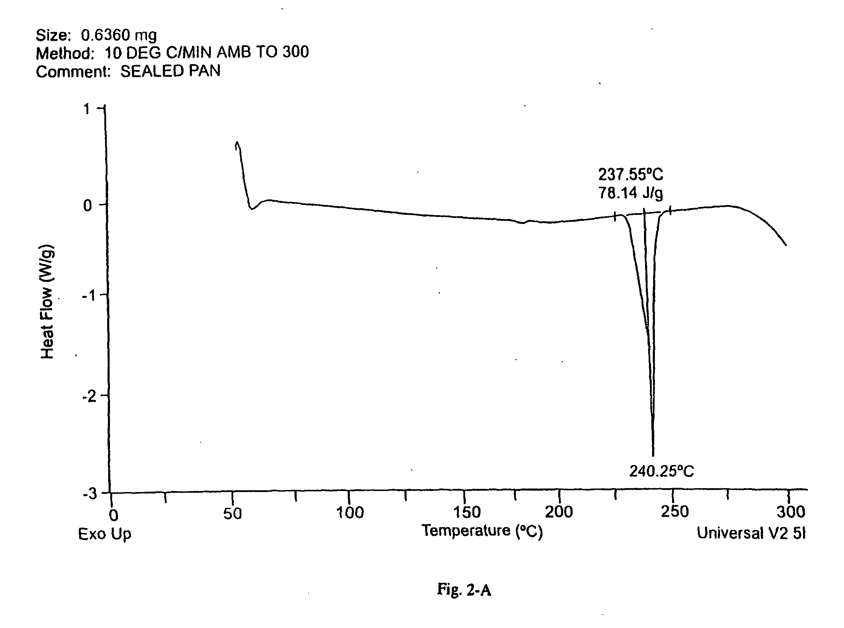

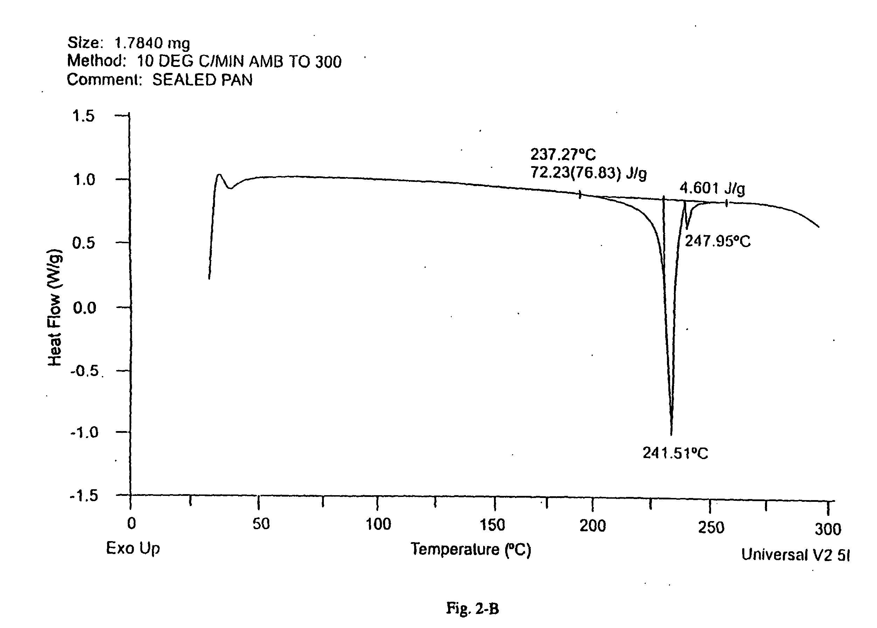

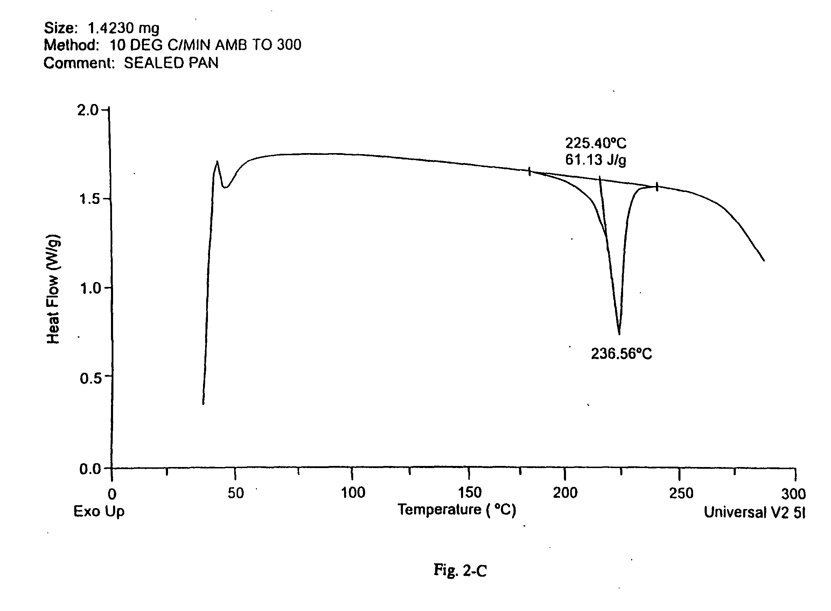

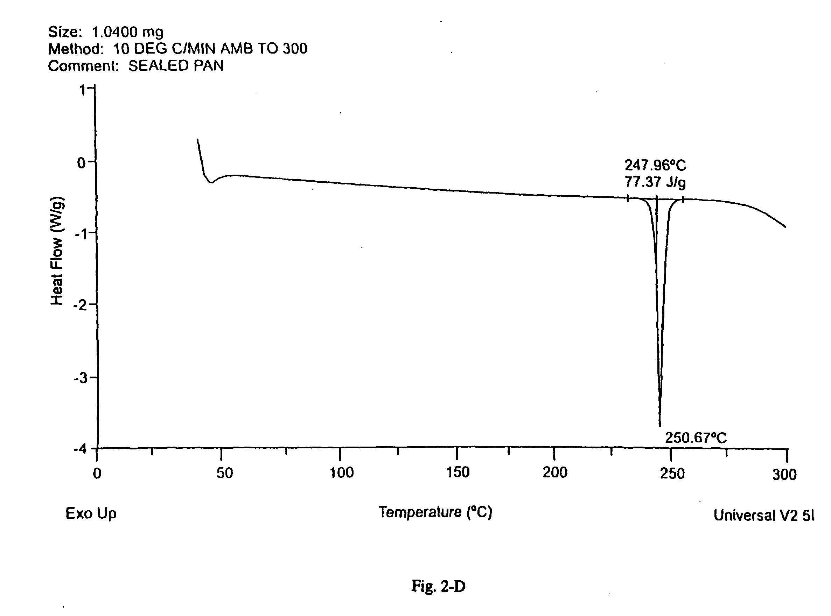

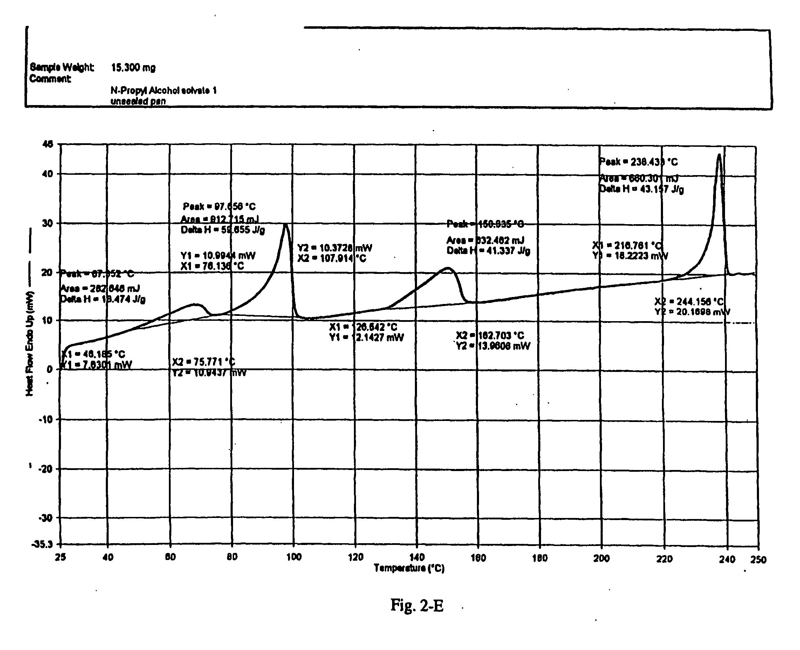

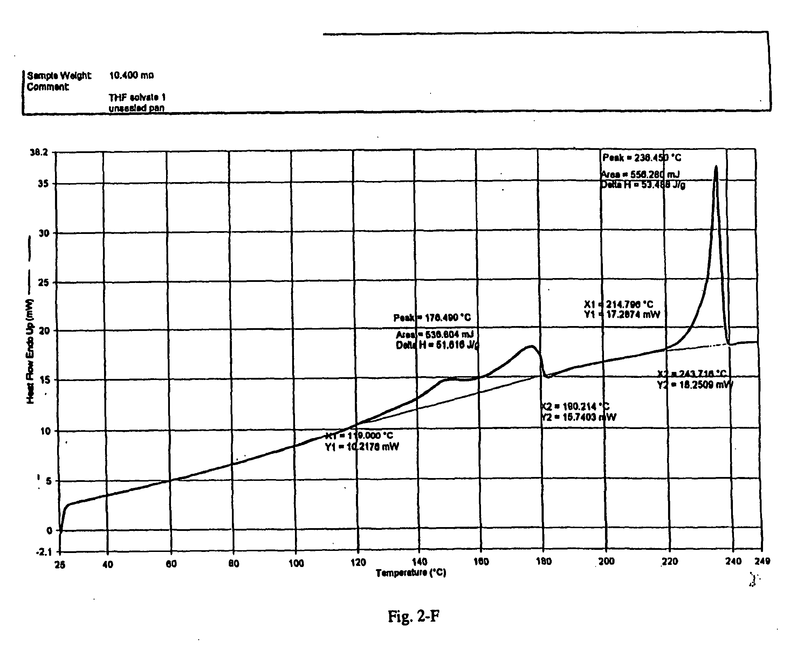

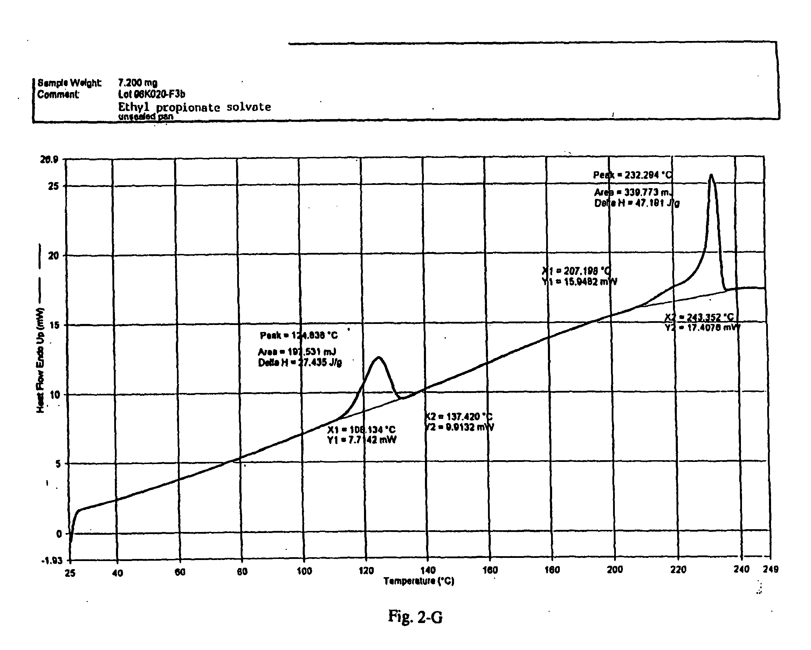

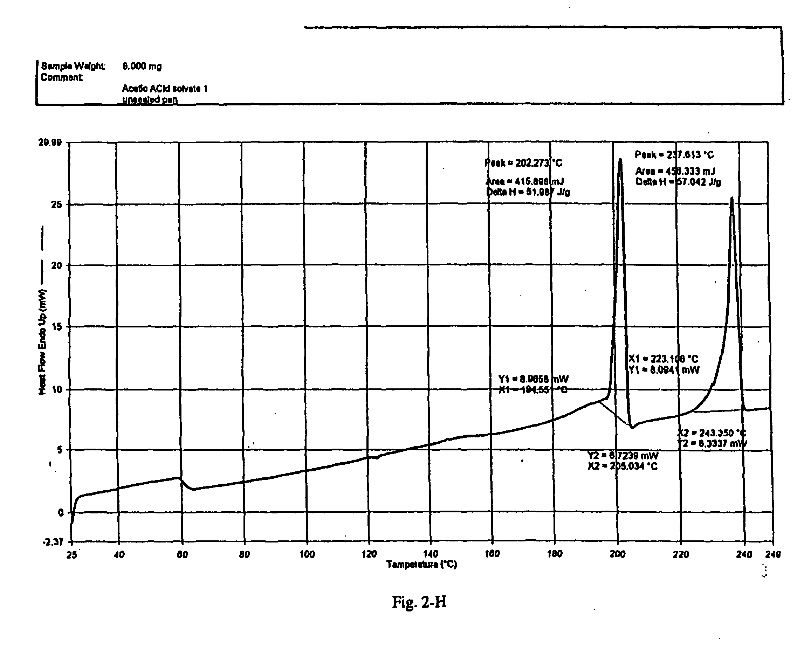

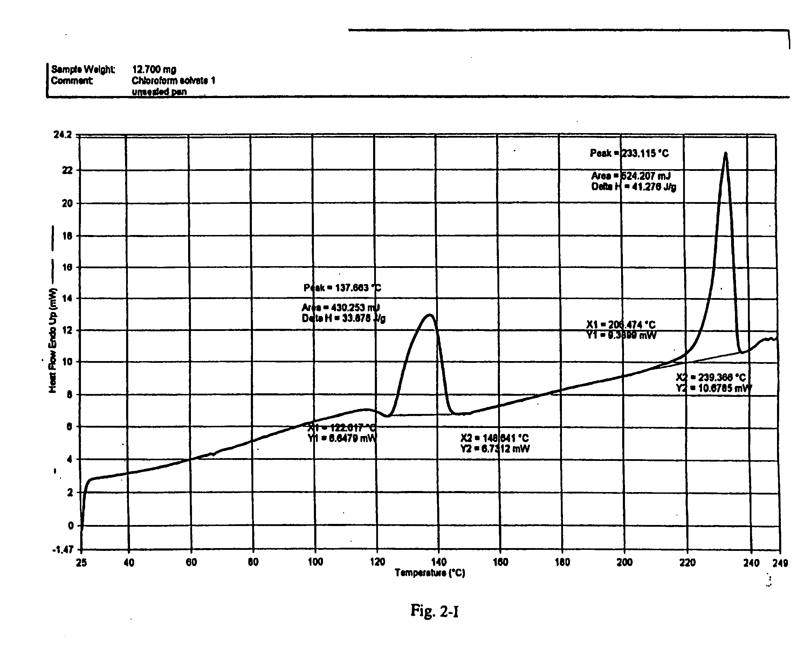

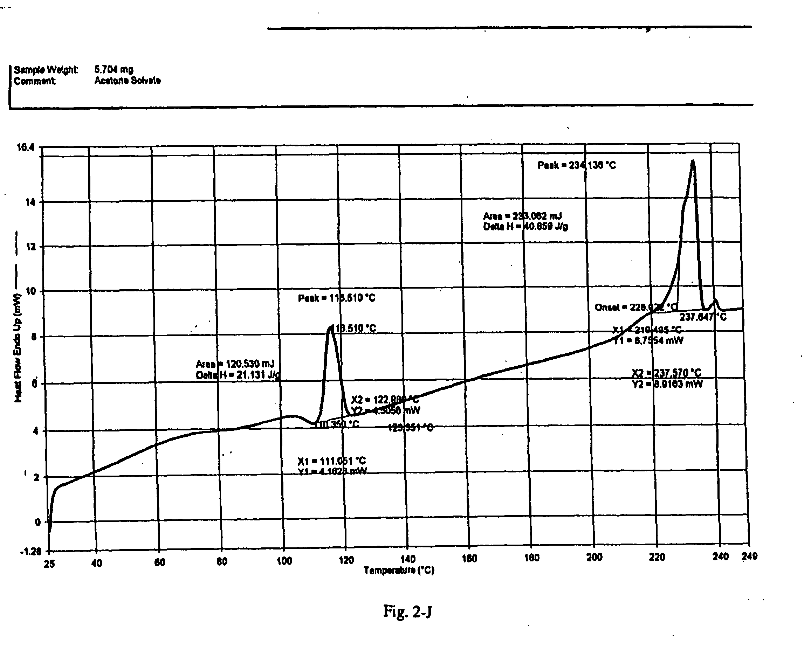

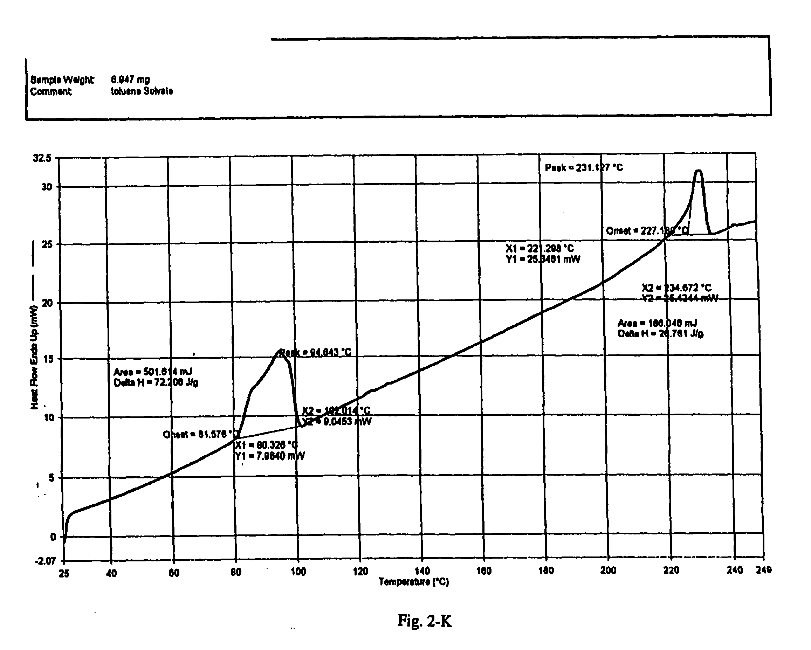

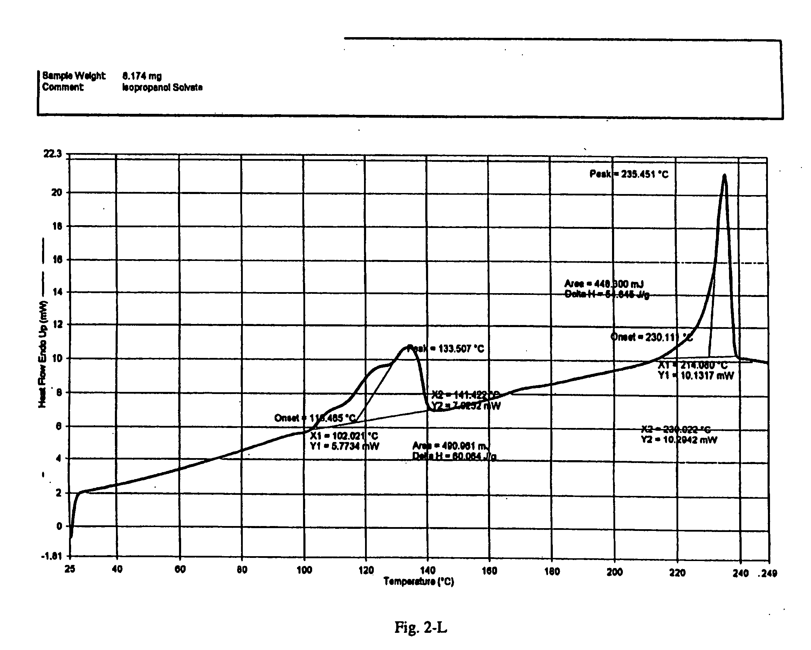

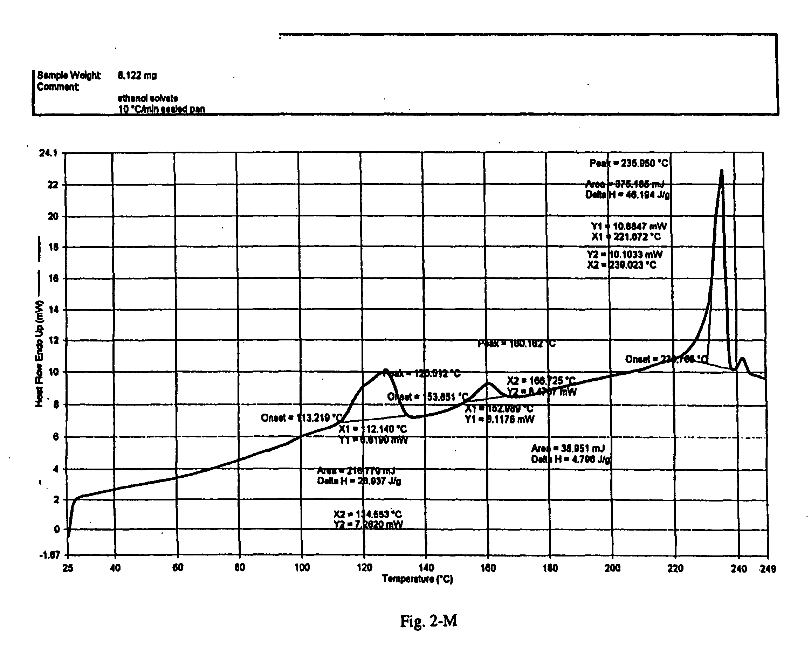

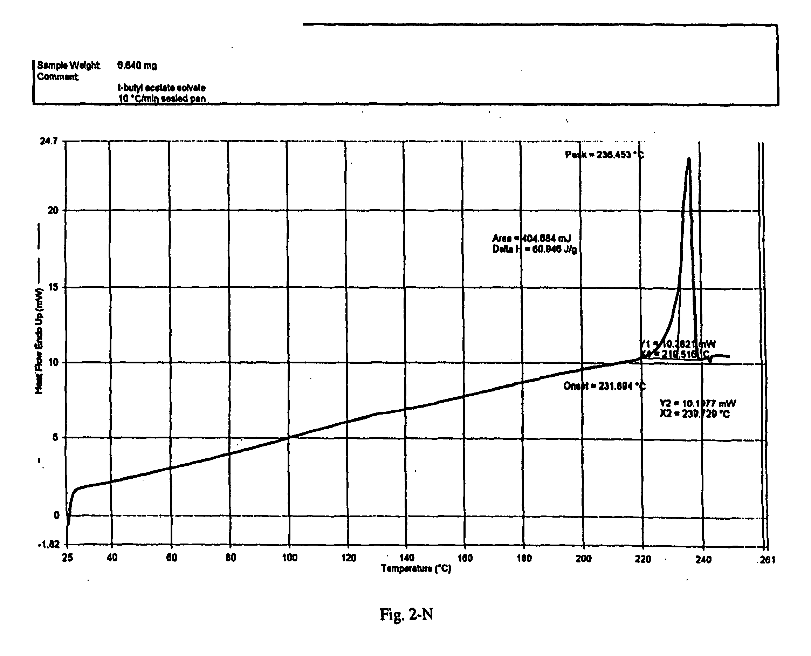

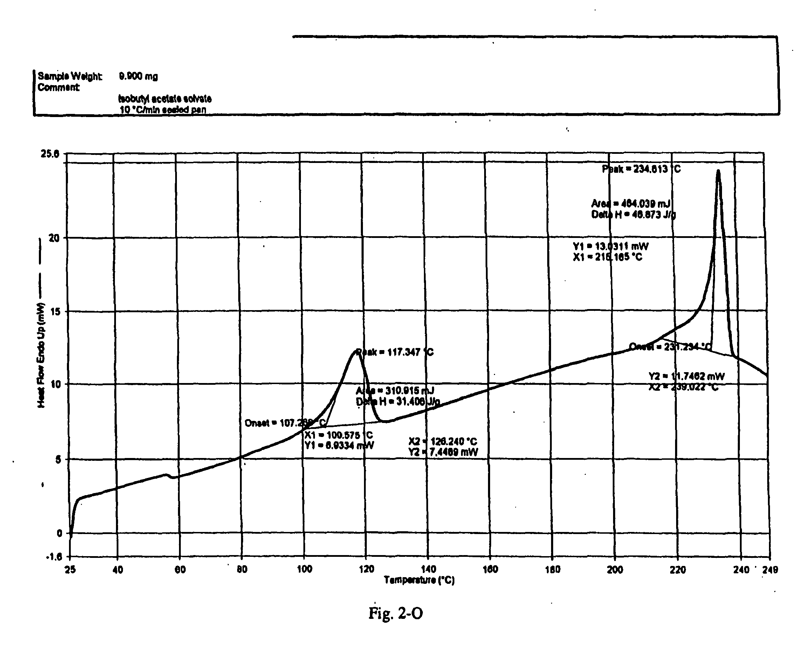

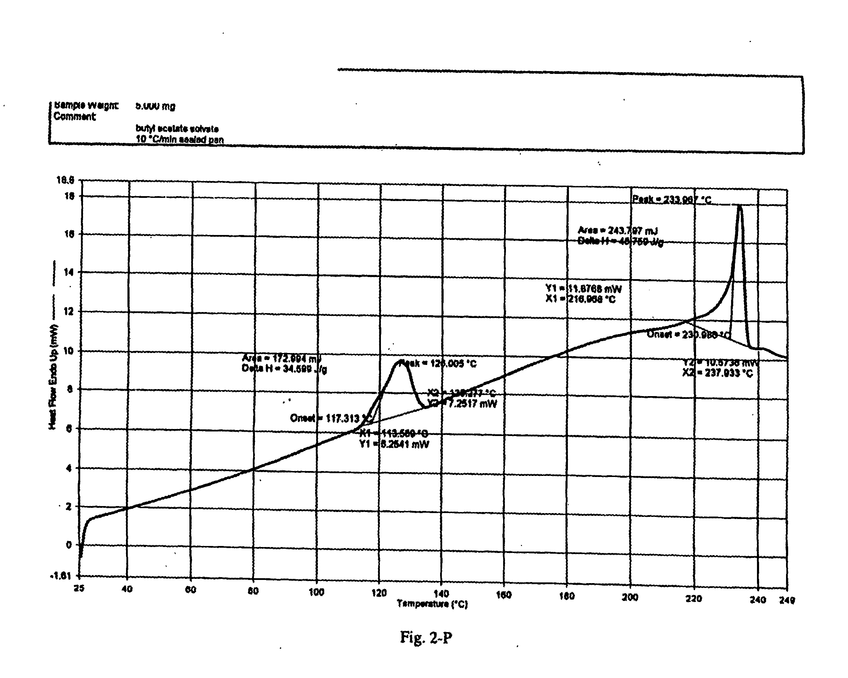

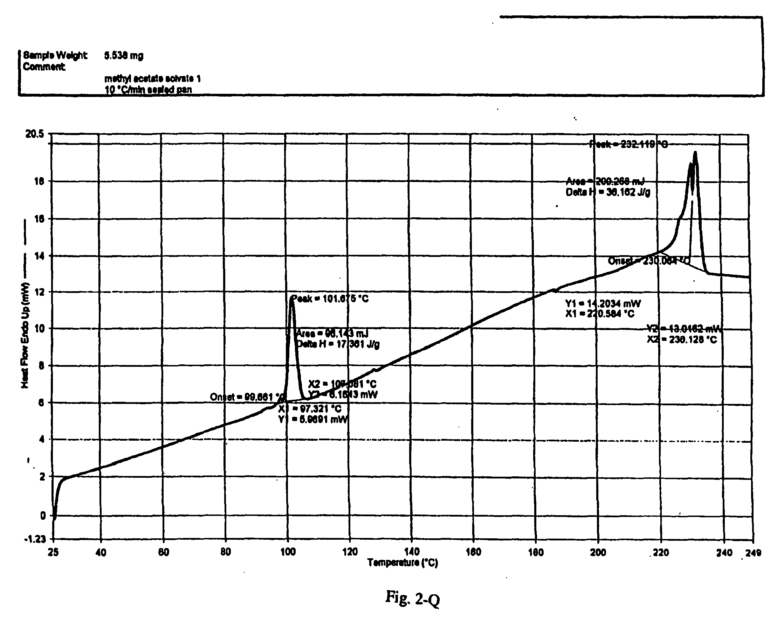

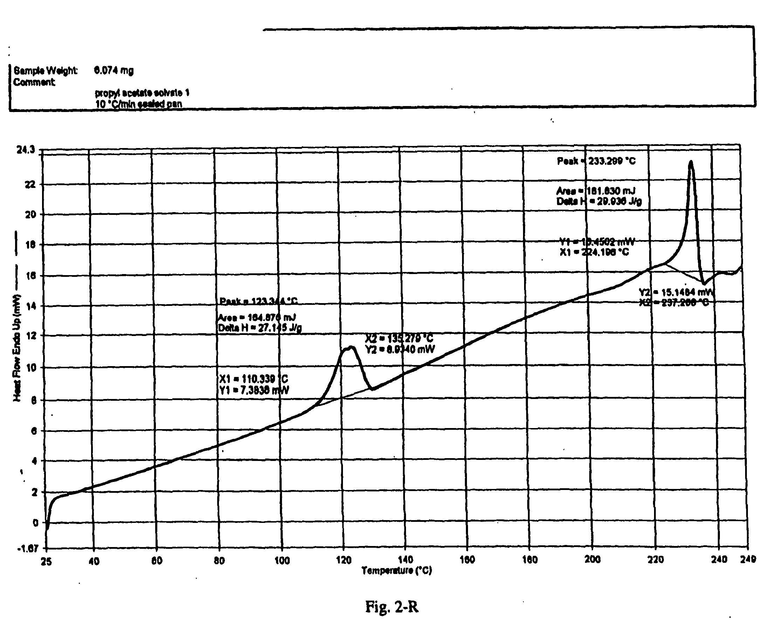

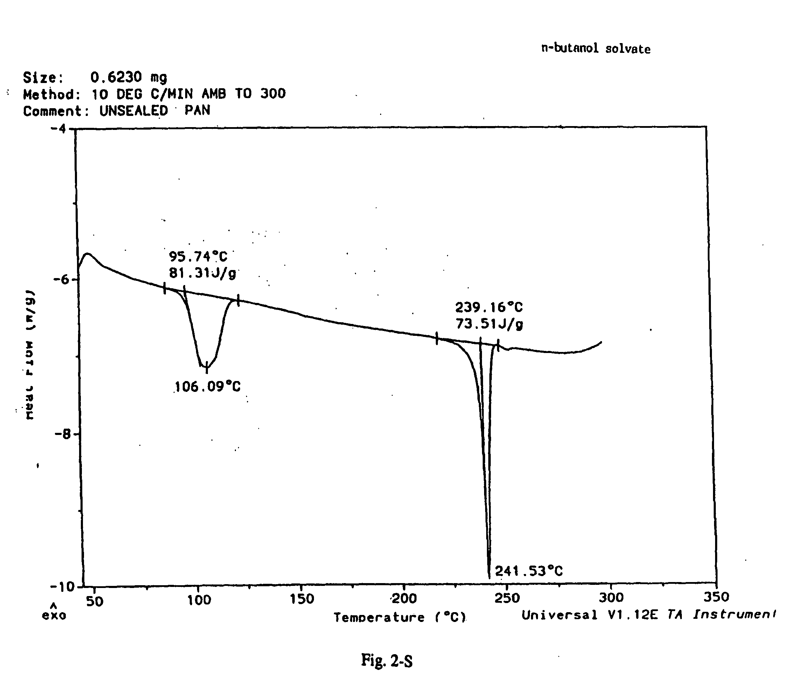

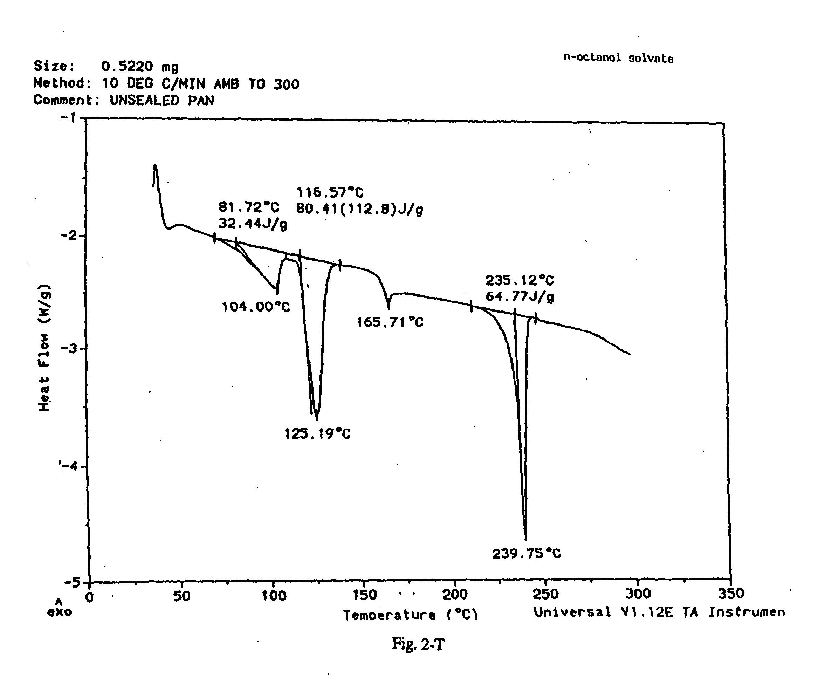

The melting of the Form H and Form L eplerenone was associated with chemical decomposition and loss of trapped solvent from the crystal lattice. The melting/decomposition temperature also was affected by the treatment of the solid prior to analysis. For example, non-milled Form L of D₉₀ particle size about 180-450 µm, prepared by direct crystallization from an appropriate solvent or from desolvation of a solvate obtained from crystallization of high purity eplerenone in an appropriate solvent or mixture of solvents, generally had a melting/decomposition range of about 237°C to about 242°C. Milled Form L having a D₉₀ particle size of about 80 to about 100 µm, prepared by crystallizing a solvate from a solution of high purity eplerenone in an appropriate solvent or mixture of solvents, desolvating the solvate and milling the resulting Form L, generally had a lower and broader melting/decomposition range of about 223°C to about 234°C. Non-milled Form H of D₉₀ particle size about 180-450 µm, prepared by desolvation of a solvate obtained by digestion of low purity eplerenone, generally had a higher melting/decomposition range of about 247°C to about 251°C. Examples of the DSC thermograms of (a) non-milled Form L directly crystallized from methyl ethyl ketone, (b) non-milled Form L prepared by desolvation of a solvate obtained by crystallization of a high purity eplerenone from methyl ethyl ketone, (c) Form L prepared by milling a desolvated solvate obtained by crystallization of high purity eplerenone from methyl ethyl ketone, and (d) non-milled Form H prepared by desolvation of a solvate obtained by digestion of low purity eplerenone from methyl ethyl ketone are shown in Figures 2-A, 2-B, 2-C and 2-D, respectively.

DSC thermograms of solvated forms of eplerenone were determined using a Perkin Elmer Pyris 1 differential scanning calorimeter. Each sample, in an amount of 1-2 mg was placed in an unsealed aluminum pan and heated to provide a rate of temperature increase of about 10°C/minute. One or more endothermal events at lower temperatures were associated with enthalpy changes that occurred as solvent was lost from the solvate crystal lattice. The highest temperature endotherm or endotherms were associated with the melting/decomposition of Form L or Form H eplerenone. Examples of the DSC thermograms are shown in Figs. 2-E through 2-T for the following solvated crystalline forms of eplerenone: n-propyl alcohol solvate, tetrahydrofuran solvate, ethyl propionate solvate, acetic acid solvate, chloroform solvate, acetone solvate, toluene solvate, isopropanol solvate, t-butyl acetate solvate, ethanol solvate, isobutyl acetate solvate, butyl acetate solvate, methyl acetate solvate, propyl acetate solvate, n-butanol solvate, and n-octanol solvate, respectively.

### 4. Infrared absorption spectroscopy

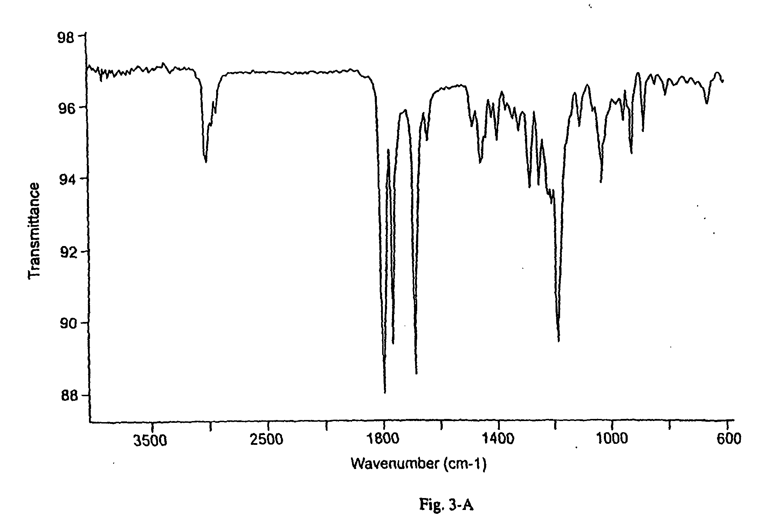

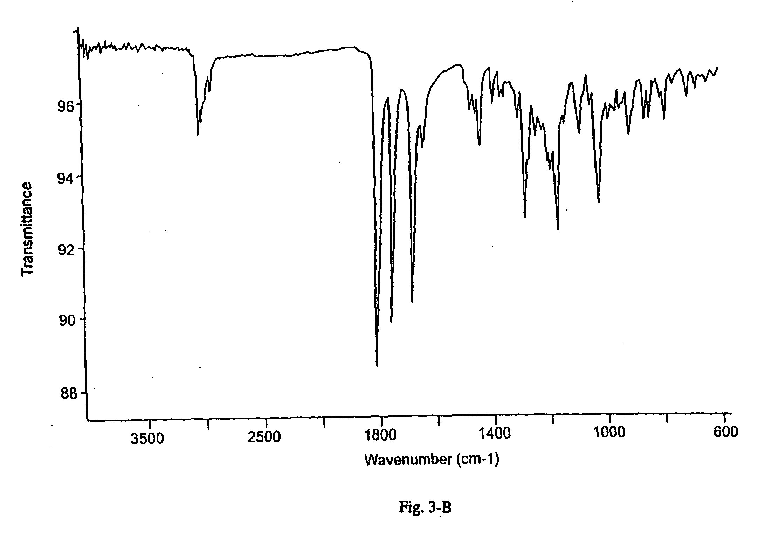

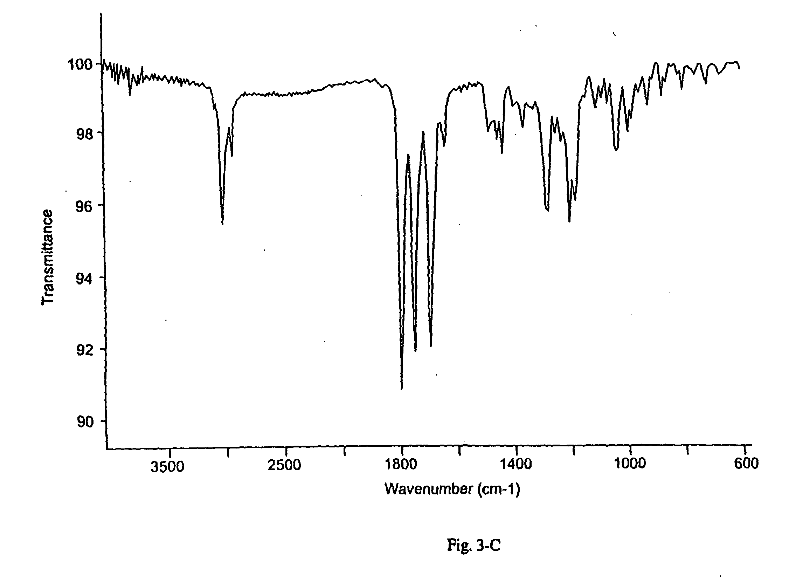

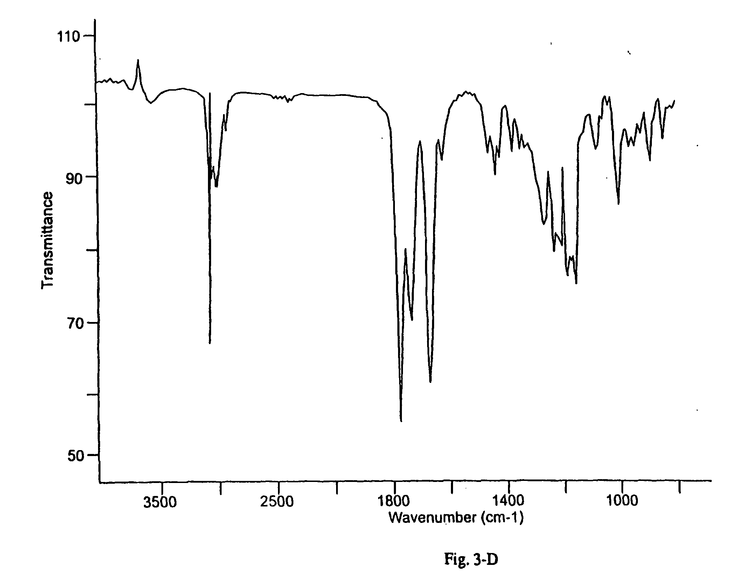

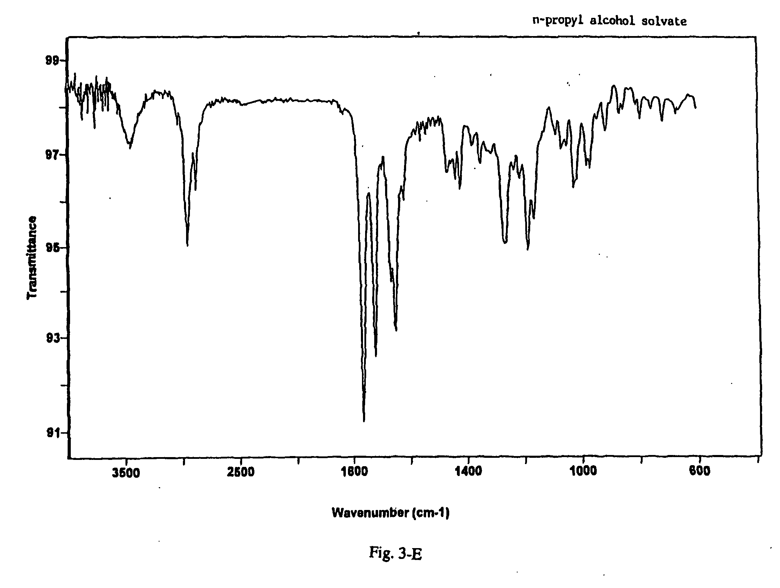

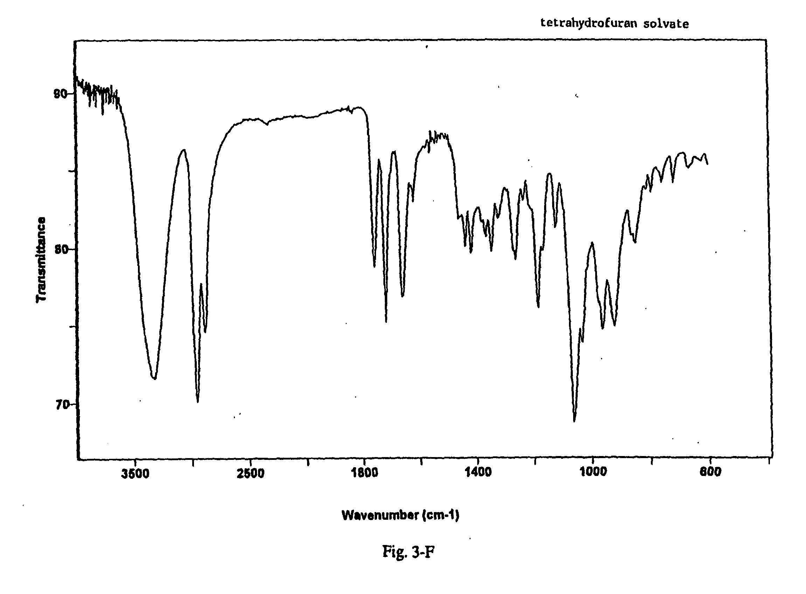

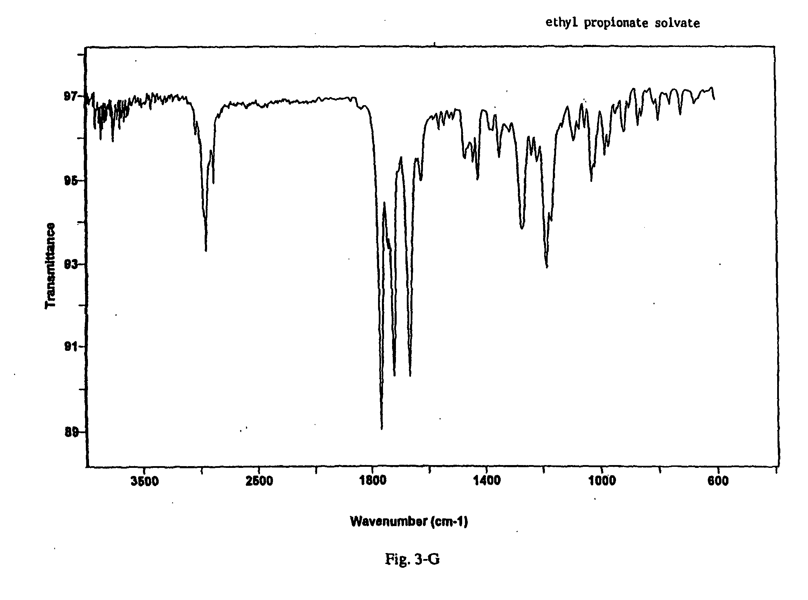

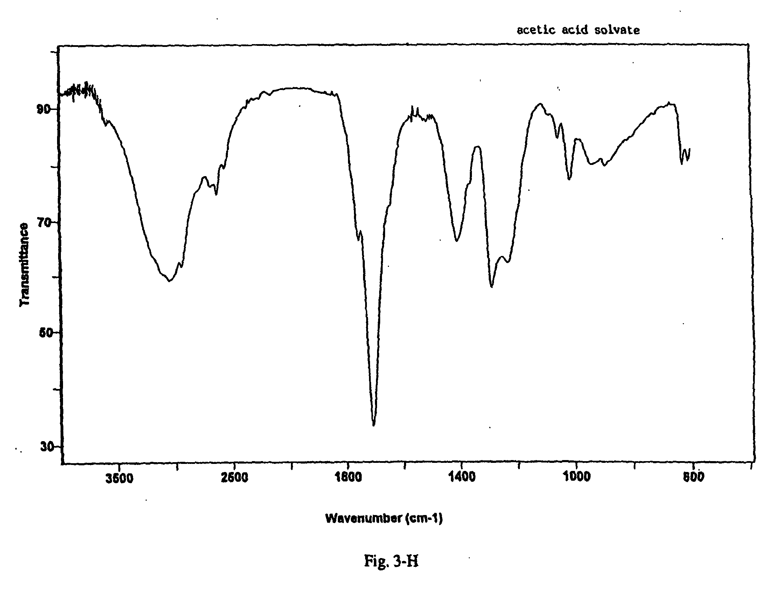

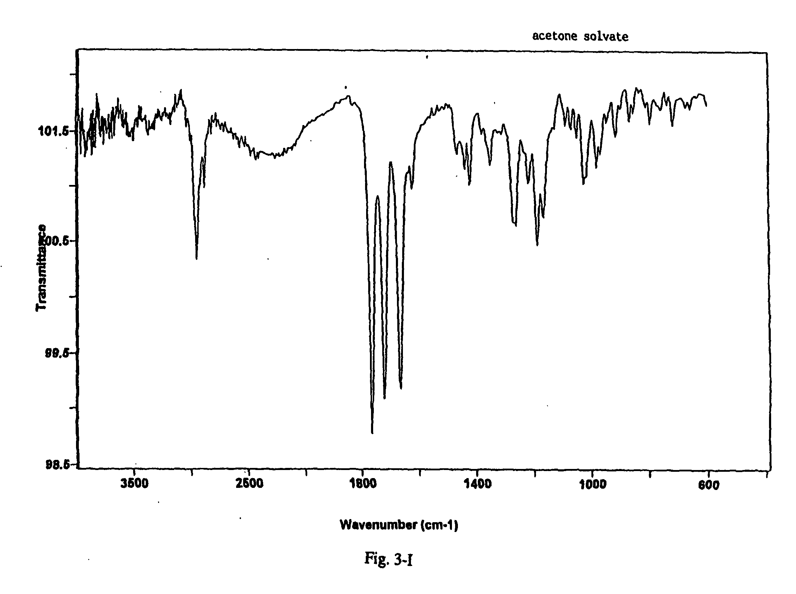

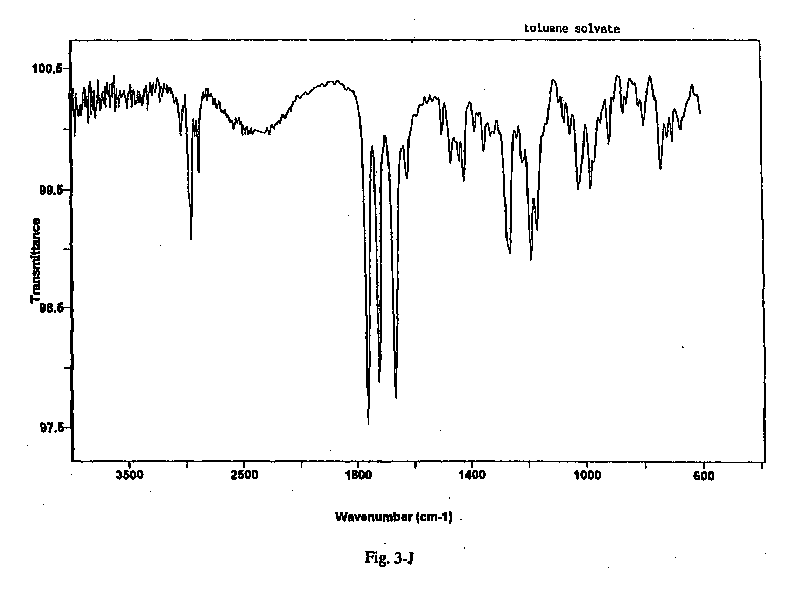

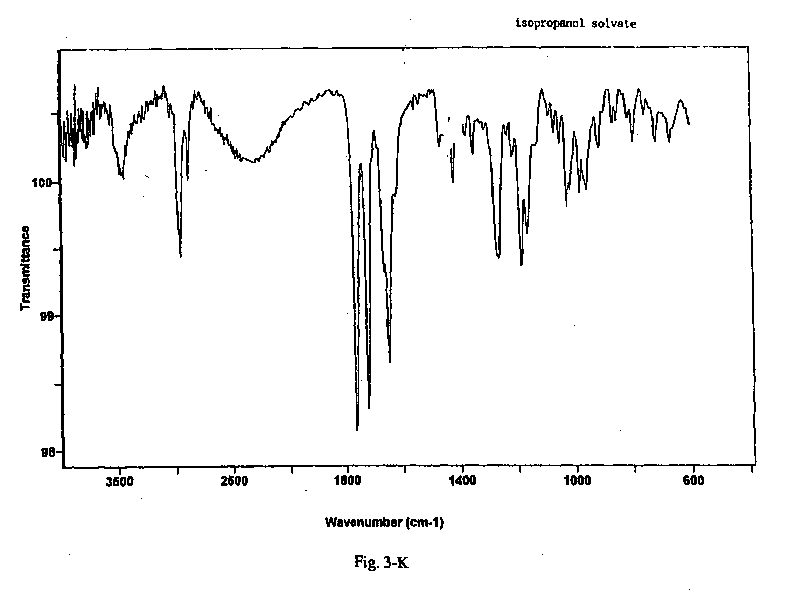

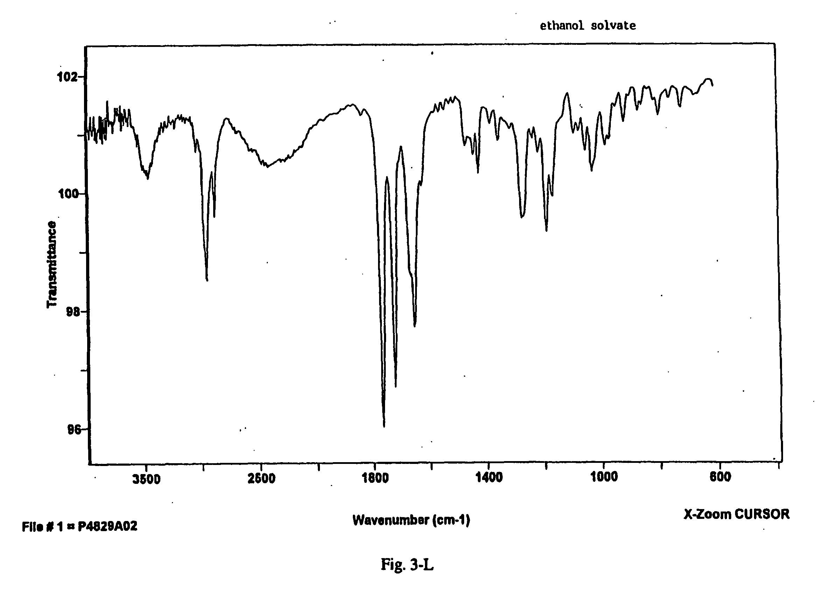

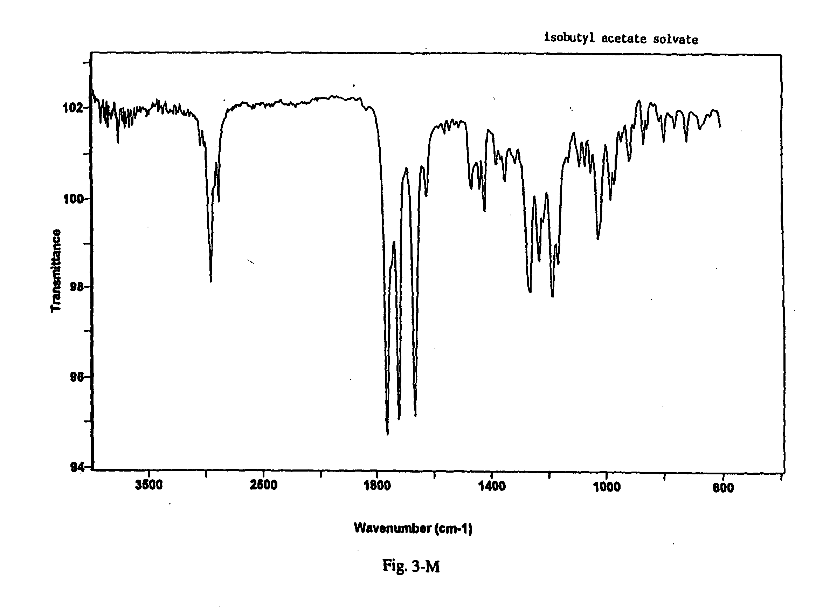

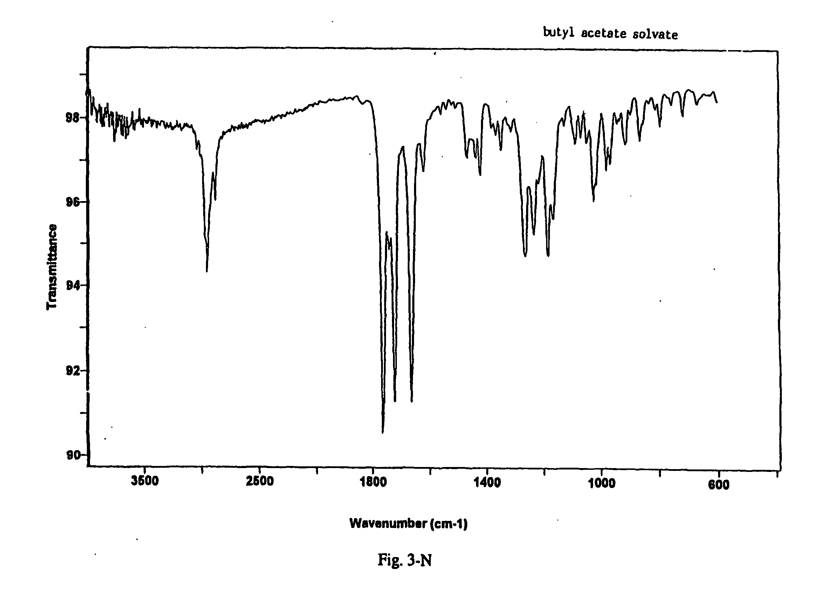

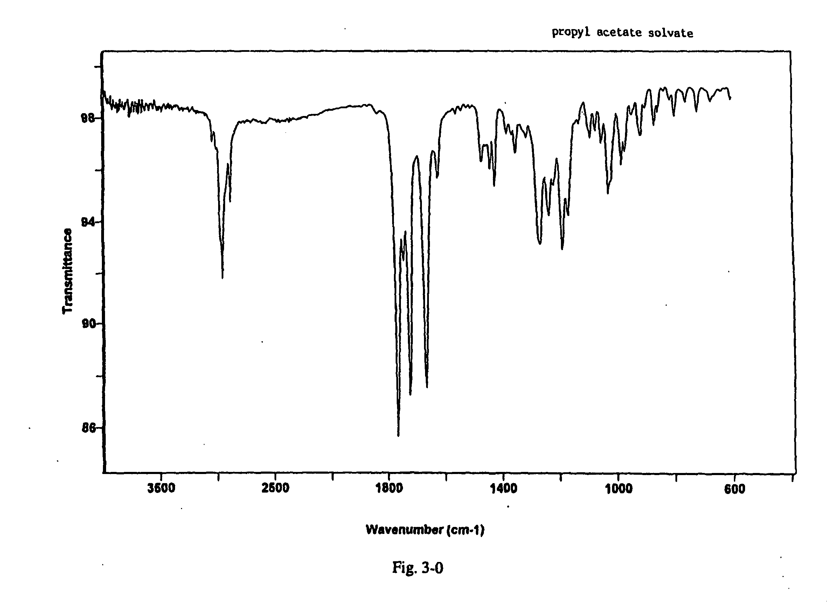

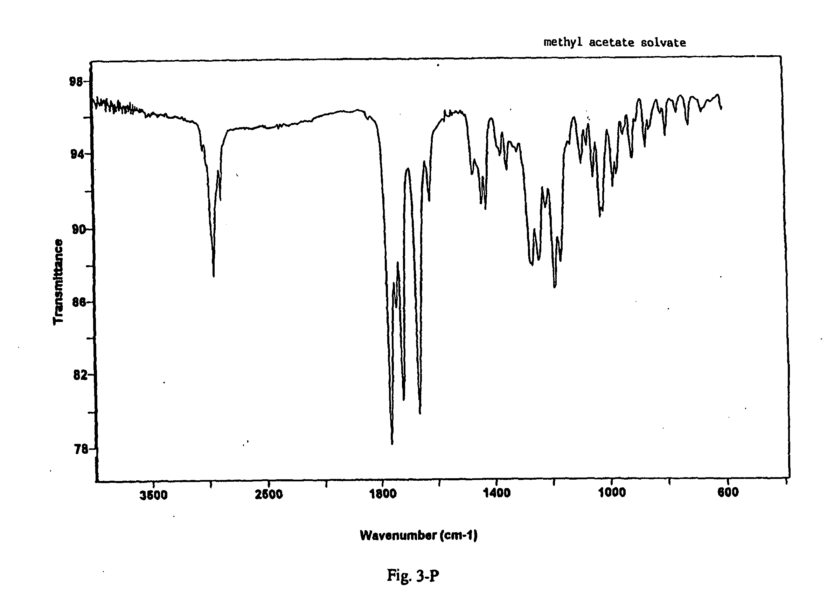

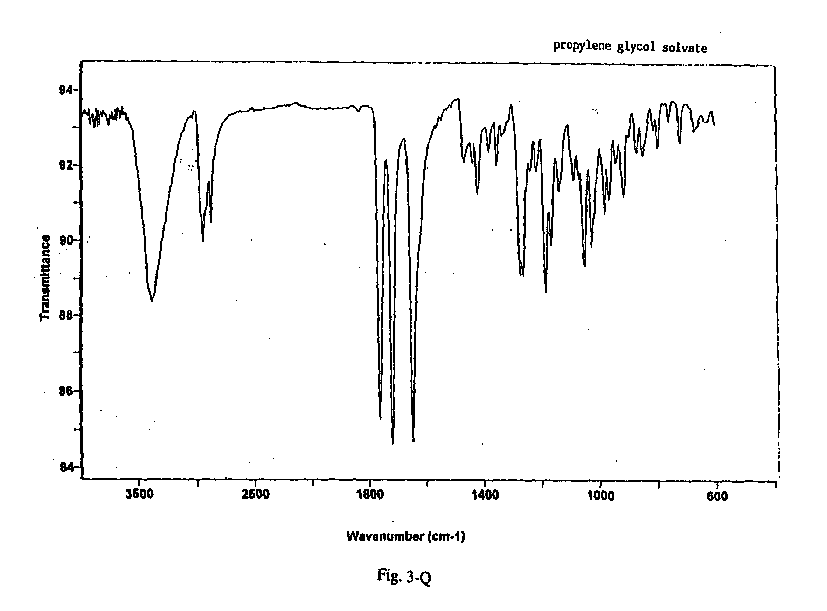

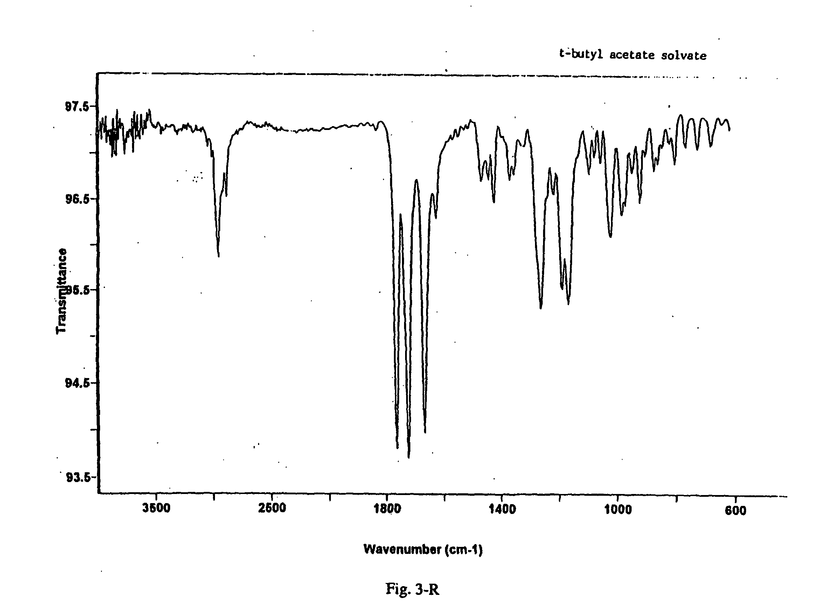

Infrared absorption spectra of non-solvated eplerenone Form H and Form L were obtained with a Nicolet DRIFT (diffuse reflectance infrared fourier transform) Magna System 550 spectrophotometer. A Spectra-Tech Collector system and a microsample cup were used. Samples (5%) were analyzed in potassium bromide and scanned from 400 to 4000 cm⁻¹. Infrared absorption spectra of eplerenone in dilute chloroform solution (3%) or in the solvated crystal forms were obtained with a Bio-rad FTS-45 spectrophotometer. Chloroform solution samples were analyzed using a solution cell of 0.2 mm path length with sodium chloride salt plates. Solvate FTIR spectra were collected using an IBM micro-MIR (multiple internal reflectance) accessory. Samples were scanned from 400 to 4000 cm⁻¹. Examples of the infrared absorption spectra of (a) Form H, (b) Form L, (c) the methyl ethyl ketone solvate and (d) eplerenone in chloroform solution are shown in Figs. 3-A, 3-B, 3-C and 3-D, respectively.

Table 2 discloses illustrative absorption bands for eplerenone in the Form H, Form L, and methyl ethyl ketone solvate crystal forms. Illustrative absorption bands for eplerenone in chloroform solution are also disclosed for comparison. Differences between Form H and either Form L or the methyl ethyl ketone solvate were observed, for example, in the carbonyl region of the spectrum. Form H has an ester carbonyl stretch of approximately 1739 cm⁻¹ while both Form L and the methyl ethyl ketone solvate have the corresponding stretch at approximately 1724 and 1722 cm⁻¹, respectively. The ester carbonyl stretch occurs at approximately 1727 cm⁻¹ in the eplerenone in chloroform solution. The change in stretching frequency of the ester carbonyl between Form H and Form L reflects a change in orientation of the ester group between the two crystal forms. In addition, the stretch of the ester of the conjugated ketone in the A-steroid ring shifts from approximately 1664-1667 cm⁻¹ in either Form H or the methyl ethyl ketone solvate to approximately 1655 cm⁻¹ in Form L. The corresponding carbonyl stretch occurs at approximately 1665 cm⁻¹ in chloroform solution.

Another difference between Form H and Form L was seen in the C-H bending region. Form H has an absorption at approximately 1399 cm⁻¹ which is not observed in Form L, the methyl ethyl ketone solvate, or the eplerenone in chloroform solution. The 1399 cm⁻¹ stretch occurs in the region of CH₂ scissoring for the C2 and C21 methylene groups adjacent to carbonyl groups.

**Table 2:**

| **IR absorption bands (cm**^{**-1**}**) for eplerenone forms** | | | | |
|---|---|---|---|---|
| **Absorption region** | **Form H** | **Form L** | **Methyl ethyl ketone solvate** | **Chloroform solution** |
| ν C=O(lactone) | 1773 | 1775 | 1767 | 1768 |
| ν C=O(ester) | 1739 | 1724 | 1722 | 1727 |
| ν C=O(3-keto) | 1664 | 1655 | 1667 | 1665 |
| ν C=C(3,4-olefin) | 1619 | 1619 | 1622 | 1623 |
| δₐₛCH₃, δCH₂, δCH₂(α to carbonyl) | 1460, 1444, 1426 | 1467, 1438, 1422, 1399 | 1467, 1438, 1422 | 1464, 1438, 1422 |
| δₛCH₃ | 1380 | 1381 | -1380 | 1378 |

Examples of infrared absorption spectra are shown in Figs. 3-E through 3-S for the following solvated crystalline forms of eplerenone: n-propyl alcohol solvate, tetrahydrofuran solvate, ethyl propionate solvate, acetic acid solvate, acetone solvate, toluene solvate, isopropanol solvate, ethanol solvate, isobutyl acetate solvate, butyl acetate solvate, propyl acetate solvate, methyl acetate solvate, propylene glycol solvate and t-butyl acetate solvate respectively.

### 5. Nuclear magnetic resonance (NMR) spectroscopy

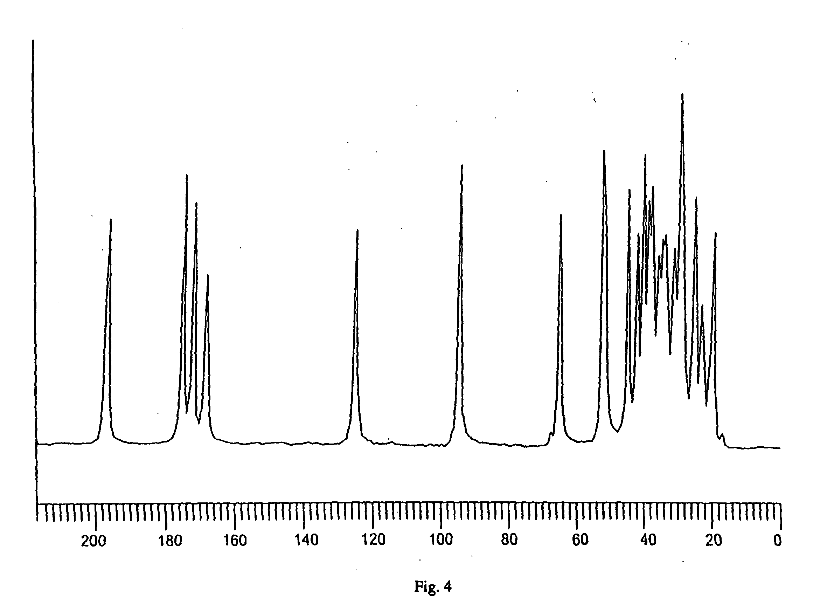

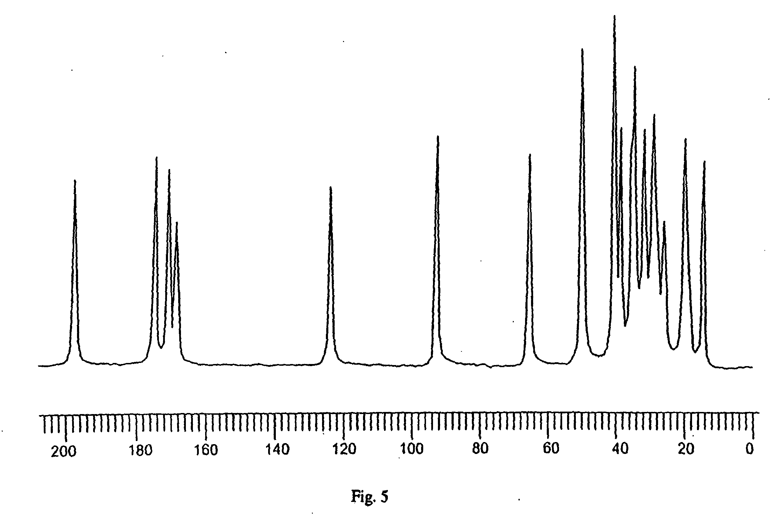

¹³C NMR spectra were obtained at a field of 31.94 MHz. Examples of the ¹³C NMR spectra of Form H and Form L eplerenone are shown in Figs. 4 and 5 respectively. The Form H eplerenone analyzed to obtain the data reflected in Fig. 4 was not phase pure and included a small amount of Form L eplerenone. Form H is most clearly distinguished by the carbon resonances at around 64.8 ppm, 24.7 ppm and 19.2 ppm. Form L is most clearly distinguished by the carbon resonances at around 67.1 ppm and 16.0 ppm.

### 6. Thermogravimetry

Thermogravimetric analysis was performed using a TA Instruments TGA 2950 thermogravimetric analyzer. Samples were placed in an unsealed aluminum pan under nitrogen purge. Starting temperature was 25°C with the temperature increased at a rate of about 10°C/minute.

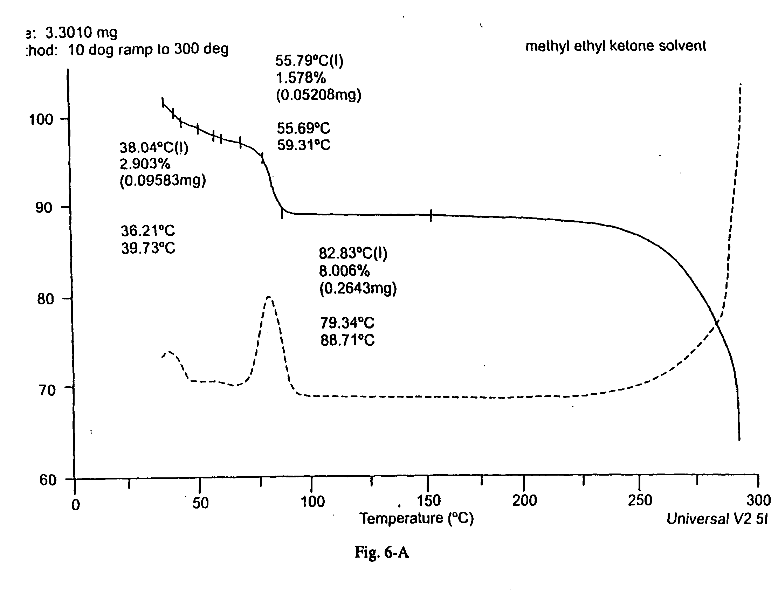

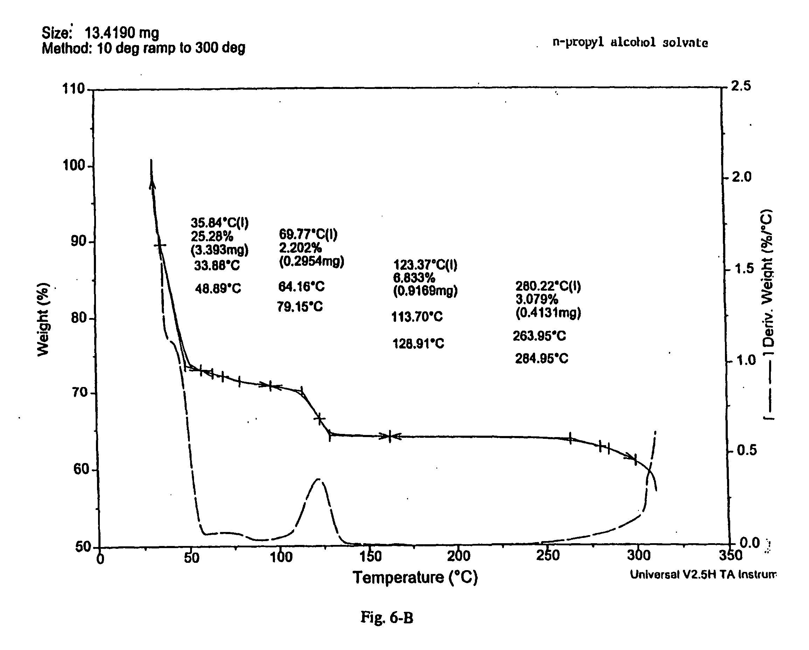

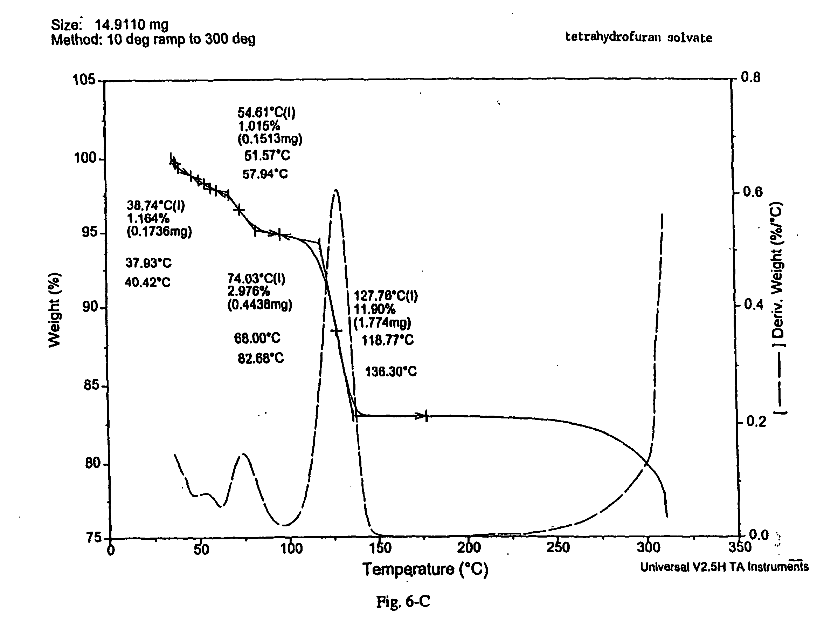

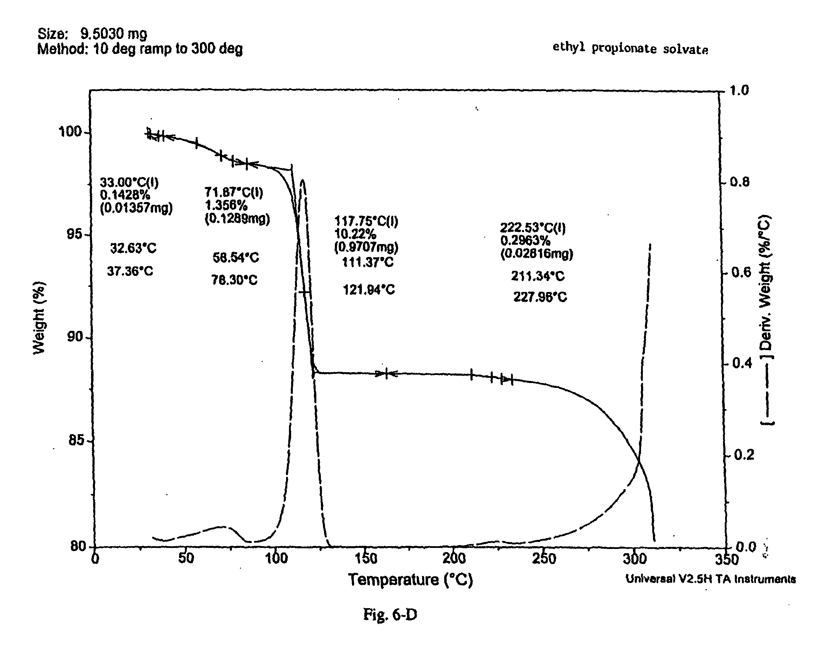

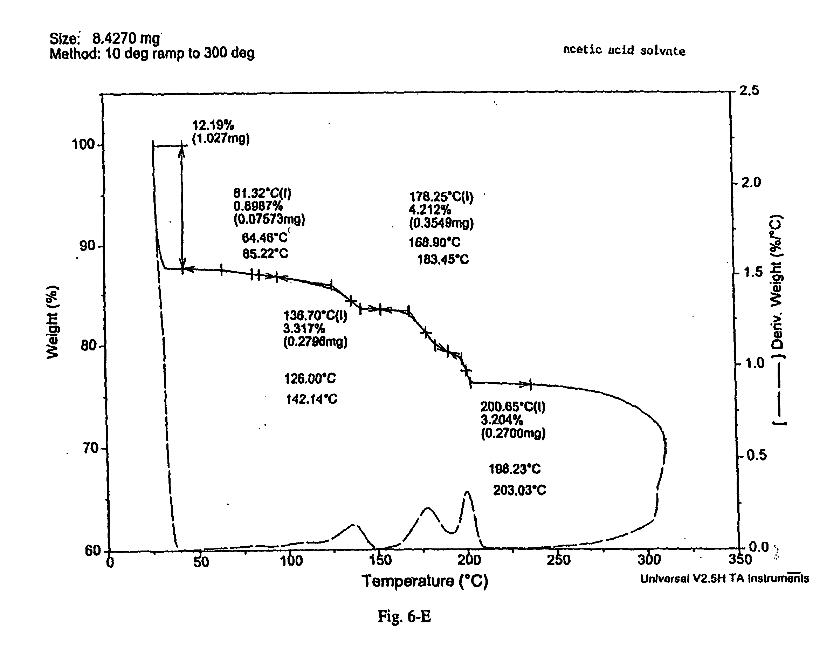

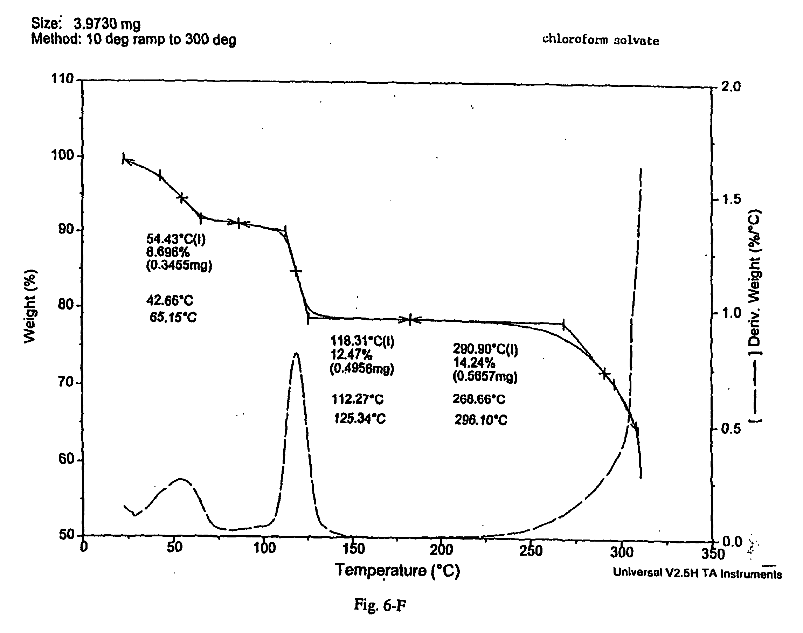

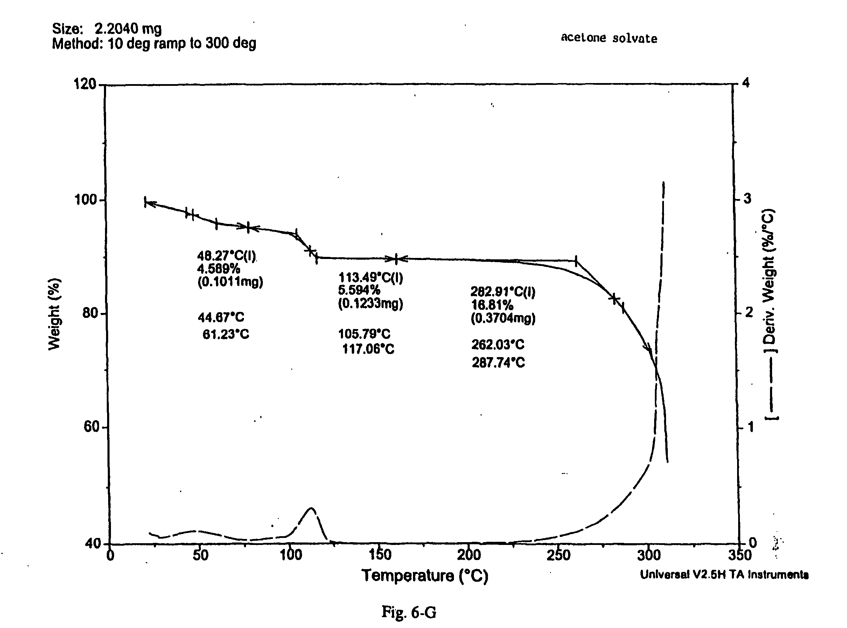

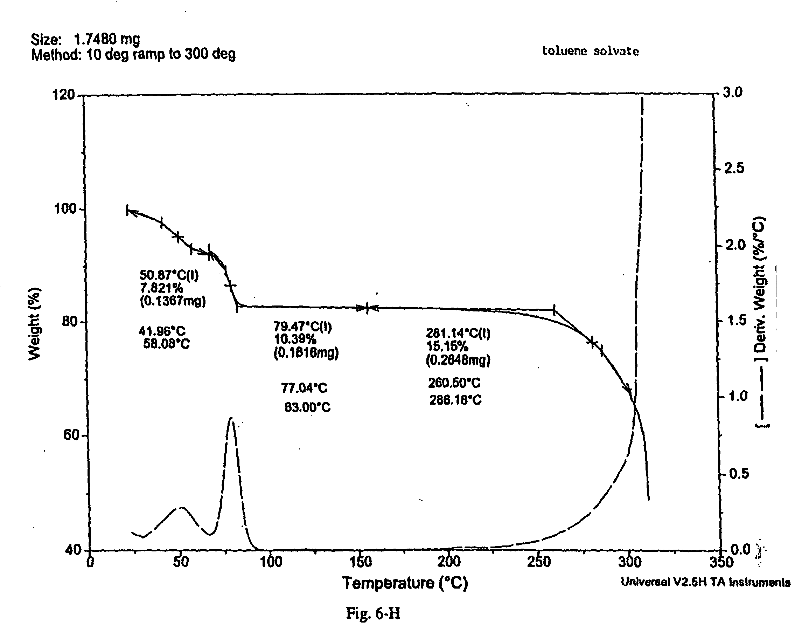

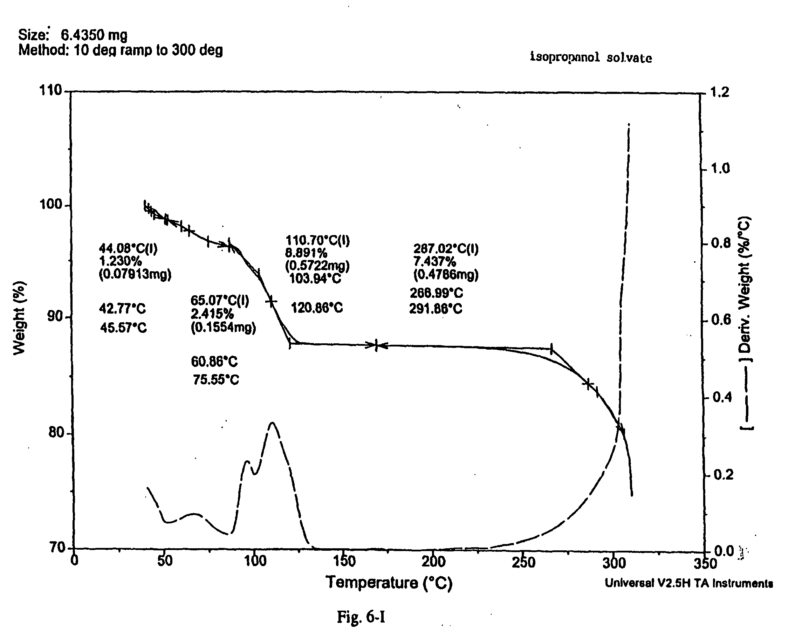

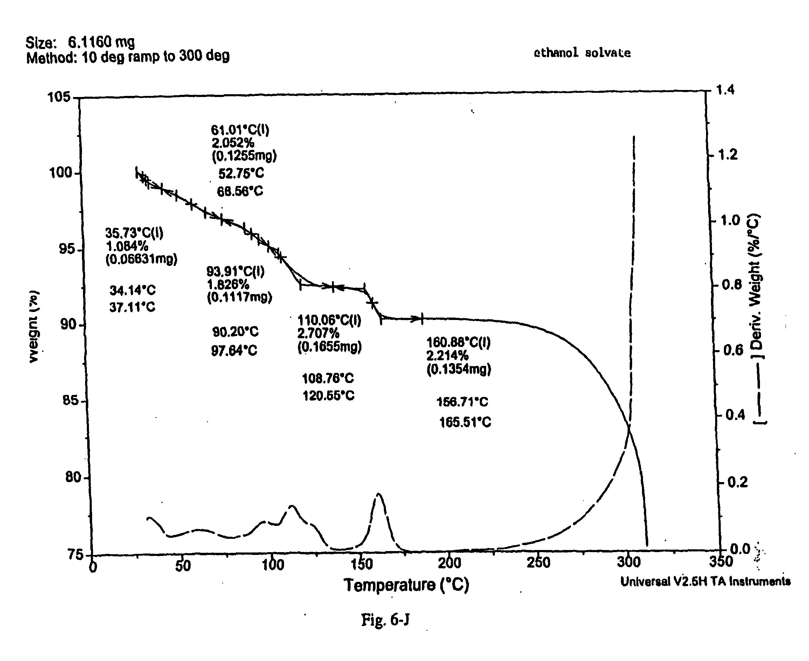

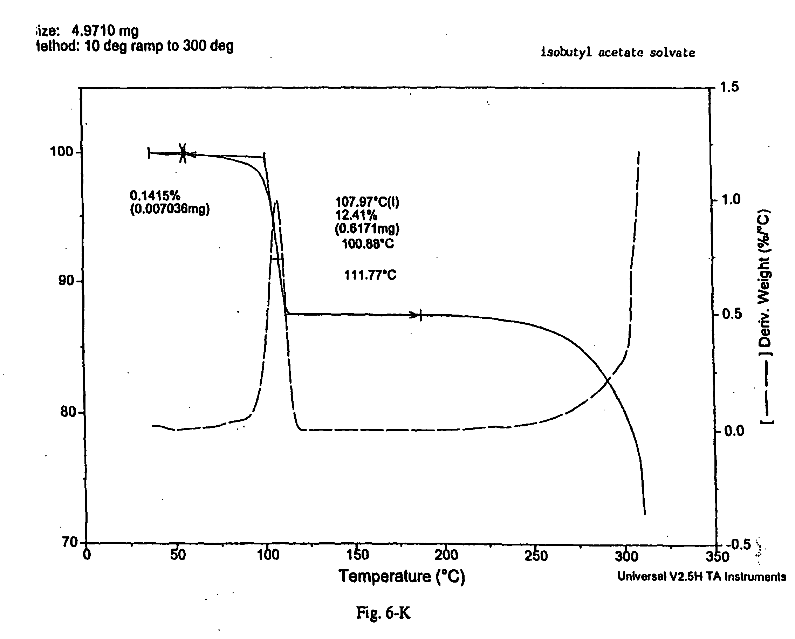

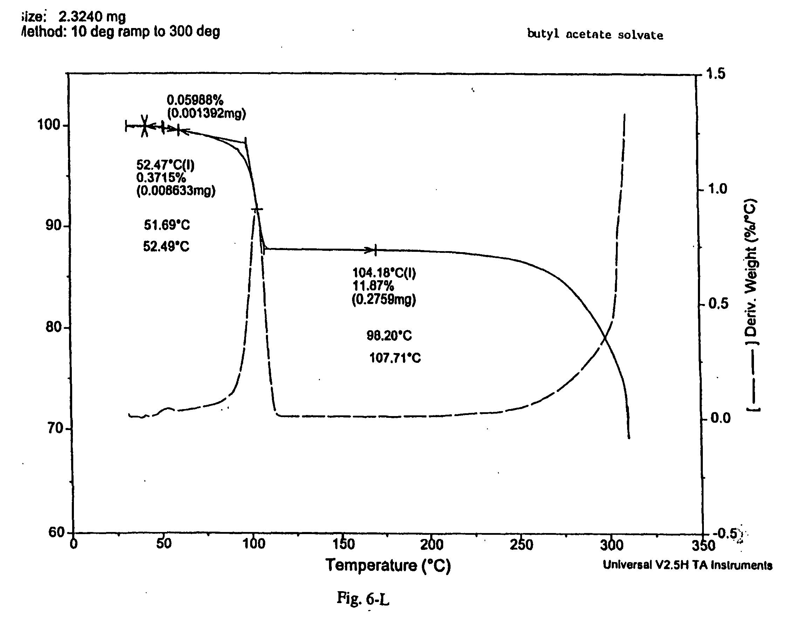

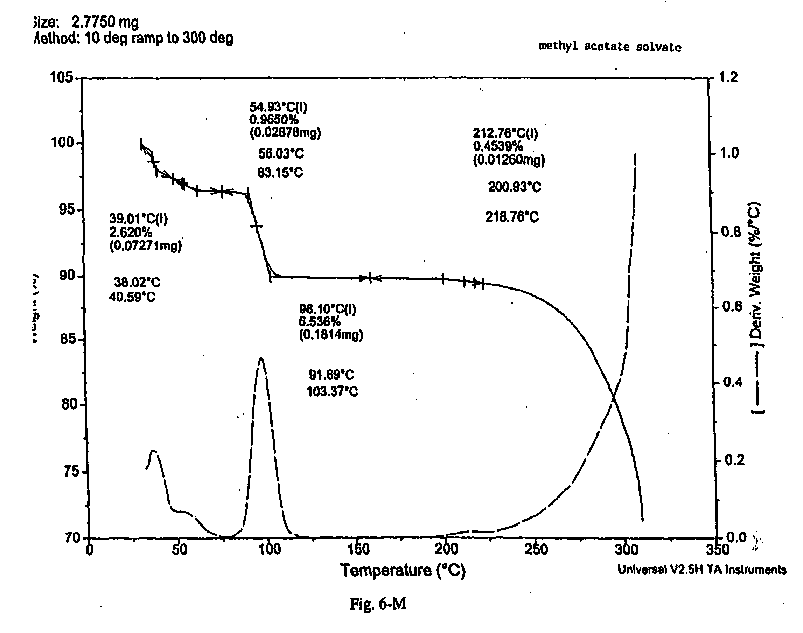

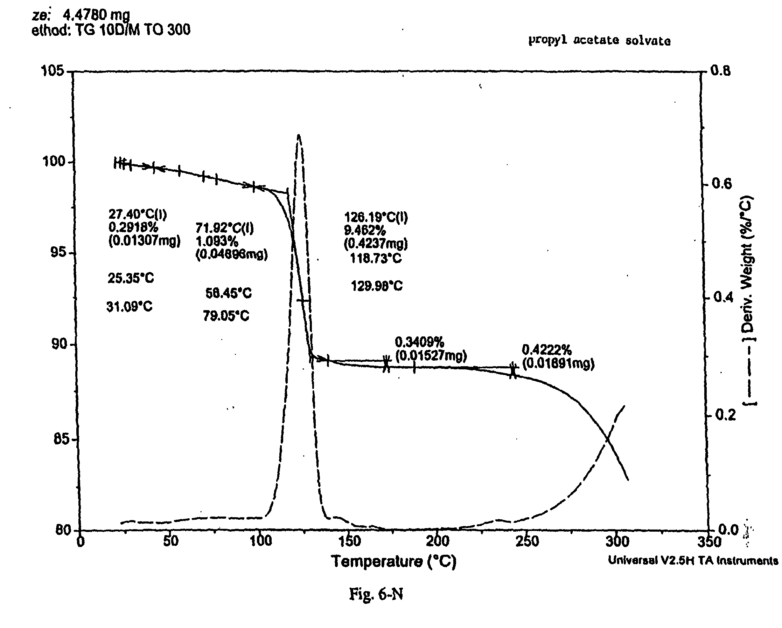

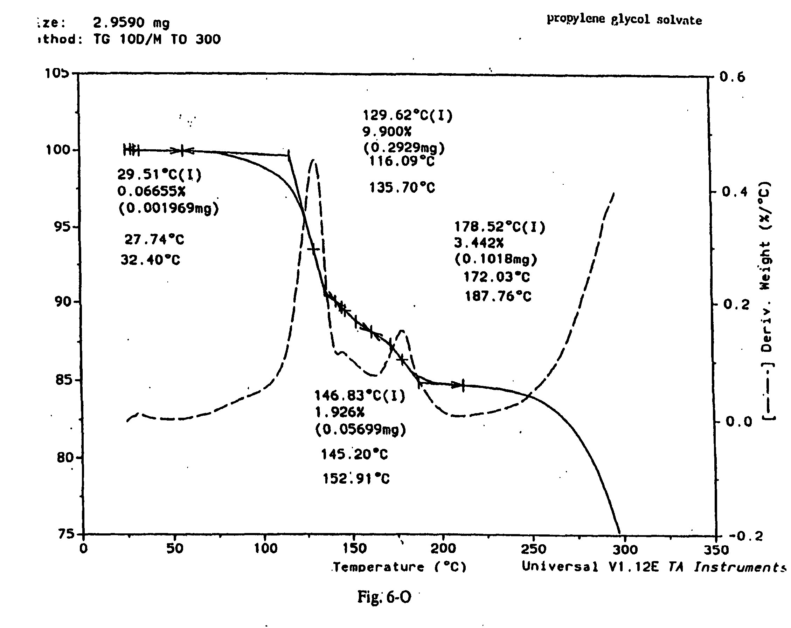

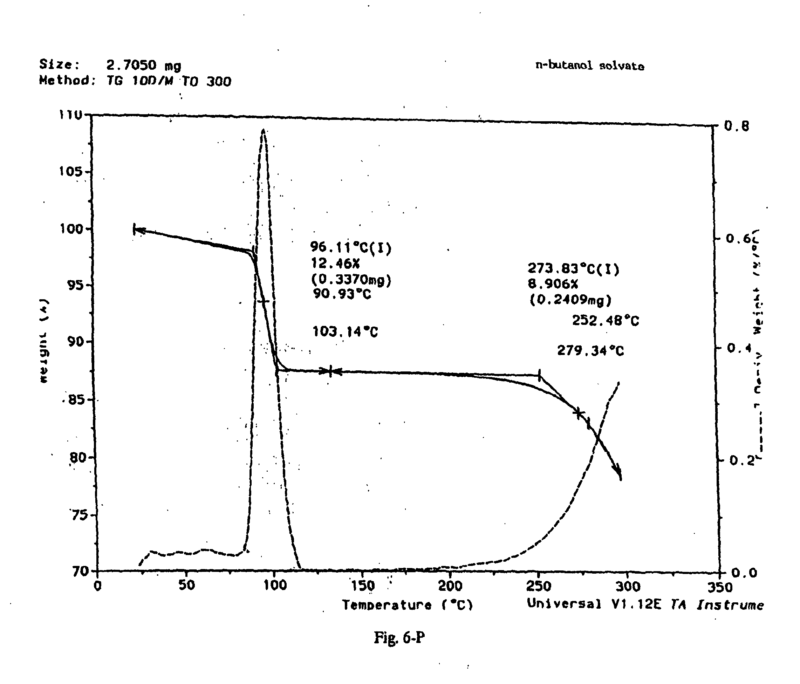

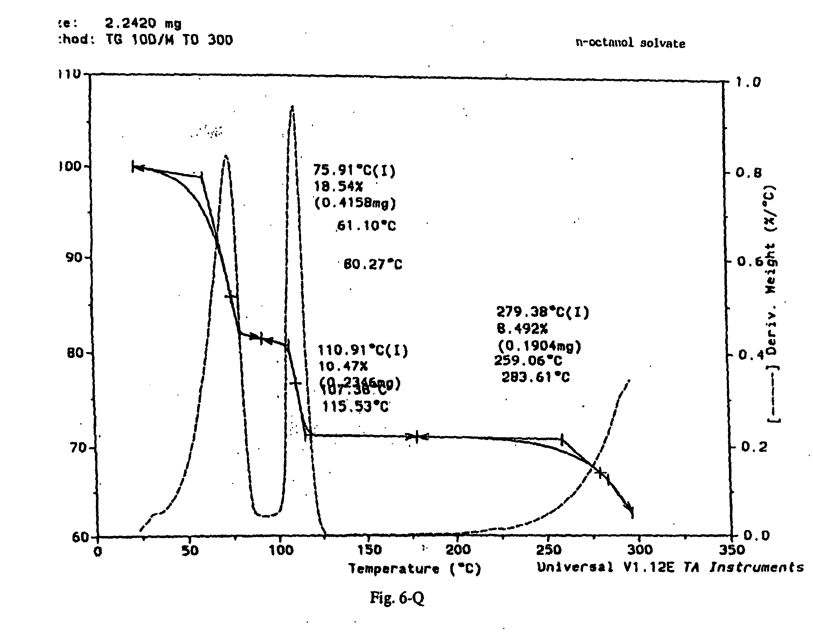

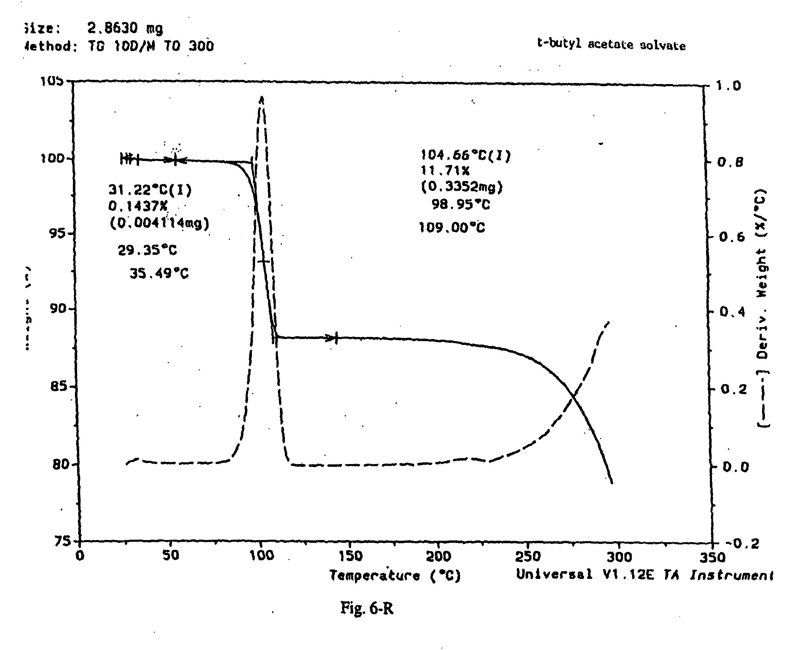

Examples of thermogravimetry analysis profiles are shown in Figs. 6-A through 6-R for the following solvated crystalline forms of eplerenone: methyl ethyl ketone solvate, n-propyl alcohol solvate, tetrahydrofuran solvate, ethyl propionate solvate, acetic acid solvate, chloroform solvate, acetone solvate, toluene solvate, isopropanol solvate, ethanol solvate, isobutyl acetate solvate, butyl acetate solvate, methyl acetate solvate, propyl acetate solvate, propylene glycol solvate, n-butanol solvate, n-octanol solvate, and t-butyl acetate solvate, respectively.

### 7. Microscopy

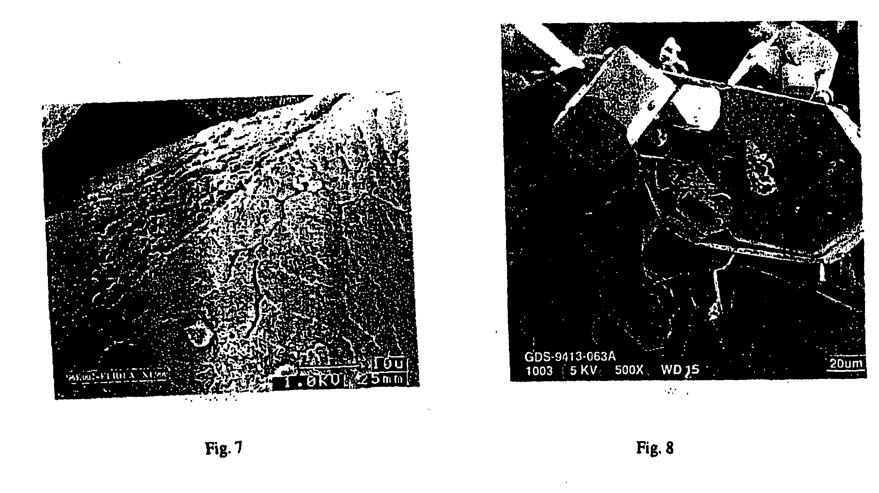

Hot-stage microscopy was performed on single crystals of the methyl ethyl ketone solvate of eplerenone using a Linkam THMS 600 Hot Stage with Zeiss Universal Polarized Light Microscope. Under polarized light at room temperature the solvate crystal was birefringent and translucent indicating that the crystal lattice was highly ordered. As the temperature increased to about 60°C, noticeable defects began to emerge along the long crystal dimension. A scanning electron micrograph of Form L eplerenone obtained by desolvation of the methyl ethyl ketone solvate is shown in Fig. 7 and reveals surface defects, pores, cracks and fractures within the crystal lattice. A scanning electron micrograph of Form L eplerenone obtained by direct crystallization from ethyl acetate is shown in Fig. 8 and does not exhibit similar surface defects, pores, cracks and fractures within the crystal lattice.

### 8. Unit cell parameters

Tables 3A, 3B and 3C below summarize the unit cell parameters determined for Form H, Form L, and several solvated crystalline forms of eplerenone.

**Table 3A:**

| **Unit cell parameters for eplerenone crystal forms** | | | |
|---|---|---|---|
| **Parameter** | **Form H** | **Form L** | **Methyl ethyl ketone solvate** |
| Crystal system | Orthorhombic | Monoclinic | Orthorhombic |
| Space group | P2₁2₁2₁ | P2₁ | P2₁2₁2₁ |
| a | 21.22 Å | 8.78 Å | 23.53 Å |
| b | 15.40 Å | 11.14 Å | 8.16 Å |
| c | 6.34 Å | 11.06 Å | 13.08 Å |
| α | 90° | 90° | 90° |
| β | 90° | 93.52° | 90° |
| γ | 90° | 90° | 90° |
| Z | 4 | 2 | 4 |
| Volume (Å) | 2071.3 | 1081.8 | 2511.4 |
| p (calculated) | 1.329 g/cm³ | 1.275 g/cm³ | 1.287 g/cm³ |
| R | 0.0667 | 0.062 | 0.088 |

**Table 3B:**

| **Unit cell parameters for eplerenone crystal forms** | | | |
|---|---|---|---|
| **Parameter** | **Acetone solvate** | **Toluene solvate** | **Butyl acetate solvate**^{**1**} |
| Crystal system | Orthorhombic | Orthorhombic | Orthorhombic |
| Space group | P2₁2₁2₁ | P2₁2₁2₁ | P2₁2₁2₁ |
| a | 23.31 Å | 23.64 Å | 23.07 Å |
| b | 13.13 Å | 13.46 Å | 13.10 Å |
| c | 8.28 Å | 8.16 Å | 8.24 Å |
| α | 90° | 90° | 90° |
| β | 90° | 90° | 90° |
| γ | 90° | 90° | 90° |
| Z | 4 | 4 | 4 |
| Volume (Å) | 2533.7 | 2596.6 | 2490.0 |
| p (calculated) | 1.239 g/cm³ | 1.296 g/cm³ | 1.334 g/cm³ |
| R | 0.058 | 0.089 | 0.093 |

| | | | |
|---|---|---|---|
| ¹The butyl acetate solvate molecules were not completely refined due to disorder of the solvent molecules in the channels. | | | |

**Table 3C:**

| **Unit cell parameters for eplerenone crystal forms** | | | |
|---|---|---|---|
| **Parameter** | **Isobutyl acetate solvate**^{**1**} | **Isopropanol solvate**^{**1**} | **Ethanol solvate**^{**1**} |
| Crystal system | Orthorhombic | Orthorhombic | Orthorhombic |
| Space group | P2₁2₁2₁ | P2₁2₁2₁ | P2₁2₁2₁ |
| a | 23.19 Å | 23.15 Å | 23.51 Å |
| b | 12.95 Å | 12.73 Å | 13.11 Å |
| c | 8.25 Å | 8.25 Å | 8.27 Å |
| α | 90° | 90° | 90° |
| β | 90° | 90° | 90° |
| γ | 90° | 90° | 90° |
| Z | 4 | 4 | 4 |
| Volume (Å) | 2476.4 | 2433.2 | 2548.6 |
| p (calculated) | 1.337 g/cm³ | 1.296 g/cm³ | 1.234 g/cm³ |
| R | 0.098 | 0.152 | 0.067 |

| | | | |
|---|---|---|---|
| ¹The solvate molecules were not completely refined due to disorder of the solvent molecules in the channels. | | | |

Additional information on selected solvated crystalline forms of eplerenone is reported in Table 4 below. The unit cell data reported in Table 3A above for the methyl ethyl ketone solvate also are representative of the unit cell parameters for many of these additional eplerenone crystalline solvates. Most of the eplerenone crystalline solvates tested are substantially isostructural to each other. While there may be some minor shifting in the X-ray powder diffraction peaks from one solvated crystalline form to the next due to the size of the incorporated solvent molecule, the overall diffraction patterns are substantially the same and the unit cell parameters and molecular positions are substantially identical for most of the solvates tested.

**Table 4:**

| **Additional information on eplerenone solvates** | | | |
|---|---|---|---|
| **Solvent** | **Stoichiometry solvent: eplerenone** | **Isostructural to methyl ethyl ketone solvate?** | **Desolvation temperature**^{**1**} **(°C)** |
| Methyl ethyl ketone | 1:1 | | 89 |
| Acetic acid | 1:2 | yes | 203 |
| Acetone | 1:1 | yes | 117 |
| Methyl acetate | 1:1 | yes | 103 |
| Propyl acetate | 1:1 | yes | 130 |
| Butyl acetate | 1:2 | yes | 108 |
| Isobutyl acetate | 1:2 | yes | 112 |
| t-Butyl acetate | --- | yes | 109 |
| Chloroform | --- | yes | 125 |
| Ethanol | 1:1 | yes | 166 |
| n-Propanol | 1:1 | yes | 129 |
| Isopropanol | 1:1 | yes | 121 |
| n-Butanol | 1:1 | yes | 103 |
| n-Octanol | --- | yes | 116 |
| Ethyl propionate | 1:1 | yes | 122 |
| Propylene glycol | --- | yes | 188 |
| Tetrahydrofuran | 1:1 | yes | 136 |
| Toluene | 1:1 | yes | 83 |

| | | | |
|---|---|---|---|
| ¹Defined as the extrapolated desolvation temperature from the final solvent weight loss step as determined by thermogravimetric analysis at a heating rate of 10°C/minute under nitrogen purge. Desolvation temperatures, however, can be affected by the method of manufacture of the solvate. Different methods can produce different numbers of nucleation sites capable of initiating desolvation in the solvate at lower temperatures. | | | |

The unit cell of the solvate is composed of four eplerenone molecules. The stoichiometry of the eplerenone molecules and solvent molecules in the unit cell is also reported in Table 4 above for a number of solvates. The unit cell of Form H is composed of four eplerenone molecules. The unit cell of Form L is composed of two eplerenone molecules. The solvate unit cells are converted during desolvation into Form H and/or Form L unit cells when the eplerenone molecules undergo translation and rotation to fill the spaces left by the solvent molecules. Table 4 also reports the desolvation temperatures for a number of different solvates.

### 9. Crystal properties of impurities

Selected impurities in eplerenone can induce the formation of Form H during desolvation of a solvate. In particular, the effect of the following two impurity molecules was evaluated: 7-methyl hydrogen 4α,5α;9α,11α-diepoxy-17 hydroxy-3-oxo-17α-pregnane-7α,21-dicarboxylate, γ-lactone (III) (the "diepoxide"); and 7-methyl hydrogen 11α,12α-epoxy-17-hydroxy-3-oxo-17α-pregn-4-ene-7α,21-dicarboxylate, γ-lactone (IV) (the "11,12-epoxide"). 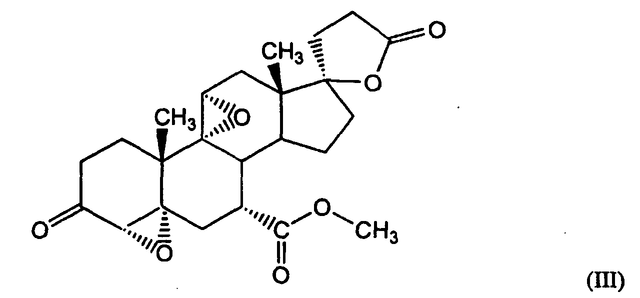 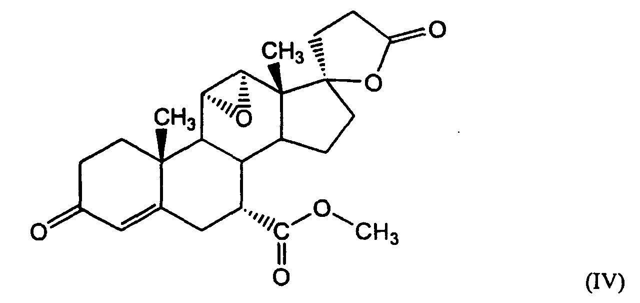 The effect of these impurities on the eplerenone crystalline form resulting from desolvation is described in greater detail in the Examples herein.

Given the similarity in single crystal structure of 7-methyl hydrogen 17-hydroxy-3-oxo-17α-pregna-4,9(11)-diene-7α,21-dicarboxylate, γ-lactone (V) (the "9,11-olefin") and Form H eplerenone, it is hypothesized that the 9,11-olefin also can induce the formation of Form H during the desolvation of the solvate.

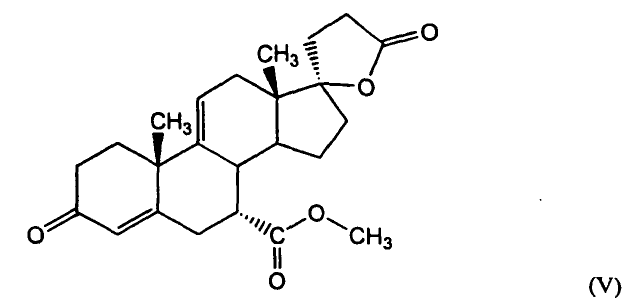

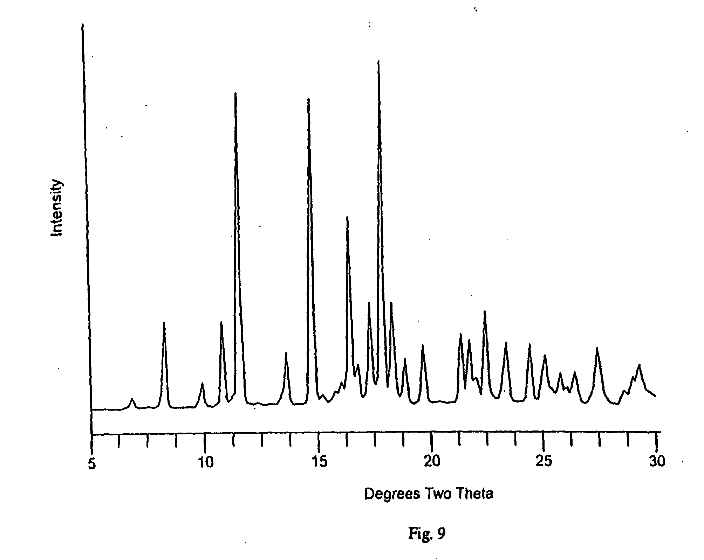

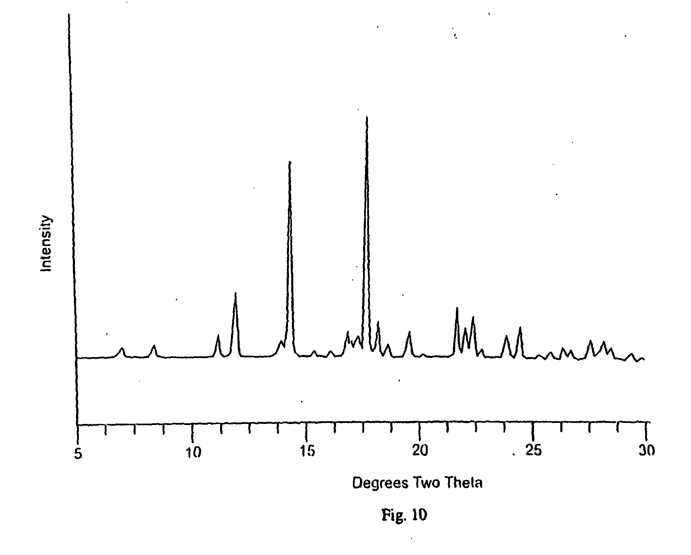

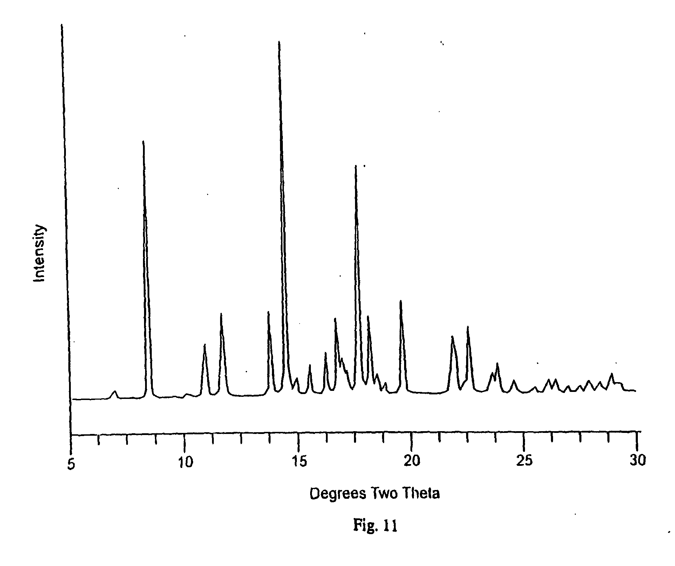

A single crystal form was isolated for each impurity compound. Representative X-ray powder diffraction patterns for the crystal forms isolated for the diepoxide, 11,12-epoxide and 9,11-olefin are given in Figs. 9, 10 and 11, respectively. The X-ray powder diffraction pattern of each impurity molecule is similar to the X-ray powder diffraction pattern of Form H, suggesting that Form H and the three impurity compounds have similar single crystal structures.

Single crystals of each impurity compound also were isolated and subjected to X-ray structure determination to verify that these three compounds adopt single crystal structures similar to that of Form H. Single crystals of the diepoxide were isolated from methyl ethyl ketone. Single crystals of the 11,12-epoxide were isolated from isopropanol. Single crystals of the 9,11-olefin were isolated from n-butanol. Crystal structure data determined for the crystalline form of each impurity compound are given in Table 5. The resulting crystal system and cell parameters were substantially the same for the Form H, diepoxide, 11,12-epoxide, and 9,11-olefin crystalline forms.

**Table 5:**

| **Unit cell parameters for crystals of impurities by comparison with Form H eplerenone** | | | | |
|---|---|---|---|---|
| **Parameter** | **Form H** | **Diepoxide** | **11,12-epoxide** | **9,11-olefin** |
| Crystal system | Orthorhombic | Orthorhombic | Orthorhombic | Orthorhombic |
| Space group | P2₁2₁2₁ | P2₁2₁2₁ | P2₁2₁2₁ | P2₁2₁2₁ |
| a | 21.22 Å | 21.328 Å | 20.90 Å | 20.90 Å |
| b | 15.40 Å | 16.16 Å | 15.55 Å | 15.74 Å |
| c | 6.34 Å | 6.15 Å | 6.38 Å | 6.29 Å |
| α | 90° | 90° | 90° | 90° |
| β | 90° | 90° | 90° | 90° |
| γ | 90° | 90° | 90° | 90° |
| Z | 4 | 4 | 4 | 4 |
| Volume (Å) | 2071.3 | 2119.0 | 2073.2 | 2069.3 |
| p (calculated) | 1.329 g/cm³ | 1.349 g/cm³ | 1.328 g/cm³ | 1.279 g/cm³ |
| R | 0.0667 | 0.0762 | 0.0865 | 0.0764 |

The four compounds reported in Table 5 crystallize into the same space group and have similar cell parameters (*i*.*e*., they are isostructural). It is hypothesized that the diepoxide, 11,12-epoxide and 9,11-olefin adopt a Form H conformation. The relative ease of isolation of a Form H packing (directly from solution) for each impurity compound indicates that the Form H lattice is a stable packing mode for this series of structurally similar compounds. It is contemplated that any compound that is substantially crystallographically isostructural to Form H eplerenone can be useful as a dopant in crystallizing Form H eplerenone from solution.

Accordingly, in a particular embodiment, there is provided a method for promoting crystallization of Form H eplerenone from a solution of eplerenone in a solvent or mixture of solvents, the method comprising doping the solution prior to crystallization with an effective amount of a compound that is crystallographically substantially isostructural to Form H eplerenone. It is to be understood that "doping" herein can be active, *i*.*e*., deliberate addition of the doping compound to the solution, or passive, *i*.*e*., arising from the presence of the doping compound as an impurity in the solution.

Preferred doping compounds according to this embodiment are the diepoxide, the 11,12-epoxide, and the 9,11-olefin, *i*.*e*., compounds (III), (IV) and (V) respectively, above.

### Preparation of eplerenone

The eplerenone starting material used to prepare the novel crystalline forms of the present invention can be prepared by methods known *per se,* including the methods set forth in above-cited International Patent Publications No. WO 97/21720 and No. WO 98/25948, particularly scheme 1 set forth in each of these publications.

### Preparation of crystalline forms

### 1. Preparation of solvated crystalline form

The solvated crystalline forms of eplerenone can be prepared by crystallization of eplerenone from a suitable solvent or a mixture of suitable solvents. A suitable solvent or mixture of suitable solvents generally comprises an organic solvent or a mixture of organic solvents that solubilizes the eplerenone together with any impurities at an elevated temperature, but upon cooling, preferentially crystallizes the solvate. The solubility of eplerenone in such solvents or mixtures of solvents generally is about 5 to about 200 mg/ml at room temperature. The solvent or mixtures of solvents preferably are selected from those solvents previously used in the process to prepare the eplerenone starting material, particularly those solvents that would be pharmaceutically acceptable if contained in the final pharmaceutical composition comprising the eplerenone crystalline form. For example, a solvent system comprising methylene chloride that yields a solvate comprising methylene chloride generally is not desirable.

Each solvent used preferably is a pharmaceutically acceptable solvent, particularly a Class 2 or Class 3 solvent as defined in "Impurities: guideline for residual solvents", International Conference On Harmonisation Of Technical Requirements For Registration Of Pharmaceuticals For Human Use (recommended for adoption at Step 4 of the ICH Process on July 17, 1997 by the ICH Steering Committee). Still more preferably, the solvent or mixture of solvents is selected from the group consisting of methyl ethyl ketone, 1-propanol, 2-pentanone, acetic acid, acetone, butyl acetate, chloroform, ethanol, isobutanol, isobutyl acetate, methyl acetate, ethyl propionate, n-butanol, n-octanol, isopropanol, propyl acetate, propylene glycol, t-butanol, tetrahydrofuran, toluene, methanol and t-butyl acetate. Still more preferably, the solvent is selected from the group consisting of methyl ethyl ketone and ethanol.

In another embodiment of this process, the solvent or mixture of solvents is selected from the group consisting of 1-propanol, 2-pentanone, acetic acid, acetone, butyl acetate, chloroform, isobutanol, isobutyl acetate, methyl acetate, ethyl propionate, n-butanol, n-octanol, propyl acetate, propylene glycol, t-butanol, tetrahydrofuran, toluene, methanol and t-butyl acetate.

In another embodiment of this process, the solvent or mixture of solvents is selected from the group consisting of 1-propanol, 2-pentanone, acetic acid, acetone, butyl acetate, chloroform, isobutanol, isobutyl acetate, methyl acetate, ethyl propionate, n-butanol, n-octanol, n-propanol, propyl acetate, propylene glycol, t-butanol, tetrahydrofuran, toluene, methanol and t-butyl acetate.

To prepare the solvated crystalline form of eplerenone, an amount of the eplerenone starting material is solubilized in a volume of the solvent and cooled until crystals form. The solvent temperature at which the eplerenone is added to the solvent generally will be selected based upon the solubility curve of the solvent or mixture of solvents. For most of the solvents described herein, for example, this solvent temperature typically is at least about 25°C, preferably from about 30°C to the boiling point of the solvent, and more preferably from about 25°C below the boiling point of the solvent to the boiling point of the solvent.

Alternatively, hot solvent may be added to the eplerenone and the mixture can be cooled until crystals form. The solvent temperature at the time it is added to the eplerenone generally will be selected based upon the solubility curve of the solvent or mixture of solvents. For most of the solvents described herein, for example, the solvent temperature typically is at least 25°C, preferably from about 50°C to the boiling point of the solvent, and more preferably from about 15°C below the boiling point of the solvent to the boiling point of the solvent.

The amount of the eplerenone starting material mixed with a given volume of solvent likewise will depend upon the solubility curve of the solvent or mixture of solvents. Typically, the amount of eplerenone added to the solvent will not completely solubilize in that volume of solvent at room temperature. For most of the solvents described herein, for example, the amount of eplerenone starting material mixed with a given volume of solvent usually is at least about 1.5 to about 4.0 times, preferably about 2.0 to about 3.5 times, and more preferably about 2.5 times, the amount of eplerenone that will solubilize in that volume of solvent at room temperature.

After the eplerenone starting material has completely solubilized in the solvent, the solution typically is cooled slowly to crystallize the solvated crystalline form of eplerenone. For most of the solvents described herein, for example, the solution is cooled at a rate slower than about 20°C/minute, preferably at a rate of about 10°C/minute or slower, more preferably at a rate of about 5°C/minute or slower, and still more preferably at a rate of about 1°C/minute or slower.

The endpoint temperature at which the solvated crystalline form is harvested will depend upon the solubility curve of the solvent or mixture of solvents. For most of the solvents described herein, for example, the endpoint temperature typically is less than about 25°C, preferably less than about 5°C, and more preferably less than about -5°C. Decreasing the endpoint temperature generally favors the formation of the solvated crystalline form.

Alternatively, other techniques may be used to prepare the solvate. Examples of such techniques include, but are not limited to, (i) dissolving the eplerenone starting material in one solvent and adding a co-solvent to aid in crystallization of the solvate crystalline form, (ii) vapor diffusion growth of the solvate, (iii) isolation of the solvate by evaporation, such as rotary evaporation, and (iv) slurry conversion.

The crystals of the solvated crystalline form prepared as described above can be separated from the solvent by any suitable conventional means such as by filtration or centrifugation. Increased agitation of the solvent system during crystallization generally results in smaller crystal particle sizes.

### 2. Preparation of Form L from solvate

Form L eplerenone can be prepared directly from the solvated crystalline form by desolvation. Desolvation can be accomplished by any suitable desolvation means such as, but not limited to, heating the solvate, reducing the ambient pressure surrounding the solvate, or combinations thereof. If the solvate is heated to remove the solvent, such as in an oven, the temperature of the solvate during this process typically does not exceed the enantiotropic transition temperature for Form H and Form L. This temperature preferably does not exceed about 150°C.

The desolvation pressure and time of desolvation are not narrowly critical. The desolvation pressure preferably is about one atmosphere or less. As the desolvation pressure is reduced, however, the temperature at which the desolvation can be carried out and/or the time of desolvation likewise is reduced. Particularly for solvates having higher desolvation temperatures, drying under vacuum will permit the use of lower drying temperatures. The time of desolvation need only be sufficient to allow for the desolvation, and thus the formation of Form L, to reach completion.

To ensure the preparation of a product that comprises substantially all Form L, the eplerenone starting material typically is a high purity eplerenone, preferably substantially pure eplerenone. The eplerenone starting material used to prepare Form L eplerenone generally is at least 90% pure, preferably at least 95% pure, and more preferably at least 99% pure. As discussed in greater detail elsewhere in this application, certain impurities in the eplerenone starting material can adversely affect the yield and Form L content of the product obtained from the process.

The crystallized eplerenone product prepared in this manner from a high purity eplerenone starting material generally comprises at least 10% Form L, preferably at least 50% Form L, more preferably at least 75% Form L, still more preferably at least 90% Form L, still more preferably at least about 95% Form L, and still more preferably substantially phase pure Form L.

### 3. Preparation of Form H from solvate

A product comprising Form H can be prepared in substantially the same manner as set forth above for the preparation of Form L by (i) using a low purity eplerenone starting material instead of a high purity eplerenone starting material, (ii) seeding the solvent system with phase pure Form H crystals, or (iii) a combination of (i) and (ii).

### 3.1. Use of impurities as crystal growth promoters and inhibitors

The presence and amount of selected impurities in the eplerenone starting material, rather than the total amount of all impurities in the eplerenone starting material, affect the potential for Form H crystal formation during desolvation of the solvate. The selected impurity generally is a Form H growth promoter or a Form L growth inhibitor. It may be contained in the eplerenone starting material, contained in the solvent or mixture of solvents before the eplerenone starting material is added, and/or added to the solvent or mixture of solvents after the eplerenone starting material is added. Bonafede *et al*. (1995), "Selective nucleation and growth of an organic polymorph by ledge-directed epitaxy on a molecular crystal substrate", J. Amer. Chem. Soc., 117(30), incorporated by reference herein, discusses use of growth promoters and growth inhibitors in polymorph systems. For the present invention, a suitable impurity generally comprises a compound having a single crystal structure substantially identical to the single crystal structure of Form H eplerenone. The impurity preferably is a compound having an X-ray powder diffraction pattern substantially identical to the X-ray powder diffraction pattern of Form H eplerenone, and more preferably is selected from the group consisting of the diepoxide, the 11,12-epoxide, the 9,11-olefin and combinations thereof.

The amount of impurity needed to prepare Form H crystals typically can depend, in part, upon the solvent or mixture of solvents and the solubility of the impurity relative to eplerenone. In the crystallization of Form H from a methyl ethyl ketone solvent, for example, the weight ratio of diepoxide to low purity eplerenone starting material typically is at least about 1:100, preferably at least about 3:100, more preferably about 3:100 to about 1:5, and still more preferably about 3:100 to about 1:10. The 11,12-epoxide has a higher solubility in methyl ethyl ketone than the diepoxide and generally requires a larger amount of the 11,12-epoxide to prepare Form H eplerenone crystals. Where the impurity comprises the 11,12-epoxide, the weight ratio of the diepoxide to the low purity eplerenone starting material typically is at least about 1:5, more preferably at least about 3:25, and still more preferably about 3:25 to about 1:5. Where both the diepoxide and the 11,12-epoxide impurities are used in the preparation of the Form H crystals, the weight ratio of each impurity to the eplerenone starting material may be lower than the corresponding ratio when only that impurity is used in the preparation of the Form H crystals.

A mixture of Form H and Form L eplerenone is generally obtained when a solvate comprising the selected impurity is desolvated. The weight fraction of Form H in the product resulting from the initial desolvation of the solvate typically is less than about 50%. Further treatment of this product by crystallization or digestion, as discussed below, generally will increase the weight fraction of Form L in the product.

### 3.2. Seeding

Form H crystals also can be prepared by seeding the solvent system with phase pure Form H crystals (or a Form H growth promoter and/or a Form L growth inhibitor as previously discussed above) prior to crystallization of the eplerenone. The eplerenone starting material can be either a low purity eplerenone or a high purity eplerenone. When the resulting solvate prepared from either starting material is desolvated, the weight fraction of Form H in the product typically is at least about 70% and may be as great as about 100%.

The weight ratio of Form H seed crystals added to the solvent system to the eplerenone starting material added to the solvent system generally is at least about 0.75:100, preferably about 0.75:100 to about 1:20, and more preferably about 1:100 to about 1:50. The Form H seed crystals can be prepared by any of the methods discussed in this application for the preparation of Form H crystals, particularly the preparation of Form H crystals by digestion as discussed below.

The Form H seed crystals may be added at one time, in multiple additions or substantially continually over a period of time. The addition of the Form H seed crystals, however, generally is completed before the eplerenone begins to crystallize from solution, *i*.*e*., the seeding is completed before the cloud point (the lower end of the metastable zone) is reached. Seeding typically is performed when the solution temperature ranges from about 0.5°C above the cloud point to about 10°C above the cloud point, preferably within about 2°C to about 3°C above the cloud point. As the temperature above the cloud point at which the seeds are added increases, the amount of seeding needed for crystallization of Form H crystals generally increases.

The seeding preferably occurs not only above the cloud point, but within the metastable zone. Both the cloud point and the metastable zone are dependent on the eplerenone solubility and concentration in the solvent or mixture of solvents. For a 12 volume dilution of methyl ethyl ketone, for example, the high end of the metastable zone generally is about 70°C to about 73°C and the lower end of the metastable zone (*i.e.*, the cloud point) is about 57°C to about 63°C. For a concentration of 8 volumes of methyl ethyl ketone, the metastable zone is even narrower because the solution is supersaturated. At this concentration, the cloud point of the solution occurs at about 75°C to about 76°C. Because the boiling point of methyl ethyl ketone is about 80°C under ambient conditions, seeding for this solution typically occurs between about 76.5°C and the boiling point.

An illustrative non-limiting example of seeding with Form H is set forth in Example 7 herein.

The crystallized eplerenone product obtained using a Form H growth promoter or Form L growth inhibitor, and/or Form H seeding generally comprises at least 2% Form H, preferably at least 5% Form H, more preferably at least 7% Form H, and still more preferably at least about 10% Form H. The remaining crystallized eplerenone product generally is Form L.

### 3.3. Preparation of Form H by grinding eplerenone

In yet another alternative, it has been discovered that a small amount of Form H can be prepared by suitable grinding eplerenone. Concentrations of Form H in ground eplerenone as high as about 3% have been observed.

### 4. Preparation of Form L from solvate prepared from low purity eplerenone

As discussed above, crystallization of low purity eplerenone to form a solvate followed by desolvation of the solvate generally yields a product comprising both Form H and Form L. A product having a greater Form L content can be prepared from low purity eplerenone in substantially the same manner as set forth above for the preparation of Form H by seeding the solvent system with phase pure Form L crystals, or by using a Form L growth promoter and/or a Form H growth inhibitor. The seeding protocol and the weight ratio of the amount of Form L seed crystals added to the solvent system to the amount of the eplerenone starting material added to the solvent system generally are similar to those ratios previously discussed above for preparation of Form H eplerenone by seeding with phase pure Form H crystals.

The crystallized eplerenone product prepared in this manner generally comprises at least 10% Form L, preferably at least 50% Form L, more preferably at least 75% Form L, more preferably at least 90% Form L, still more preferably at least about 95% Form L, and still more preferably substantially phase pure Form L.

The seeding protocols described herein relating to the preparation of Form H eplerenone also may allow for improved control of the particle size of the crystallized eplerenone.

### 5. Crystallization of Form L directly from solution

Form L eplerenone also can be prepared by direct crystallization of eplerenone from a suitable solvent or mixture of solvents without formation of an intermediate solvate and the accompanying need for desolvation. Typically, (i) the solvent has a molecular size that is incompatible with the available channel space in the solvate crystal lattice, (ii) the eplerenone and any impurities present are soluble in the solvent at elevated temperatures, and (iii) cooling results in crystallization of non-solvated Form L eplerenone. The solubility of eplerenone in the solvent or mixture of solvents generally is about 5 to about 200 mg/ml at room temperature. The solvent or mixture of solvents preferably comprises one or more solvents selected from the group consisting of methanol, ethyl acetate, isopropyl acetate, acetonitrile, nitrobenzene, water and ethyl benzene.

To crystallize Form L eplerenone directly from solution, an amount of the eplerenone starting material is solubilized in a volume of the solvent and cooled until crystals form. The solvent temperature at which the eplerenone is added to the solvent generally will be selected based upon the solubility curve of the solvent or mixture of solvents. For most of the solvents described herein, this solvent temperature typically is at least about 25°C, preferably from about 30°C to the boiling point of the solvent, and more preferably from about 25°C below the boiling point of the solvent to the boiling point of the solvent.

Alternatively, hot solvent can be added to the eplerenone and the mixture cooled until crystals form. The solvent temperature at the time it is added to the eplerenone generally is selected based upon the solubility curve of the solvent or mixture of solvents. For most of the solvents described herein, this solvent temperature typically is at least 25°C, preferably from about 50°C to the boiling point of the solvent, and more preferably from about 15°C below the boiling point of the solvent to the boiling point of the solvent.

The amount of the eplerenone starting material mixed with a given volume of solvent likewise will depend upon the solubility curve of the solvent or mixture of solvents. Typically, the amount of eplerenone added to the solvent will not completely dissolve in that volume of solvent at room temperature. For most of the solvents described herein, the amount of eplerenone starting material mixed with a given volume of solvent usually is about 1.5 to about 4.0 times, preferably about 2.0 to about 3.5 times, for example about 2.5 times, the amount of eplerenone that will dissolve in that volume of solvent at room temperature.

To ensure preparation of a product that comprises substantially phase pure Form L, the eplerenone starting material generally is a high purity eplerenone. The eplerenone starting material preferably is at least about 65% pure, more preferably at least about 90% pure, still more preferably at least about 98% pure, and most preferably at least about 99% pure.

After the eplerenone starting material has completely dissolved in the solvent, the solution typically is cooled slowly to crystallize Form L eplerenone. For most of the solvents described herein, for example, the solution is cooled at a rate slower than about 1°C/minute, preferably at a rate of about 0.2°C/minute or slower, and more preferably at a rate of about 0.05°C/minute to about 0.1°C/minute.

The endpoint temperature at which the Form L crystals are harvested will depend upon the solubility curve of the solvent or mixture of solvents. For most of the solvents described herein, the endpoint temperature typically is less than about 25°C, preferably less than about 5°C, and more preferably less than about -5°C.

Alternatively, other techniques can be used to prepare Form L eplerenone crystals. Examples of such techniques include, but are not limited to, (i) dissolving the eplerenone starting material in one solvent and adding a co-solvent to aid in crystallization of Form L eplerenone, (ii) vapor diffusion growth of Form L eplerenone, (iii) isolation of Form L eplerenone by evaporation, such as rotary evaporation, and (iv) slurry conversion.

Crystals of Form L eplerenone prepared as described above can be separated from the solvent by any suitable conventional means such as by filtration or centrifugation.

In addition, Form L eplerenone can be prepared by digesting (as described below) a slurry of high purity eplerenone in methyl ethyl ketone and filtering the digested eplerenone at the boiling point of the slurry.

### 6. Preparation of Form H directly from solution

It is hypothesized that if crystallization is performed above the enantiotropic transition temperature (Tₜ) for Form H and Form L, particularly if Form H growth promoters or Form L growth inhibitors are present or the solvent is seeded with phase pure Form H crystals, Form H will crystallize directly from solution since Form H is more stable at these higher temperatures. The solvent system used preferably comprises a high boiling solvent such as nitrobenzene. Suitable Form H growth promoters include, but are not limited to, the diepoxide and 11,12-olefin compounds defined hereinabove.

### 7. Digestion of eplerenone with a solvent

The solvated crystalline forms, Form H and Form L of eplerenone also can be prepared by digestion of an eplerenone starting material in a suitable solvent or mixture of solvents. In the digestion process, a slurry of eplerenone is heated at the boiling point of the solvent or mixture of solvents. For example, an amount of eplerenone starting material is combined with a volume of solvent or mixture of solvents, heated to reflux, and the distillate is removed while an additional amount of the solvent is added simultaneously with the removal of the distillate. Alternatively, the distillate can be condensed and recycled without addition of more solvent during the digestion process. Typically, once the original volume of solvent has been removed or condensed and recycled, the slurry is cooled and solvated crystals form. The solvated crystals can be separated from the solvent by any suitable conventional means such as by filtration or centrifugation. Desolvation of the solvate as previously described yields either Form H or Form L eplerenone depending upon the presence or absence of selected impurities in the solvated crystals.

A suitable solvent or mixture of solvents generally comprises one or more of the solvents previously disclosed herein. The solvent can be selected, for example, from the group consisting of methyl ethyl ketone and ethanol.

The amount of eplerenone starting material added to the solvent used in the digestion process generally is sufficient to maintain a slurry (i.e., the eplerenone in the solvent or mixture of solvents is not completely solubilized) at the boiling point of the solvent or mixture of solvents. Illustratively, eplerenone concentrations of about 0.25 g/ml in methyl ethyl ketone or about 0.125 g/ml in ethanol can be useful.

The slurry generally is cooled slowly once solvent turnover is complete to crystallize the solvated crystalline form of eplerenone. For the solvents tested, the slurry is cooled at a rate slower than about 20°C/minute, preferably about 10°C/minute or slower, more preferably about 5°C/minute or slower, and still more preferably about 1°C/minute or slower.

The endpoint temperature at which the solvated crystalline form is harvested will depend upon the solubility curve of the solvent or mixture of solvents. For most of the solvents described herein, the endpoint temperature typically is less than about 25°C, preferably less than about 5°C, and more preferably less than about -5°C.

If a product comprising primarily or exclusively Form L is desired, a high purity eplerenone starting material typically is digested. The high purity eplerenone starting material preferably is at least about 98% pure, more preferably at least about 99% pure, and still more preferably at least about 99.5% pure. The digested eplerenone product prepared in this manner generally comprises at least about 10%, preferably at least about 50%, more preferably at least about 75%, still more preferably at least about 90%, still more preferably at least about 95% Form L, and most preferably substantially phase pure Form L.

If a product comprising primarily or exclusively Form H is desired, a low purity eplerenone starting material typically is digested. The low purity eplerenone starting material generally contains only as much Form H growth promoter and/or Form L growth inhibitor as is needed to yield Form H. Preferably, the low purity eplerenone starting material is at least about 65% pure, more preferably at least about 75% pure, and still more preferably at least about 80% pure. The digested eplerenone product prepared in this manner generally comprises at least about 10%, preferably at least about 50%, more preferably at least about 75%, still more preferably at least about 90%, still more preferably at least about 95% Form H, and most preferably substantially phase pure Form H.

### 8. Preparation of amorphous eplerenone

Amorphous eplerenone can be prepared in small quantities by suitable comminution of solid eplerenone, such as by crushing, grinding and/or micronizing. Phase pure amorphous eplerenone, i.e., amorphous eplerenone substantially free of crystalline eplerenone, can be prepared, for example, by lyophilizing a solution of eplerenone, particularly an aqueous solution of eplerenone. These processes are illustrated in Examples 13 and 14 herein.

### Additional processing considerations

### 1. Thermodynamic stability considerations

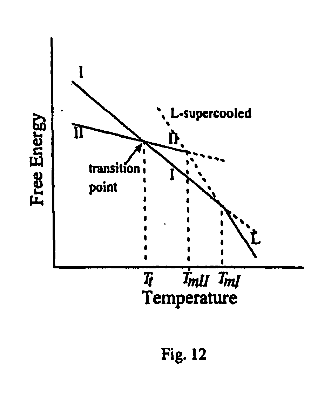

Form L is more thermodynamically stable than Form H at ambient temperatures. As described in Example 5 herein, when an organic slurry containing equivalent amounts of Form H and Form L was allowed to stand overnight at room temperature and the residual solids were then collected and analyzed by X-ray powder diffraction, the analytical results indicated that the eplerenone had completely converted to Form L. Differential scanning calorimetry (DSC) data discussed hereinabove indicate that Form H is more thermodynamically stable than Form L at higher temperatures since it has the higher melting/decomposition temperature. Taken together, the slurry conversion and DSC data indicate that Form H and Form L are related enantiotropically, *i*.*e*., a change in the stability relationship between the two polymorphs occurs around a transition temperature (Tₜ), with Form L being the more stable at lower temperatures. Fig. 12 shows the relationship of Gibbs free energy to temperature generally observed for enantiotropically related polymorphs such as Form H and Form L eplerenone, wherein I and II refer to Forms H and L respectively, Tₜ refers to the transition temperature, Tₘ refers to the melting points of Form H and Form L, and L refers to the liquid or melt state.

Accordingly, processing temperatures preferably are maintained below the transition temperature during preparation of compositions comprising Form L. For example, drying temperatures employed for desolvation typically are less than about 150°C, preferably less than about 125°C, more preferably less than about 115°C, more preferably less than about 110°C, and still more preferably about 80°C to about 110°C. In addition, cooling (such as by use of liquid nitrogen) may be necessary during particle size reduction process steps to maintain the temperature of the Form L crystals below the transition temperature.

### 2. Intrinsic micronizing considerations

The method used to prepare crystalline eplerenone may affect properties of the resulting crystal form. For example, Form L prepared by desolvation of a solvate exhibits a higher incidence of surface defects, pores, cracks and fractures within the crystal lattice than Form L prepared by direct crystallization from solution. This "intrinsic micronizing" of the desolvated crystal results in an increase in both the available surface area of the crystal and the dissolution rate of the crystal. Dissolution time, therefore, can be shortened by selection of Form L crystals prepared by desolvation, lengthened by selection of Form L crystals prepared by direct crystallization, or otherwise adjusted by selection of an appropriate combination of Form L crystals prepared by desolvation and Form L crystals prepared by direct crystallization.

Intrinsic micronizing also can effectively reduce or eliminate need for crystal particle size reduction during processing steps where Form L crystals prepared by desolvation are used in the preparation of the pharmaceutical composition. One disadvantage of using such Form L crystals, however, is the need for a desolvation step that is not required for Form L crystals prepared by direct crystallization.

### Product-by-process solid state forms

Embodiments of the present invention also include specific solid state eplerenone forms and combinations thereof prepared in accordance with the processes disclosed in this application. In particular, Form H eplerenone, alone or in combination with one or more additional solid state forms (including solvated crystal forms, Form L and amorphous eplerenone, prepared as set forth in this application, is an embodiment of the present invention. Further, solvated crystal forms useful as intermediates in preparation of Form H eplerenone by desolvation, and prepared as set forth in this application, are embodiments of the present invention.

### Combinations of solid state forms

In combinations comprising a first solid state form of eplerenone and a second solid state form of eplerenone, wherein the first and second solid state forms of eplerenone are selected from Form H, Form L, solvated eplerenone and amorphous eplerenone, any suitable weight ratio of the first to the second solid state form can be used. In general in such combinations, the weight ratio of the first to the second solid state form preferably is about 1:99 to about 99:1, and is more preferably at least about 1:9, more preferably at least about 1:1, more preferably at least about 2:1, more preferably at least about 5:1, and most preferably at least about 9:1.

According to an embodiment of the present invention, the first solid state form is Form H and the second solid state form is Form L.

In another embodiment, a third solid state form is also present.

### Eplerenone particle size

Although each of the above solid state forms of eplerenone and combinations thereof can embrace a broad range of eplerenone particle sizes, it has been discovered that reduction of the particle size of a solid state form of eplerenone to a D₉₀ particle size of less than about 400 µm can improve bioavailability of unformulated eplerenone and of pharmaceutical compositions comprising that solid state form of eplerenone. Accordingly, the D₉₀ particle size of the unformulated eplerenone or of the eplerenone used as a starting material in preparing a pharmaceutical composition generally is less than about 400 µm, preferably less than about 200 µm, more preferably less than about 150 µm, still more preferably less than about 100 µm, and still more preferably less than about 90 µm.

In one embodiment, the D₉₀ particle size is not less than about 25 µm. A D₉₀ particle size in the range of about 25 to about 400 µm will generally be found to have acceptable bioavailability for most purposes, and avoids the cost and increased need for environmental emission control associated with milling to smaller dimensions. Acceptable bioavailability in this size range is especially obtainable where a substantial fraction of the eplerenone is present as Form H eplerenone, due at least in part to the higher dissolution rate of that crystal form. A suitable range of D₉₀ particle size according to this embodiment is about 40 to about 100 µm. Another suitable range is about 30 to about 50 µm. Yet another suitable range is about 50 to about 150 µm. Still another suitable range is about 75 to about 125 µm.

Any milling, grinding, micronizing or other particle size reduction method known in the art can be used to bring the solid state eplerenone into any desired size range as set forth above. For example, air-jet or fragmentation milling can be effective for this purpose.

Where highest possible bioavailability is desired with less regard to cost, it has been discovered that reduction of the particle size of a solid state form of eplerenone to a D₉₀ particle size of less than about 15 µm can further enhance bioavailability of unformulated eplerenone and of pharmaceutical compositions comprising that solid state form of eplerenone, even by comparison with D₉₀ particle size ranges defined above. In one embodiment, therefore, the D₉₀ particle size is about 0.01 µm (10 nm) to about 15 µm. Preferably in this embodiment, the D₉₀ particle size is less than about 10 µm, more preferably less than about 1 µm, still more preferably less than about 800 nm, still more preferably less than about 600 nm, and most preferably less than about 400 nm. Depending on the application, a suitable D₉₀ particle size range is about 100 to about 800 nm. Another suitable range is about 200 nm to about 600 nm. Yet another suitable range is about 400 nm to about 800 nm. Still another suitable range is about 500 nm to about 1 µm.

Solid state forms of eplerenone having a D₉₀ particle size less than about 15 µm can be prepared in accordance with applicable particle size reduction techniques known in the art. Such techniques include, but are not limited to, those described in the following patents and publications, each of which is incorporated herein by reference.
U.S. Patent No. 4,826,689 to Violanto & Fischer.
U.S. Patent No. 5,145,684 to Liversidge *et al*.
U.S. Patent No. 5,298,262 to Na & Rajagopalan.
U.S. Patent No. 5,302,401 to Liversidge *et al*.
U.S. Patent No. 5,336,507 to Na & Rajagopalan.
U.S. Patent No. 5,340,564 to Illig & Sarpotdar.
U.S. Patent No. 5,346,702 to Na & Rajagopalan.
U.S. Patent No. 5,352,459 to Hollister *et al*.
U.S. Patent No. 5,354,560 to Lovrecich.
U.S. Patent No. 5,384,124 to Courteille *et al*.
U.S. Patent No. 5,429,824 to June.
U.S. Patent No. 5,503,723 to Ruddy *et al*.
U.S. Patent No. 5,510,118 to Bosch *et al*.
U.S. Patent No. 5,518,187 to Bruno *et al*.
U.S. Patent No. 5,518,738 to Eickhoff *et al*.
U.S. Patent No. 5,534,270 to De Castro.
U.S. Patent No. 5,536,508 to Canal *et al*.
U.S. Patent No. 5,552,160 to Liversidge *et al*.
U.S. Patent No. 5,560,931 to Eickhoff *et al*.
U.S. Patent No. 5,560,932 to Bagchi *et al*.
U.S. Patent No. 5,565,188 to Wong *et al*.
U.S. Patent No. 5,569,448 to Wong *et al*.
U.S. Patent No. 5,571,536 to Eickhoff *et al*.
U.S. Patent No. 5,573,783 to Desieno & Stetsko.
U.S. Patent No. 5,580,579 to Ruddy *et al*.
U.S. Patent No. 5,585,108 to Ruddy *et al*.
U.S. Patent No. 5,587,143 to Wong.
U.S. Patent No. 5,591,456 to Franson *et al*.
U.S. Patent No. 5,622,938 to Wong.
U.S. Patent No. 5,662,883 to Bagchi *et al*.
U.S. Patent No. 5,665,331 to Bagchi *et al*.
U.S. Patent No. 5,718,919 to Ruddy *et al*.
U.S. Patent No. 5,747,001 to Wiedmann *et al*.
International Patent Publication No. WO 93/25190.
International Patent Publication No. WO 96/24336.
International Patent Publication No. WO 98/35666.

In an illustrative process, coarse solid state eplerenone is added to a liquid medium in which it is essentially insoluble to form a premix suspension. The concentration of the eplerenone in the liquid medium can vary from about 0.1% to about 60%, and preferably is about 5% to about 30%, by weight. The apparent viscosity of the premix suspension is preferably less than about 1000 cP.

The premix can be directly subjected to mechanical means, for example using a ball mill, to reduce the D₉₀ particle size of the eplerenone to a desired range. Alternatively, the premix can first be agitated, e.g., using a roller mill or a Cowles type mixer, until a homogeneous dispersion is observed in which there are no large agglomerates visible to the naked eye, and then subjected to attrition, for example using a recirculating media mill.

The particles can be milled in presence of a surface modifying agent, for example a polymer or wetting agent. Alternatively, the particles can be contacted with a surface modifying agent after attrition. The surface modifying agent can reduce agglomeration of the particles, and have other benefits.

The particles should be reduced in size at a temperature that does not significantly degrade the eplerenone. Processing temperatures of less than about 30-40°C are ordinarily preferred. If desired, the processing equipment can be cooled with conventional cooling equipment. The method is conveniently carried out at ambient temperature and at processing pressures that are safe and effective for the milling process. For example, ambient processing pressures are typical of ball mills, attritor mills and vibratory mills. Control of the temperature can be achieved by jacketing or immersion of the milling chamber in ice water. Processing pressures from about 0.07 to about 3.5 kg/cm² are contemplated, with pressures of about 0.7 to 1.4 kg/cm² being typical.

After milling is completed, the grinding medium is separated from milled product, in either a dry or liquid dispersion form, using conventional separation techniques, such as filtration, sieving through a mesh screen or the like.

### Pharmaceutical compositions

Also embraced within this invention is a class of pharmaceutical compositions comprising (i) Form H eplerenone, optionally together with one or more additional solid state forms of eplerenone selected from the group consisting of Form L, solvated crystal forms and amorphous eplerenone, and (ii) one or more pharmaceutically acceptable carriers and/or diluents and/or adjuvants (collectively referred to herein as "excipients") and, optionally, (iii) one or more active ingredients other than eplerenone. In a preferred embodiment, essentially the entire amount of eplerenone contained in the composition is present as phase pure Form H; however, if a combination of solid state forms is present, preferred weight ratios of solid state forms are as set out hereinabove.

Alternatively, essentially the entire amount of eplerenone contained in the composition can present as phase pure solvated crystalline eplerenone or as amorphous eplerenone.

In another embodiment of the invention, the composition comprises both Form H and Form L. The weight ratio of Form L to Form H in the composition generally is about 1:20 to about 20:1. In other embodiments, this weight ratio is about 10:1 to about 1:10; about 5:1 to about 1:5; about 2:1 to about 1:2; illustratively, the weight ratio can be about 1:1.

Compositions of the invention can be adapted to any suitable route of administration, including without limitation oral, buccal, sublingual, parenteral, *e*.*g*., intravascular, intraperitoneal, subcutaneous or intramuscular, topical and rectal (*e*.*g*., by suppository) routes. These compositions comprise eplerenone in a desired amount in combination with one or more pharmaceutically-acceptable excipients appropriate to the desired route of administration.

### 1. Oral compositions and excipients therefor

Oral dosage forms of such compositions preferably comprise one or more excipients selected from the group consisting of diluents, disintegrants, binding agents and adhesives, wetting agents, lubricants and anti-adherent agents. More preferably, such oral dosage forms are tableted or encapsulated for convenient administration. The resulting tablets or capsules can contain an immediate-release formulation and/or a controlled-release formulation as can be provided, for example, in a dispersion of eplerenone in hydroxypropylmethylcellulose (HPMC).

Through appropriate selection and combination of excipients, compositions can be provided exhibiting improved performance with respect to, among other properties, efficacy, bioavailability, clearance time, stability, compatibility of the eplerenone with excipients, safety, dissolution profile, disintegration profile and/or other pharmacokinetic, chemical and/or physical properties. The excipients preferably are water soluble or water dispersible and have wetting properties to offset the low aqueous solubility of the eplerenone. Where the composition is formulated as a tablet, the combination of excipients selected provides tablets that can exhibit, among other properties, improved dissolution and disintegration profiles, hardness, crushing strength and/or friability.

### 1.1. Diluents

Compositions of the invention optionally comprise one or more pharmaceutically acceptable diluents as excipients. Suitable diluents illustratively include, either individually or in combination, lactose, including anhydrous lactose and lactose monohydrate; starches, including directly compressible starch and hydrolyzed starches (*e*.*g*., Celutab™ and Erndex™); mannitol; sorbitol; xylitol; dextrose (*e*.*g*., Cerelose™ 2000) and dextrose monohydrate; dibasic calcium phosphate dihydrate; sucrose-based diluents; confectioner's sugar; monobasic calcium sulfate monohydrate; calcium sulfate dihydrate; granular calcium lactate trihydrate; dextrates; inositol; hydrolyzed cereal solids; amylose; celluloses including microcrystalline cellulose, food grade sources of α- and amorphous cellulose (*e*.*g*., Rexcel™) and powdered cellulose; calcium carbonate; glycine; bentonite; polyvinylpyrrolidone; and the like. Such diluents, if present, constitute in total about 5% to about 99%, preferably about 10% to about 85%, and more preferably about 20% to about 80%, of the total weight of the composition. The diluent or diluents selected preferably exhibit suitable flow properties and, where tablets are desired, compressibility.

Lactose and microcrystalline cellulose, either individually or in combination, are preferred diluents. Both diluents are chemically compatible with eplerenone. The use of extragranular microcrystalline cellulose (that is, microcrystalline cellulose added to a wet granulated composition after a drying step) can be used to improve hardness (for tablets) and/or disintegration time. Lactose, especially lactose monohydrate, is particularly preferred. Lactose typically provides compositions having suitable release rates of eplerenone, stability, pre-compression flowability, and/or drying properties at a relatively low diluent cost. It provides a high density substrate that aids densification during granulation (where wet granulation is employed) and therefore improves blend flow properties.

### 1.2. Disintegrants

Compositions of the invention optionally comprise one or more pharmaceutically acceptable disintegrants as excipients, particularly for tablet formulations. Suitable disintegrants include, either individually or in combination, starches, including sodium starch glycolate (*e*.*g*., Explotab™ of Pen West) and pregelatinized corn starches (*e*.*g*., National™ 1551, National™ 1550, and Colocorn™ 1500), clays (*e*.*g*., Veegum™ HV), celluloses such as purified cellulose, microcrystalline cellulose, methylcellulose, carboxymethylcellulose and sodium carboxymethylcellulose, croscarmellose sodium (*e*.*g*., Ac-Di-Sol™ of FMC), alginates, crospovidone, and gums such as agar, guar, locust bean, karaya, pectin and tragacanth gums.

Disintegrants may be added at any suitable step during the preparation of the composition, particularly prior to granulation or during a lubrication step prior to compression. Such disintegrants, if present, constitute in total about 0.2% to about 30%, preferably about 0.2% to about 10%, and more preferably about 0.2% to about 5%, of the total weight of the composition.

Croscarmellose sodium is a preferred disintegrant for tablet or capsule disintegration, and, if present, preferably constitutes about 0.2% to about 10%, more preferably about 0.2% to about 7%, and still more preferably about 0.2% to about 5%, of the total weight of the composition. Croscarmellose sodium confers superior intragranular disintegration capabilities to granulated compositions of the present invention.

### 1.3. Binding agents

Compositions of the invention optionally comprise one or more pharmaceutically acceptable binding agents or adhesives as excipients, particularly for tablet formulations. Such binding agents and adhesives preferably impart sufficient cohesion to the powder being tableted to allow for normal processing operations such as sizing, lubrication, compression and packaging, but still allow the tablet to disintegrate and the composition to be absorbed upon ingestion. Suitable binding agents and adhesives include, either individually or in combination, acacia; tragacanth; sucrose; gelatin; glucose; starches such as, but not limited to, pregelatinized starches (*e*.*g*., National™ 1511 and National™ 1500); celluloses such as, but not limited to, methylcellulose and sodium carboxymethylcellulose (*e*.*g*., Tylose™); alginic acid and salts of alginic acid; magnesium aluminum silicate; polyethylene glycol (PEG); guar gum; polysaccharide acids; bentonites; polyvinylpyrrolidone (povidone or PVP), for example povidone K-15, K-30 and K-29/32; polymethacrylates; HPMC; hydroxypropylcellulose (*e*.*g*., Klucel™); and ethylcellulose (*e*.*g*., Ethocel™). Such binding agents and/or adhesives, if present, constitute in total about 0.5% to about 25%, preferably about 0.75% to about 15%, and more preferably about 1% to about 10%, of the total weight of the composition.

HPMC is a preferred binding agent used to impart cohesive properties to the powder blend of the eplerenone formulation. HPMC, if present, constitutes in total about 0.5% to about 10%, preferably about 1% to about 8%, and more preferably about 2% to about 4%, of the total weight of the composition. Low molecular weight HPMC having a viscosity of about 2 to about 8 cP typically can be used, although viscosities of about 2 cP to about 6 cP are preferred, particularly viscosities of about 2 cP to about 4 cP. HPMC viscosities are measured as a 2 percent solution in water at 20°C. Methoxy content of the HPMC typically is about 15% to about 35%, whereas hydroxypropyl content is typically up to about 15%, preferably about 2% to about 12%.

### 1.4. Wetting agents

Eplerenone is largely insoluble in aqueous solution. Accordingly, compositions of the invention optionally but preferably comprise one or more pharmaceutically acceptable wetting agents as excipients. Such wetting agents are preferably selected to maintain the eplerenone in close association with water, a condition that is believed to improve the relative bioavailability of the composition.

Non-limiting examples of surfactants that can be used as wetting agents in compositions of the present invention include quaternary ammonium compounds, for example benzalkonium chloride, benzethonium chloride and cetylpyridinium chloride, dioctyl sodium sulfosuccinate, polyoxyethylene alkylphenyl ethers, for example nonoxynol 9, nonoxynol 10, and octoxynol 9, poloxamers (polyoxyethylene and polyoxypropylene block copolymers), polyoxyethylene fatty acid glycerides and oils, for example polyoxyethylene (8) caprylic/capric mono- and diglycerides (*e*.*g*., Labrasol™ of Gattefossé), polyoxyethylene (35) castor oil and polyoxyethylene (40) hydrogenated castor oil; polyoxyethylene alkyl ethers, for example polyoxyethylene (20) cetostearyl ether, polyoxyethylene fatty acid esters, for example polyoxyethylene (40) stearate, polyoxyethylene sorbitan esters, for example polysorbate 20 and polysorbate 80 (*e*.*g*., Tween™ 80 of ICI), propylene glycol fatty acid esters, for example propylene glycol laurate (*e*.*g*., Lauroglycol™ of Gattefossé), sodium lauryl sulfate, fatty acids and salts thereof, for example oleic acid, sodium oleate and triethanolamine oleate, glyceryl fatty acid esters, for example glyceryl monostearate, sorbitan esters, for example sorbitan monolaurate, sorbitan monooleate, sorbitan monopalmitate and sorbitan monostearate, tyloxapol, and mixtures thereof. Such wetting agents, if present, constitute in total about 0.25% to about 15%, preferably about 0.4% to about 10%, and more preferably about 0.5% to about 5%, of the total weight of the composition.

Wetting agents that are anionic surfactants are preferred. Sodium lauryl sulfate is a particularly preferred wetting agent. Sodium lauryl sulfate, if present, constitutes about 0.25% to about 7%, more preferably about 0.4% to about 4%, and still more preferably about 0.5% to about 2%, of the total weight of the composition.

### 1.5. Lubricants, glidants and anti-adherents

Compositions of the invention optionally comprise one or more pharmaceutically acceptable lubricants and/or glidants as excipients. Suitable lubricants and/or glidants include, either individually or in combination, glyceryl behapate (*e*.*g*., Compritol™ 888); stearic acid and salts thereof, including magnesium, calcium and sodium stearates; hydrogenated vegetable oils (*e*.*g*., Sterotex™); colloidal silica; talc; waxes; boric acid; sodium benzoate; sodium acetate; sodium fumarate; sodium chloride; DL-leucine; polyethylene glycols (*e*.*g*., Carbowax™ 4000 and Carbowax™ 6000); sodium oleate; sodium lauryl sulfate; and magnesium lauryl sulfate. Such lubricants and/or glidants, if present, constitute in total about 0.1% to about 10%, preferably about 0.2% to about 8%, and more preferably about 0.25% to about 5%, of the total weight of the composition.

Magnesium stearate is a preferred lubricant used, for example, to reduce friction between the equipment and granulated mixture during compression of tablet formulations.

Suitable anti-adherents include talc, cornstarch, DL-leucine, sodium lauryl sulfate and metallic stearates. Talc is a preferred anti-adherent or glidant used, for example, to reduce formulation sticking to equipment surfaces and also to reduce static in the blend. Talc, if present, constitutes about 0.1% to about 10%, more preferably about 0.25% to about 5%, and still more preferably about 0.5% to about 2%, of the total weight of the composition.

### 1.6. Other excipients

Other excipients such as colorants, flavors and sweeteners are known in the pharmaceutical art and can be used in compositions of the present invention. Tablets can be coated, for example with an enteric coating, or uncoated. Compositions of the invention can further comprise, for example, buffering agents.

### 1.7. Preferred oral compositions

In one embodiment, a composition of the present invention comprises eplerenone in a desired amount and one or more cellulosic excipients. The term "cellulosic excipient" embraces excipients comprising cellulose or a derivative thereof, including without restriction purified cellulose, microcrystalline cellulose, and alkylcelluloses and their derivatives and salts (*e*.*g*., methylcellulose, ethylcellulose, hydroxypropylcellulose, HPMC, carboxymethylcellulose, sodium carboxymethylcellulose including croscarmellose sodium, *etc*.). Preferably, at least one such cellulosic excipient present is selected from the group consisting of (C₁₋₆ alkyl)celluloses and their derivatives and salts. Still more preferably, this cellulosic excipient is selected from the group consisting of hydroxy(C₂₋₄ alkyl)-(C₁₋₄ alkyl)-celluloses and their derivatives and salts.

Compositions of this embodiment preferably further comprise one or more excipients selected from the group consisting of diluents, disintegrants, binding agents, wetting agents, lubricants and anti-adherent agents. More preferably, these compositions comprise one or more excipients selected from the group consisting of lactose, microcrystalline cellulose, croscarmellose sodium, HPMC, sodium lauryl sulfate, magnesium stearate and talc. Still more preferably, these compositions comprise lactose monohydrate, microcrystalline cellulose, croscarmellose sodium and HPMC, most preferably further comprising one or more additional excipients selected from the group consisting of sodium lauryl sulfate, magnesium stearate and talc.

Individual excipients listed above in the present embodiment optionally can be replaced with other suitable excipients if desired. Acceptable substitute excipients are chemically compatible both with eplerenone and with the other excipients. Although other diluents, disintegrants, binding agents and adhesives, wetting agents, lubricants and/or anti-adherent or glidant agents can be employed, compositions comprising nanoparticulate eplerenone, lactose, microcrystalline cellulose, croscarmellose sodium and HPMC, and, optionally, sodium lauryl sulfate, magnesium stearate and/or talc generally possess a superior combination of pharmacokinetic, chemical and/or physical properties relative to such other compositions.

In another embodiment, a composition of the invention comprises:
about 1% to about 95% eplerenone;
about 5% to about 99% of a pharmaceutically acceptable diluent;
about 0.5% to about 30% of a pharmaceutically acceptable disintegrant; and
about 0.5% to about 25% of a pharmaceutically acceptable binding agent;
all percentages being by weight. Such a composition optionally can additionally comprise about 0.25% to about 15% of a pharmaceutically acceptable wetting agent; about 0.1% to about 10% of a pharmaceutically acceptable lubricant; and/or about 0.1% to about 15% of a pharmaceutically acceptable anti-adherent agent.

In still another embodiment, a composition of the invention is in the form of an oral unit dosage form, preferably a tablet or capsule, comprising eplerenone and a cellulosic excipient as defined above. Preferably, the composition comprises one or more excipients selected from the group consisting of lactose monohydrate, microcrystalline cellulose, croscarmellose sodium, hydroxypropyl methylcellulose, sodium lauryl sulfate, magnesium stearate and talc.

### 2. Parenteral compositions

Solid state eplerenone forms of the invention can be administered parenterally, for example by intravenous, intramuscular or subcutaneous injection of a suspension of the solid state eplerenone in a carrier liquid such as, for example, saline, dextrose solution or water. Suspension compositions can comprise appropriate excipient ingredients selected from those disclosed for oral compositions hereinabove.

### 3. Transdermal compositions

Other compositions can be in the form of a topical or transdermal ointment or cream, having dispersed therein solid state eplerenone in an amount of, for example, about 0.075% to about 30%, preferably about 0.2% to about 20% by weight and more preferably about 0.4% to about 15%, by weight. Such a topical or transdermal composition can desirably include a compound which enhances absorption or penetration of the eplerenone through skin. Examples of such dermal penetration enhancing compounds include dimethylsulfoxide and related compounds.

The novel solid state forms of eplerenone also can be administered transdermally using a patch either of a reservoir and porous membrane type or of a solid matrix type. In either case, the eplerenone is delivered continuously from a reservoir or from microcapsules through a membrane into an eplerenone-permeable adhesive, which is in contact with the skin or mucosa of the subject. If the eplerenone is absorbed through the skin, a controlled and predetermined flow of the eplerenone can be administered to the recipient. In the case of microcapsules, the encapsulating agent can also function as the membrane.

### Methods of treatment or prophylaxis

The present invention also embraces a method for treatment and/or prophylaxis of an aldosterone-mediated condition or disorder, the method comprising treating a subject having or susceptible to such condition or disorder with a therapeutically effective amount of solid state eplerenone or a pharmaceutical composition containing solid state eplerenone, at least a detectable fraction of the solid state eplerenone being Form H eplerenone and the balance comprising one or more of Form L eplerenone, solvated crystalline eplerenone and amorphous eplerenone. Such a method is useful for treatment and/or prophylaxis of a condition or disorder in a subject where administration of an aldosterone antagonist is indicated, including, but not limited to, treatment of conditions of hyperaldosteronism such as hypertension, heart failure including cardiac insufficiency, cirrhosis of the liver, excess collagen, fibrosis, benign prostate hypertrophy and depression.

Besides being useful for human treatment, these solid state forms of eplerenone and pharmaceutical compositions thereof are also useful for veterinary treatment of companion, exotic and farm animals, for example horses, dogs, and cats.

Solid state forms of eplerenone and compositions thereof also can be used (i) in combination therapies partially or completely in place of other aldosterone receptor antagonists, and/or (ii) in combination therapies with other drugs. The phrase "combination therapy" embraces administration of each drug in a sequential manner in a regimen that will provide beneficial effects of the drug combination, as well as co-administration of the drugs in a substantially simultaneous manner, such as in a single capsule or injection having a fixed ratio of these active agents or in multiple, separate dosage forms or injections, one for each agent. Non-limiting examples of such combination therapy include treatment of cardiovascular diseases using a combination of an aldosterone receptor antagonist and an angiotensin II receptor antagonist as described in International Patent Publication No. WO 96/24373, treatment of congestive heart failure using a combination of an aldosterone receptor antagonist and an angiotensin II antagonist as described in International Patent Publication No. WO 96/40257, and treatment of heart failure using a combination of an aldosterone receptor antagonist, an ACE inhibitor and a diuretic as described in International Patent Publication No. WO 96/24372, all of these publications being incorporated herein by reference.

### EXAMPLES

The following Examples contain detailed descriptions of methods of preparation of various solid state forms of eplerenone described herein. These detailed descriptions fall within the scope of the invention and illustrate the invention without in any way restricting that scope. All percentages are by weight unless otherwise indicated. The eplerenone starting material used in each of the following Examples was prepared in accordance with scheme 1 set forth in above-cited International Patent Publication No. WO 98/25948.

### Example 1: Preparation of methyl ethyl ketone solvate from high purity eplerenone starting material and preparation of Form L eplerenone from the solvate

### A. Preparation of methyl ethyl ketone solvate

High purity eplerenone (>99% purity with <0.2% total diepoxide and 11,12-epoxide) in an amount of 437 mg was dissolved in 10 ml methyl ethyl ketone by heating to boiling on a hot plate with magnetic stirring at 900 rpm. The resulting solution was allowed to cool to room temperature with continuous magnetic stirring. Once at room temperature, the solution was transferred to a 1°C bath with continued stirring for 1 hour. Solid methyl ethyl ketone solvate was collected from the cold solution by vacuum filtration.

### B. Preparation of Form L eplerenone

The solid methyl ethyl ketone solvate prepared as above was dried in an oven at 100°C for four hours at ambient atmospheric pressure. The dried solid was determined to be pure Form L by DSC and XRPD analysis.

### Example 2: Preparation of additional solvates from high purity eplerenone starting material

Additional solvated crystalline forms were prepared substantially as in Example 1 by replacing methyl ethyl ketone with each of the following solvents: n-propanol, 2-pentanone, acetic acid, acetone, butyl acetate, chloroform, ethanol, isobutanol, isobutyl acetate, isopropanol, methyl acetate, ethyl propionate, n-butanol, n-octanol, propyl acetate, propylene glycol, t-butanol, tetrahydrofuran and toluene.

### Example 3: Preparation of methyl ethyl ketone solvate by vapor diffusion growth

Eplerenone (>99.9% purity) in an amount of 400 mg was dissolved in 20 ml methyl ethyl ketone by warming on a hot plate to form a stock solution. An 8 ml amount of the stock solution was diluted to 10 ml with methyl ethyl ketone, the resulting solution being referred to as an 80% dilution sample. A 4 ml amount of the stock solution was diluted to 10 ml with methyl ethyl ketone (a 40% dilution sample). A 2 ml amount of the stock solution was diluted to 10 ml with methyl ethyl ketone (a 20% dilution sample). The various dilution samples in 20 ml scintillation vials were transferred to a dessicator jar containing a small amount of hexane as an anti-solvent. The dessicator jar was sealed and hexane vapor allowed to diffuse into the methyl ethyl ketone solutions. Crystals of the methyl ethyl ketone solvate of eplerenone grew in the 80% dilution sample within 24 hours.

### Example 4: Preparation of solvate crystal forms of eplerenone by rotary evaporator

About 400 mg of eplerenone (>99.9% purity) is weighed into a 250 ml round bottom flask. A solvent selected from methyl ethyl ketone and the solvents listed in Example 2, in an amount of 150 ml, is added to the flask and, if necessary, the solution is heated gently until the eplerenone is dissolved. The resulting clear solution is placed on a Buchi rotary evaporator with a bath temperature of about 85°C and the solvent is removed under vacuum. Solvent removal is stopped when approximately 10 ml of solvent remain in the flask. The resulting solids are analyzed by an appropriate method (*e*.*g*., XRPD, DSC, TGA, microscopy, *etc*.) for determination of crystal form.

### Example 5: Slurry conversion

Approximately 150 mg of Form L eplerenone and 150 mg of Form H eplerenone were added to 5 ml ethyl acetate. The resulting slurry was magnetically stirred at 300 rpm overnight. The next day a sample of the resulting solid was collected by filtration. Analysis of the sample by XRPD indicated that the sample was entirely composed of Form L eplerenone.

### Example 6: Preparation of (a) solvate from low purity eplerenone starting material and (b) Form H crystalline eplerenone from resulting solvate

Samples containing varying amounts of the diepoxide or the 11,12-epoxide impurity as herein defined were prepared by adding the desired amount of the impurity to a 7 ml scintillation vial together with an amount of eplerenone sufficient to provide a total sample mass of 100 mg. The content of the impurity in each sample is given in Tables 6A and 6B, where the impurity is respectively the diepoxide or the 11,12-epoxide. A micro-flea magnetic stirrer was added to each scintillation vial along with 1 ml of methyl ethyl ketone. The vials were loosely capped and the solids were dissolved by heating to reflux on a hot plate with magnetic stirring. When dissolution was complete, the resulting solutions were allowed to cool to room temperature, with continued stirring. The resulting solids were then collected by vacuum filtration and immediately analyzed by XRPD. The solids were then placed in a 100°C oven and dried for 1 hour at ambient atmospheric pressure. The dried solids were analyzed by XRPD for Form H content by monitoring the area of the Form H diffraction peak at about 12.1 degrees 2θ. All XRPD diffraction patterns were recorded using an Inel Multipurpose diffractometer.

**Table 6A:**

| **Composition of eplerenone starting materials in Example 6** | | |
|---|---|---|
| **% Diepoxide** | **Eplerenone (mg)** | **Diepoxide (mg)** |
| 0 | 100.44 | 0 |
| 1 | 99.08 | 1.24 |
| 2 | 98.09 | 2.24 |
| 3 | 97.08 | 3.04 |
| 5 | 95.09 | 5.04 |

**Table 6B:**

| **Composition of eplerenone starting materials in Example 6** | | |
|---|---|---|
| **% 11,12-Epoxide** | **Eplerenone (mg)** | **11,12-Epoxide (mg)** |
| 0 | 101.38 | 0 |
| 1 | 99.23 | 1.10 |
| 5 | 94.97 | 5.36 |
| 10 | 90.13 | 10.86 |

### A. Diepoxide results

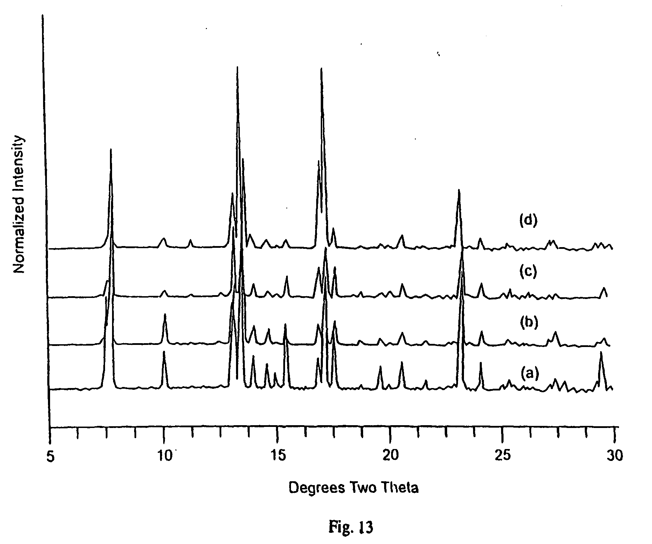

Fig. 13 shows the XRPD patterns for methyl ethyl ketone solvate wet cake obtained from the (a) 0%, (b) 1%, (c) 3% and (d) 5% diepoxide-doped methyl ethyl ketone crystallizations. The peak intensities have been normalized for ease of comparison. No peaks characteristic of Form H or of the diepoxide are present in the diffraction patterns. The patterns are characteristic of the methyl ethyl ketone solvate of eplerenone.

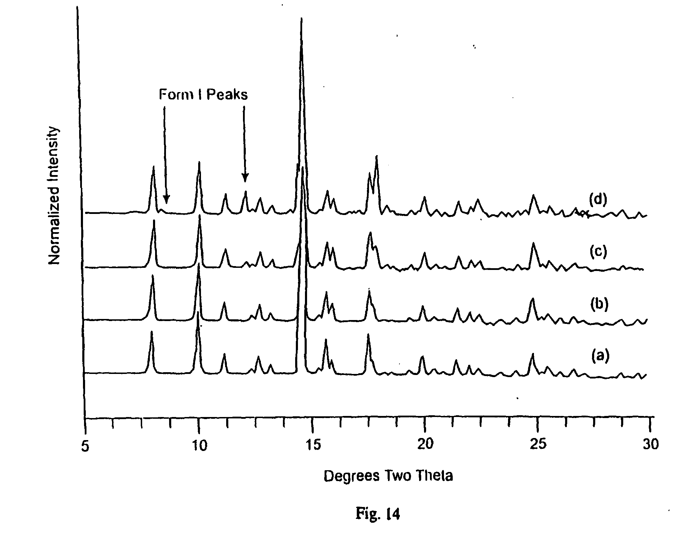

Fig. 14 shows the XRPD patterns for the dried solids obtained from the (a) 0%, (b) 1%, (c) 3% and (d) 5% diepoxide-doped methyl ethyl ketone crystallizations. The peak intensities have been normalized for ease of comparison. No Form H was detected in the dried samples corresponding to the methyl ethyl ketone crystallizations where diepoxide doping level was 0% or 1%. Form H was detected in the dried samples corresponding to the methyl ethyl ketone crystallizations where doping level was 3% or 5%. The area of the Form H diffraction peak at about 12.1 degrees 2θ and the estimated Form H content for each sample are given in Table 6C.

**Table 6C:**

| **Data from methyl ethyl ketone crystallizations in Example 6** | | | |
|---|---|---|---|
| **% Diepoxide in starting material** | **% Diepoxide in crystals (by HPLC)** | **Form H peak area 12.1° 2θ** | **Estimated % Form H** |
| 0 | 0 | None detected | 0 |
| 1 | 0.29 | None detected | 0 |
| 3 | 0.58 | 1168 | 10 |
| 5 | 1.05 | 4175 | 30 |

The results reported in Table 6C confirm that presence of the diepoxide affects formation of Form H eplerenone during desolvation. Formation of Form H is induced when the diepoxide is incorporated into and/or adsorbed onto the methyl ethyl ketone solvate crystals.

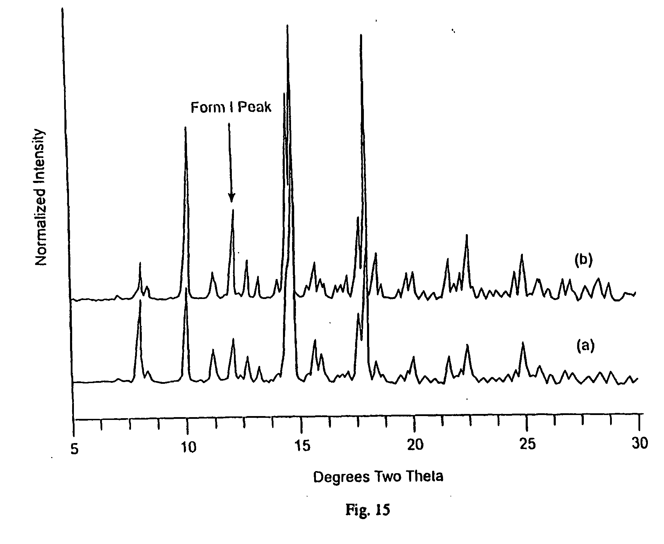

A second 3% diepoxide doping experiment was conducted to analyze the impact of route of preparation on the amount of Form H formed during desolvation. In this experiment, the methyl ethyl ketone solvate obtained from the doped crystallization was divided into two portions. The first portion was left untreated while the second portion was lightly ground in a mortar and pestle to induce a higher level of crystal defects. The two portions were both dried at 100°C for 1 hour at ambient atmospheric pressure. The dried solids were analyzed by XRPD. The XRPD patterns are given in Fig. 15 for the dried solids from the methyl ethyl ketone crystallization with 3% doping of diepoxide (a) without and (b) with grinding of the solvate prior to drying. The XRPD patterns indicated a greater amount of Form H in the ground sample relative to the unground sample. These results suggest that the conditions under which the methyl ethyl ketone solvate is isolated and handled can affect the crystal form that results from the desolvation.

### B. 11,12-Epoxide results

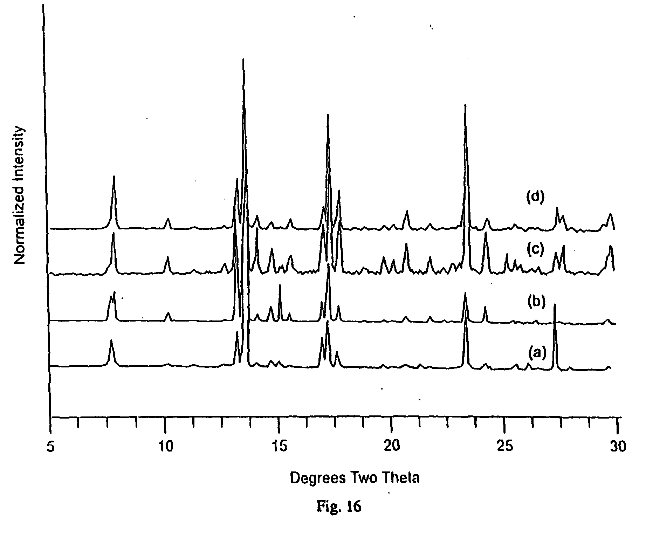

Fig. 16 shows the XRPD patterns for methyl ethyl ketone solvate wet cake obtained from the (a) 0%, (b) 1%, (c) 5% and (d) 10% 11,12-epoxide-doped methyl ethyl ketone crystallizations. The peak intensities have been normalized for ease of comparison. No peaks characteristic of Form H or of the 11,12-epoxide are present in the diffraction patterns. The patterns are characteristic of the methyl ethyl ketone solvate of eplerenone.

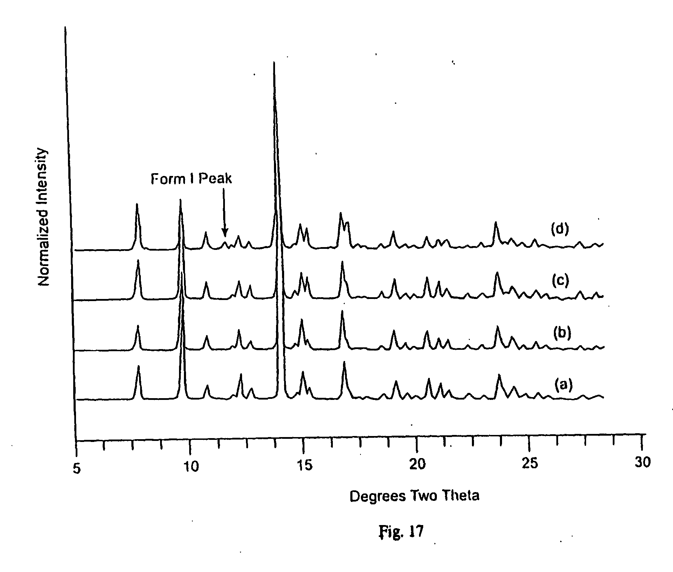

Fig. 17 shows the XRPD patterns for the dried solids obtained from the (a) 0%, (b) 1%, (c) 5% and (d) 10% 11,12-epoxide-doped methyl ethyl ketone crystallizations. The peak intensities have been normalized for ease of comparison. No Form H was detected in the dried samples corresponding to the methyl ethyl ketone crystallizations where 11,12-epoxide doping level was 0%, 1% or 5%. Form H was detected in the dried samples corresponding to the methyl ethyl ketone crystallization where 11,12-epoxide doping level was 10%. The area of the Form H diffraction peak at about 12.1 degrees 2θ and estimated Form H content for each sample are given in Table 6D.

**Table 6D:**

| **Data from methyl ethyl ketone crystallizations in Example 6** | | |
|---|---|---|
| **% 11,12-Epoxide in starting material** | **Form H peak area 12.1°2θ** | **Estimated % Form H** |
| 0 | None detected | 0 |
| 1 | None detected | 0 |
| 5 | None detected | 0 |
| 10 | 1541 | 10-15 |

The results reported in Table 6D confirm that presence of the 11,12-epoxide impacts formation of Form H eplerenone during desolvation. The level of impurity in a methyl ethyl ketone crystallization required to induce formation of Form H eplerenone appears to be greater for the 11,12-epoxide than for the diepoxide.

### Example 7: Effect of crystallization and drying on final crystal form

The following four experiments analyzing effect of crystallization and drying on the final crystal form were conducted: (i) methyl ethyl ketone crystallization of eplerenone (2³+3 statistical design of experiment), (ii) crystallization of poor quality mother liquor residue, (iii) crystallization of high purity eplerenone with form H seeding, and (iv) crystallization of low purity eplerenone with form L seeding. Variables in these experiments included cooling rate, starting material purity level, and endpoint temperature of crystallization. For purposes of this Example, high purity eplerenone was defined as ultra-pure (by HPLC) milled eplerenone and low purity eplerenone was defined as 89% pure eplerenone. To prepare the low purity eplerenone, stripped-down mother liquors from the process for preparation of eplerenone were analyzed and blended to yield a material that was 61.1% eplerenone, 12.8% diepoxide and 7.6% 11,12-epoxide. This material was then blended with a sufficient amount of high purity eplerenone to yield the 89% eplerenone.

### A. Methyl ethyl ketone crystallization

In the methyl ethyl ketone crystallization experiment, all runs were performed using 60 g high purity eplerenone. High endpoint was defined as 45°C and low endpoint was defined as 5°C. High cooling rate was defined as 3°C/minute and low cooling rate was defined as 0.1°C/minute. Center points were 1.5°C/minute cooling rate, 94.5% pure eplerenone, and a 25°C endpoint.

After a background reading was taken with the FTIR, 250 ml methyl ethyl ketone was charged to a 1 liter Mettler RC-1, MP10 reactor and stirred at 100 rpm. After several scans, eplerenone was charged to the reactor followed by an additional 470 ml methyl ethyl ketone. Agitation was increased to 500 rpm to suspend solids and the batch temperature was increased to 80°C. The batch temperature was held at 80°C to ensure dissolution of the eplerenone. Black or white specks generally were visible in the resulting transparent solution. The batch temperature was then reduced by ramp cooling at the desired rate to the desired endpoint, where it was maintained for 1 hour before being pulled into a transfer flask and filtered to provide a wet cake. The reactor, transfer flask and wet cake were then washed with 120 ml methyl ethyl ketone. About 10 g of each wet cake was dried in a vacuum oven under nominal conditions of 75°C with a light nitrogen bleed. The wet cake was dried by fluid bed drying under high and low conditions. High conditions for fluid bed drying were defined as 100°C with a blower setting of 4, while low conditions for fluid bed drying were defined as 40°C with a blower setting of 1.

### B. Crystallization of poor quality mother liquor residue

In the experiment involving crystallization of poor quality mother liquor residue, 60 g of the 61.1% pure eplerenone and 720 ml methyl ethyl ketone were charged directly to a 1 liter Mettler RC-1, MP10 reactor. The impure eplerenone was not blended with high purity eplerenone prior to being charged to the reactor. The resulting mixture was heated to 80°C and was an opaque slurry at that temperature. Crystallization continued and the mixture was filtered at 45°C under fast cooling conditions.

### C. Form H seeding

In the Form H seeding experiment, 60 g high purity eplerenone and 720 ml methyl ethyl ketone were charged to a 1 liter Mettler RC-1, MP10 reactor. The mixture was heated to 80°C and then cooled to 25°C at a cooling rate of 1.5°C/minute. When the solution had cooled to 62°C, it was seeded with 3 g of phase pure Form H crystals to initiate crystallization. The Form H seed crystals were prepared by the digestion process described in Example 9 below.

### D. Form L seeding

In the Form L seeding experiment, 66.6 g of 89.3% eplerenone (prepared by mixing 48.3 g of high purity eplerenone with 18.3 g of 61.1% eplerenone) and 720 ml methyl ethyl ketone were charged to a 1 liter Mettler RC-1, MP10 reactor. The mixture was heated to 80°C and then cooled to 25°C at a cooling rate of 1.5°C/minute. When the solution had cooled to 63°C, it was seeded with 3 g of phase pure Form L crystals to initiate crystallization. The Form L seed crystals were prepared by the crystallization and desolvation process described in Example 1 above.

### E. Results

Results from the experiments are reported in Table 7A.

In the methyl ethyl ketone crystallization experiment, Form H was detected only where low purity eplerenone containing the diepoxide was used. Elevated levels of the diepoxide in the final product were also observed with higher cooling rates.

The experiment involving crystallization of poor quality mother liquor residue yielded poor quality material that appeared to be a mixture of the diepoxide and Form H eplerenone when analyzed by XRPD.

The Form H seeding experiment (where high purity eplerenone was seeded with Form H) yielded a product that was 77% Form H based on XRPD analysis, but entirely Form H based on DSC. The XRPD model, however, had not been tested for linearity beyond about 15% Form H. This experiment was the only one of the four experiments of this Example where Form H was created in the absence of the diepoxide.

The Form L seeding experiment (where low purity eplerenone was seeded with Form L) yielded a product that was entirely Form L.

The data obtained for high condition fluid bed drying of eplerenone appeared to correspond to the data obtained for vacuum oven drying. The low condition fluid bed drying yielded results that differed from those for vacuum oven drying.

**Table 7A:**

| **Results of Example 7** | | | | | | | | |
|---|---|---|---|---|---|---|---|---|
| **Cooling rate (°C/min.)** | **Cooling endpoint (°C)** | **Starting material % purity** | **Nucleation temp. (°C)** | **% 11,12-Epoxide**^{**1**} | **% Diepoxide**^{**1**} | **Assay for desolvated crystal** | **% yield** | **% Form H (by XRPD)** |
| 3 | 45 | 94.5 | 57.0 | ND | ND | 100.3 | 66.1 | ND |
| 3 | 5 | 94.5 | 54.9 | ND | ND | 100.3 | 98.1 | ND |
| 0.1 | 45 | 94.5 | 60.9 | ND | ND | 100.3 | | ND |
| 0.1 | 5 | 94.5 | 63.4 | ND | ND | 100.5 | 79.3 | ND |
| 3 | 45 | 61.1 | | 4.8 | 36.6 | 43.3 | 27 | 100² |
| 3 | 45 | 89.3 | 52.2 | 0.49 | 0.88 | 98.3 | 62 | 29 |
| 3 | 5 | 89.3 | 53.3 | 0.56 | 1.0 | 98.1 | 87 | 9 |
| 1.5 | 25 | 100 | 59.0 | 0.18 | 0.36 | 99.4 | 75 | 5 |
| 0.1 | 45 | 89.3 | 63.3 | 0.20 | 0.44 | 99.4 | 36 | 31 |
| 0.1 | 5 | 89.3 | 61.4 | 0.18 | 0.40 | 99.5 | 87 | ND |
| 1.5 | 25 | 100 | 60.6 | 0.18 | 0.36 | 99.5 | 79.2 | ND |
| 1.5 | 25 | 100 | 55.9 | 0.38 | 0.80 | 98.6 | 80.5 | <3% |
| 1.5 | 25 | 100 seeded Form H | | 0.03 | ND | 100.4 | 82.2 | 77/100³ |
| 1.5 | 25 | 89.3 seeded Form L | | 0.33 | 0.50 | 97.5 | 80.2 | ND |

| | | | | | | | | |
|---|---|---|---|---|---|---|---|---|
| ¹ Weight % after drying solvate in a vacuum oven at 75°C. | | | | | | | | |
| ² Appears to be mixture of Form H and diepoxide when analyzed by XPRD. | | | | | | | | |
| ³ Appears to be 77% Form H by XPRD and 100% Form H by DSC. ND = none detected. | | | | | | | | |

### F. Material purity

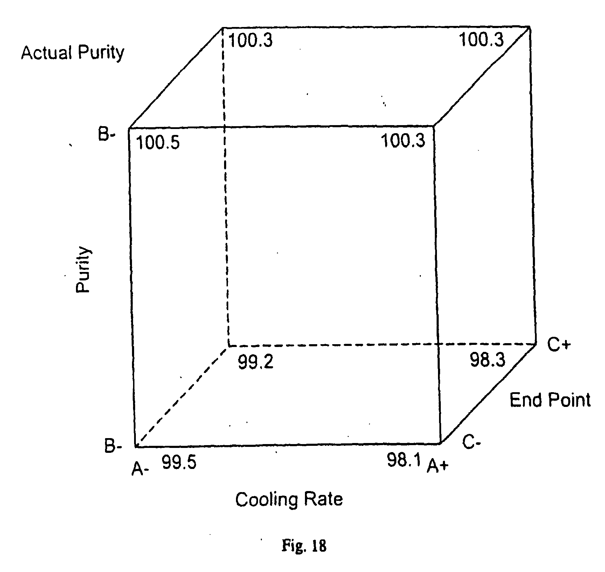

A cube plot of product purity, starting material purity, cooling rate and endpoint temperature based on the data reported in Table 7A is shown in Fig. 18. The cube plot suggests that use of a higher purity material at the start of crystallization will yield a higher purity product. The endpoint temperature of crystallization does not appear greatly to affect product purity. The cooling rate, however, appears to have an effect with slightly less pure product resulting from a faster cooling rate. In fact, the level of diepoxide generally was higher with faster cooling rates.

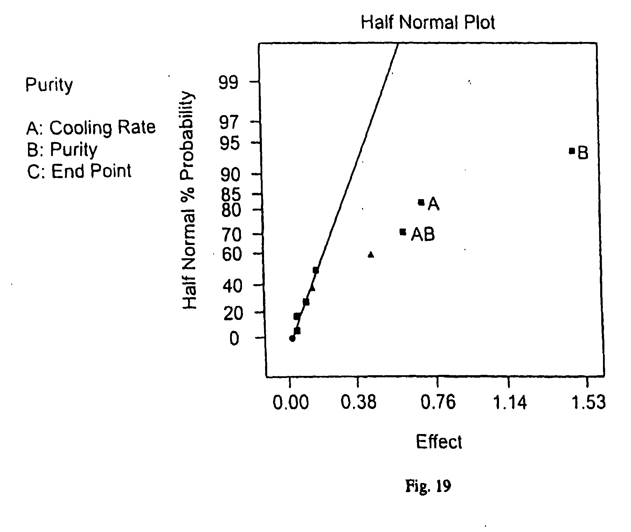

Fig. 19 shows a half normal plot that was prepared using the results of the cube plot to determine which variables, if any, had a statistically significant effect on product purity. Starting material purity had the greatest statistically significant effect on product purity, although the effect of cooling rate and the interaction between cooling rate and starting material purity were also seen as statistically significant.

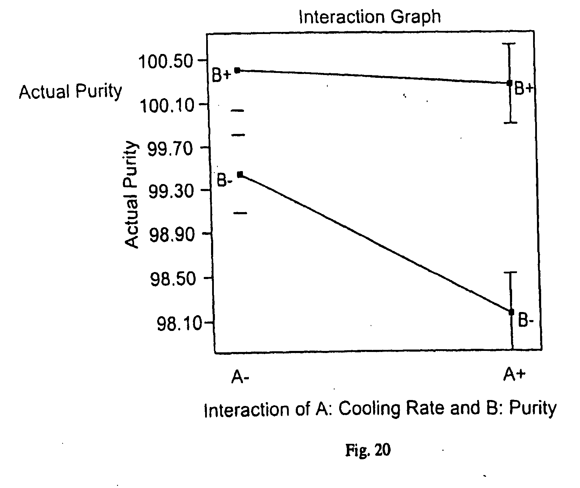

Fig. 20 is an interaction graph based on these results, showing the interaction between starting material purity and cooling rate on product purity. With the high purity eplerenone the cooling rate appears to have little or no effect on final purity. With the low purity eplerenone (89.3% eplerenone starting material), however, the product purity decreases as cooling rate increases. This result suggests that more impurities crystallize out when crystallization is conducted at higher cooling rates.

### G. Form H content

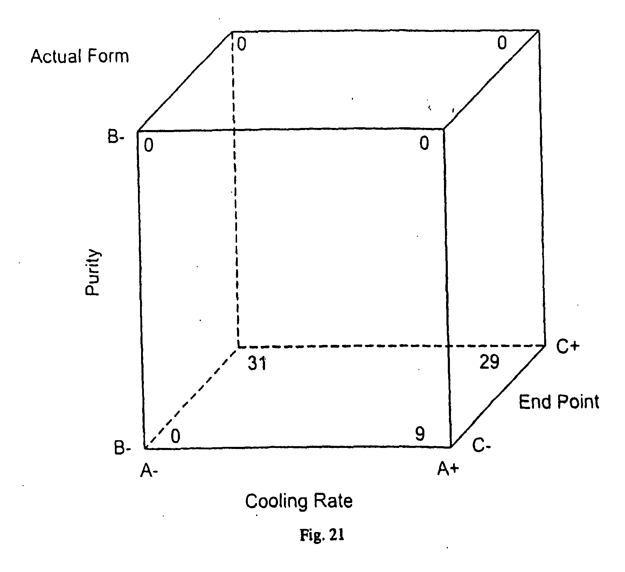

A cube plot of Form H weight fraction, starting material product purity, cooling rate and endpoint temperature based on the data reported in Table 7A is shown in Fig. 21. The cube plot suggests that use of a higher purity eplerenone at the start of crystallization will yield a lower amount of Form H. The endpoint temperature of crystallization also appears to have an effect on the form of the final product. The cooling rate does not appear to greatly affect the formation of Form H although some Form H may result from faster cooling at the low endpoint temperature in the presence of impurities.

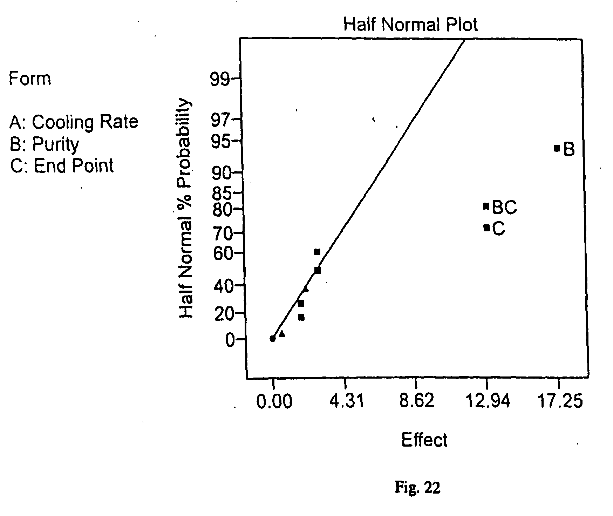

Fig. 22 shows a half normal plot that was prepared using the results of the cube plot to determine which variables, if any, had a statistically significant effect on the amount of Form H in the final material. Starting material purity, endpoint temperature of the crystallization and the interaction between these two variables were seen as statistically significant effects.

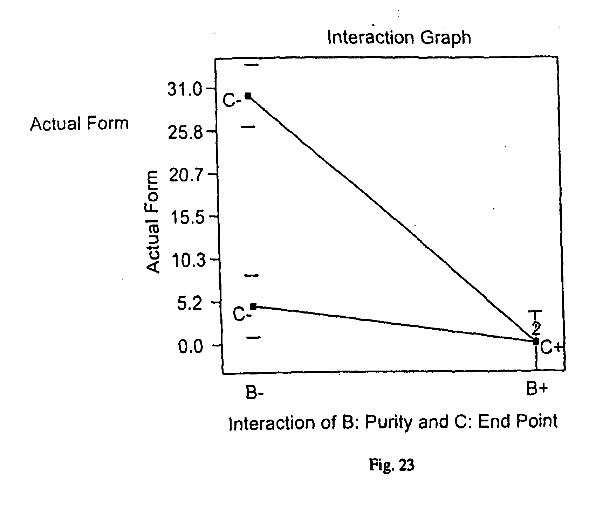

Fig. 23 is an interaction graph based on these results, showing the interaction between starting material purity and endpoint temperature on final Form H content. With the high purity eplerenone, endpoint temperature appears to have little effect on Form H content. No Form H resulted in either case with pure eplerenone. With low purity eplerenone (89.3% eplerenone starting material), however, Form H was present in both cases, with significantly more Form H at higher endpoint temperatures.

Table 7B reports the weight fraction of Form H measured in materials dried using either a fluid bed (Lab-Line/P.R.L. Hi-Speed fluid bed dryer, Lab-Line Instruments, Inc.) or a vacuum oven (Baxter Scientific Products vacuum drying oven, Model DP-32). Similar Form H content was observed for comparable materials dried in either the high fluid bed or the vacuum oven. A difference was observed, however, for comparable materials dried in the low fluid bed relative to the vacuum oven.

**Table 7B:**

| **Effect of process variables on Form H content** | | | | |
|---|---|---|---|---|
| **Cooling rate** | **Endpoint** | **Impurity level** | **Drying conditions** | **% Form H** |
| High | High | High | Vacuum Oven | 29 |
| High | High | High | High Fluid Bed | 25 |
| High | High | High | Low Fluid Bed | 4.7 |
| Low | Low | Low | Vacuum Oven | ND |
| Low | Low | Low | High Fluid Bed | ND |
| Low | Low | Low | Low Fluid Bed | 5.5 |
| ND = none detected. | | | | |

### Example 8: Crystallization of Form L from methyl ethyl ketone with desolvation

Form H eplerenone in an amount of 10 g was combined with 80 ml methyl ethyl ketone. The mixture was heated to reflux (79°C) and stirred at this temperature for about 30 minutes. The resulting slurry was then cooled with a stepwise, holdpoint protocol by maintaining the slurry at 65°C, 50°C, 35°C and 25°C for about 90 minutes at each temperature. The slurry was filtered and rinsed with about 20 ml methyl ethyl ketone. The resulting isolated solid was initially dried on the filter and then in a vacuum oven at 40-50°C. The drying was completed in the vacuum oven at 90-100°C. The desolvated solid was obtained with an 82% recovery. XRPD, MIR and DSC confirmed that the solid had Form L crystalline structure.

### Example 9: Digestion of low purity eplerenone starting material with a solvent to prepare Form H

### A. Digestion with ethanol solvent

Low purity eplerenone (64% assay by HPLC) in an amount of 24.6 g was combined with 126 ml of ethanol 3A. The slurry was heated to reflux and the distillate removed. An additional 126 ml of ethanol 3A was simultaneously added as 126 ml of solvent was removed via atmospheric distillation. Upon completion of the solvent turnover, the mixture was cooled to 25°C and stirred for 1 hour. The resulting solid was filtered and rinsed with ethanol 3A, and was then air-dried to yield the ethanol solvate. The solvate was further dried in a vacuum oven at 90-100°C for 6 hours to obtain 14.9 g of Form H eplerenone.

### B. Digestion with methyl ethyl ketone solvent

In an alternative digestion process, 1 g of low purity eplerenone (about 65% assay) was digested in 4 ml methyl ethyl ketone for 2 hours, after which the mixture was allowed to cool to room temperature. Once cooled, the resulting solid was collected by vacuum filtration and determined to be the methyl ethyl ketone solvate by XRPD analysis. The solid was dried at 100°C for 30 to 60 minutes. The dried solid was determined to be pure Form H by XPRD.

### Example 10: Digestion of high purity eplerenone with a solvent to prepare Form L

### A. Digestion with ethanol solvent

High purity eplerenone in an amount of 1 g was digested in 8 ml ethanol for approximately 2 hours. The solution was then allowed to cool to room temperature and the solids were collected by vacuum filtration. Analysis of the solids by XRPD immediately after filtration indicated that the solids were a solvate (presumably the ethanol solvate). The solids were subsequently dried at 100°C at ambient atmospheric pressure for 30 minutes. The dried solids were analyzed by XRPD and determined to be predominantly Form L (no Form H was detected).

### B. Digestion with methyl ethyl ketone solvent

High purity eplerenone in an amount of 1 g was digested in 4 ml methyl ethyl ketone for 2 hours, after which the solution was allowed to cool to room temperature and the solids were collected by vacuum filtration. The solids were immediately analyzed by XRPD and determined to be a solvate of eplerenone (presumably the methyl ethyl ketone solvate). The solvate was subsequently dried at 100°C at ambient atmospheric pressure for 30 to 60 minutes. The dried solids were analyzed by XRPD and determined to be primarily Form L with no diffraction peaks for Form H present.

### Example 11: Crystallization of Form L directly from solution

### Procedure A

Eplerenone in an amount of 2.5 g was dissolved in ethyl acetate by heating to 75°C. The solution was held at 75°C for 30 minutes to ensure complete dissolution, and was then cooled to 13°C at a cooling rate of 1°C/minute. The resulting slurry was stirred with an overhead stirrer at 750 rpm for 2 hours. Solids were collected by vacuum filtration and dried in a vacuum oven at 40°C for 1 hour. The XRPD pattern and DSC thermogram of the solid were characteristic of Form L eplerenone. TGA of the solid indicated no weight loss from the solid up to 200°C.

### Procedure B

In an alternative procedure, 2 g eplerenone was dissolved in 350 ml of a mixture of 15% acetonitrile and 85% water by heating on a hot plate with magnetic stirring. Once the eplerenone had dissolved, the solution was allowed to cool to room temperature overnight with magnetic stirring. The resulting solid was collected by vacuum filtration. The crystals were birefringent and had a triangular, plate-like crystal habit. The solid had an XRPD and DSC analysis characteristic of Form L eplerenone. TGA indicated no weight loss up to 200°C.

### Procedure C

In another alternative procedure, 640 mg eplerenone was placed in a 50 ml flask with 20 ml ethyl benzene. The resulting slurry was heated to 116°C and became a clear solution, which was then cooled to 25°C over 30 minutes. Nucleation began at 84°C during the cooling period. The resulting solids were filtered from the solution and air-dried to give 530 mg of solids (83% recovery). Hot-stage microscopy and XRPD confirmed that the solids were Form L eplerenone crystals.

### Procedure D

In another alternative procedure, 1.55 g eplerenone was added to 2.0 ml nitrobenzene and heated to 200°C. The resulting slurry was stirred overnight at 200°C and became a clear solution, which was then allowed to cool to room temperature by natural air convection to isolate a solid. The solid was determined to be Form L eplerenone by XRPD and polarized light microscopy.

### Procedure E

In another alternative procedure, 5.0 g eplerenone (purity >99%) was added to 82 g (104 ml) methanol. Under stirring action at 210 rpm, the solution was heated to 60°C and held at that temperature for 20 minutes to ensure complete dissolution. The solution was then cooled to -5°C at a rate of 0.16°C/minute under stirring. The resulting crystals were collected by filtration and dried in a vacuum oven at 40°C for 20 hours. The dried solids were determined to be pure Form L eplerenone by DSC and XRPD analysis.

### Procedure F

In an alternative procedure, 6.0 g eplerenone (ethanol solvate containing 9% ethanol and having a corrected purity of 95.2%) was added to 82 g (104 ml) methanol. Under stirring action at 210 rpm, the solution was heated to 60°C and held at that temperature for 20 minutes to ensure complete dissolution. The solution was then cooled to 50°C at a rate of 0.14°C/minute and then held at that temperature for about 2.5 hours. The solution was then cooled to -5°C at a rate of 0.13°C/minute under stirring. Crystals were collected by filtration and dried in a vacuum oven at 40° C for 16 hours. The dried solids were determined to be pure Form L eplerenone by DSC and XRPD analysis.

### Example 12: Crystallization of Form H directly from solution

The diepoxide in an amount of 150.5 mg and eplerenone in an amount of 2.85 g were added to 1.5 ml nitrobenzene. The mixture was magnetically stirred at 200°C for several hours. The resulting slurry was then allowed to cool to room temperature by natural air convection. The sample was dried and analyzed by polarized light microscopy and XRPD. The XPRD analysis indicated that the sample was a mixture of Form H and Form L. The crystals were translucent by microscopy, indicating that desolvation (and conversion to either Form H or Form L) did not occur.

### Example 13: Preparation of amorphous eplerenone by comminution

Approximately one-half of a steel Wig-L-Bug container was filled with about 60 g eplerenone (>99.9% purity). A steel ball and cap were placed on the sample container and agitated for 30 seconds by the Wig-L-Bug apparatus. The eplerenone was scraped off the surface of the Wig-L-Bug container and the container agitated for an additional 30 seconds. The resulting solid was analyzed by XRPD and DSC and was determined to be a mixture of amorphous eplerenone and Form L crystalline eplerenone.

### Example 14: Preparation of amorphous eplerenone by lyophilization

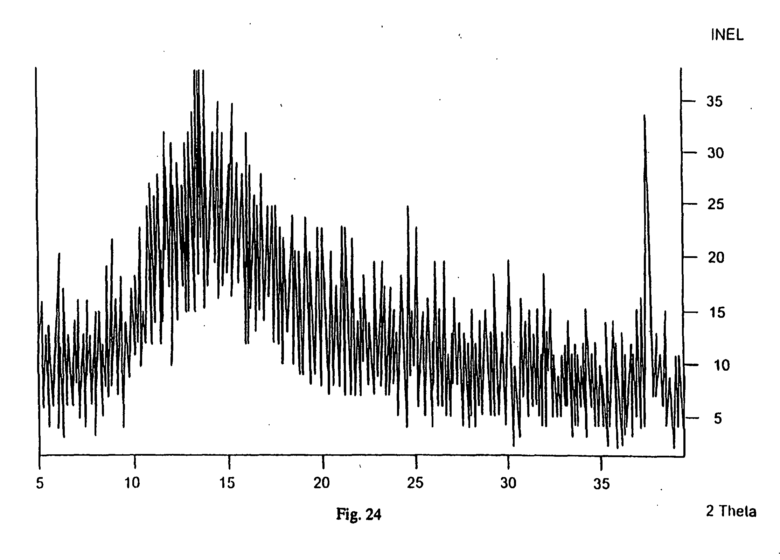

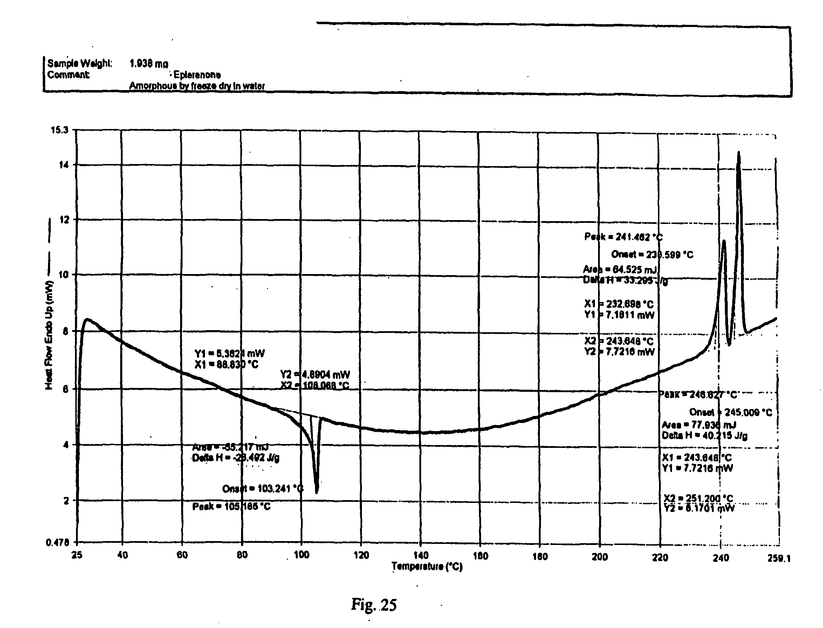

Approximately 100 mg of crude eplerenone was weighed into a beaker containing 400 ml water. The resulting mixture was heated slightly for 5 minutes, and then sonicated and heated with stirring for an additional 5 minutes to provide a dispersion. Approximately 350 ml of the eplerenone dispersion was filtered into a 1000 ml round bottom flask containing 50 ml of HPLC water. The dispersion was flash frozen in a dry ice/acetone bath over a time period of 1-2 minutes. The flask was attached to a Labconco Freezone 4.5 freeze dryer and the contents dried overnight. The solids in the flask were transferred to a small brown bottle. A small aliquot was observed under polarized light microscopy at 10X, 1.25X optivar in cargille oil (1.404) and observed to be at least 95% amorphous eplerenone. Figures 24 and 25 show the XRPD pattern and DSC thermogram obtained for the amorphous eplerenone. The peak observed at 39 degrees 2θ in Fig. 24 is attributable to the aluminum sample container.

### Example 15: Solubility of Form L eplerenone

The aqueous solubility of Form L eplerenone was measured at pH 7 (100 mM phosphate buffer) at 5, 25 and 40°C. Approximately 30 mg of Form L eplerenone was mixed with approximately 10 ml of buffer to form a slurry of eplerenone at both 5 and 25°C. Approximately 40 mg of Form II eplerenone was mixed with approximately 10 ml of buffer to form a slurry of eplerenone at 40°C. Samples were prepared in duplicate for each condition. The slurries were allowed to equilibrate in water shaker baths at the appropriate temperature and the solutions were analyzed for eplerenone content by ultraviolet visible analysis (245 nm) at time intervals of 1, 5, 12, 19, 27 and 36 days. Data from each temperature were appropriately averaged to determine the solubility of eplerenone at each temperature and are reported in Table 8. The residual solids from each time point were analyzed by DSC and TGA at the end of the 36 day equilibration and were determined to be Form L eplerenone.

**Table 8:**

| **Solubility of Form L eplerenone** | |
|---|---|
| **Temperature (°C)** | **Form L solubility (mg/ml)** |
| 5 | 0.24 |
| 25 | 0.29 |
| 40 | 0.39 |

### Example 16: Measurement of Intrinsic Dissolution Rates

Intrinsic dissolution rates were measured for the following four eplerenone polymorph samples: (i) Form L eplerenone prepared by direct crystallization from acetonitrile using water as an anti-solvent in the same manner as in Example 11, Procedure B; (ii) Form H eplerenone prepared by digestion in ethanol in the same manner as in Example 10, Procedure A, (iii) a mixture of 5% Form H and 95% Form L; and (iv) Form L eplerenone that was micronized to provide the following particle size distribution: 10% by weight of the particles under 9 µm, 50% by weight of the particles under 22 µm, and 90% by weight of the particles under 41 µm.

Eplerenone in an amount of 150 mg was weighed and placed into a VanKel intrinsic dissolution cavity. The powder was compressed at 8280 kPa using a Carver press to form tablets. The sample was then mounted on the intrinsic dissolution apparatus. The dissolution medium used was 1% sodium dodecyl sulfate (SDS) in HPLC water. All tests were conducted at 37°C for 2 hours. Before the start of the experiment, 500 ml of the dissolution medium was equilibrated at 37°C for 30 minutes in the dissolution bathing chamber. An initial sample was taken from each dissolution vessel, serving as the initiation time (T₀) for the test. The eplerenone tablets were then lowered into the dissolution medium. Samples were drawn at determined intervals for the determination of rate of dissolution. Care was taken to avoid air bubbles forming at the surface of the tablet. The samples were analyzed by UV absorbance detection at 243 nm. Intrinsic dissolution rates were calculated from the slope of the linear portion of the concentration versus time profiles corrected for volume and normalized for the surface area of the dissolution tablet (0.5 cm²).

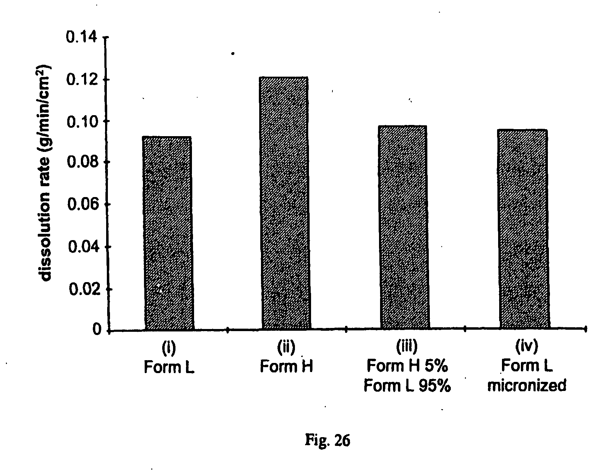

Fig. 26 reports the intrinsic dissolution rates measured for the four samples. These studies indicate that Form H eplerenone has faster intrinsic dissolution rate than Form L eplerenone. XRPD measurements comparing compressed and uncompressed eplerenone confirmed that the polymorphs did not interconvert upon compression or during the course of the dissolution studies.

### Example 17: Eplerenone polymorph composition

Tablets containing 25 mg, 50 mg, 100 mg and 200 mg doses of Form L eplerenone are prepared having the composition shown in Table 9.

**Table 9:**

| **Composition of tablets of Example 17** | |
|---|---|
| **Ingredient** | **Weight %** |
| Form L eplerenone | 29.41 |
| Form H eplerenone | Not detected |
| Lactose monohydrate, NF (#310) | 42.00 |
| Microcrystalline cellulose, NF (Avicel™ PH-101) | 18.09 |
| Croscarmellose sodium, NF (Ac-Di-Sol™) | 5.00 |
| HPMC, USP (#2910, Pharmacoat™ 603) | 3.00 |
| Sodium lauryl sulfate, NF | 1.00 |
| Talc, USP | 1.00 |
| Magnesium stearate, NF | 0.5 |
| Total | 100.00 |

### Example 18: Eplerenone polymorph composition

Capsules (hard gelatin capsule, #0) are prepared containing a 100 mg dose of eplerenone and have the composition shown in Table 10.

**Table 10:**

| **Composition of 100 mg capsules of Example 18** | |
|---|---|
| **Ingredient** | **Amount (mg)** |
| Form L eplerenone | 90.0 |
| Form H eplerenone | 10.0 |
| Lactose, hydrous, NF | 231.4 |
| Microcrystalline cellulose, NF | 45.4 |
| Talc, USP | 10.0 |
| Croscarmellose sodium, NF | 8.0 |
| Sodium lauryl sulfate, NF | 2.0 |
| Colloidal silicon dioxide, NF | 2.0 |
| Magnesium stearate, NF | 1.2 |
| Total capsule fill weight | 400.0 |

### Example 19: Eplerenone Polymorph Composition

Capsules (hard gelatin capsule, size #0) are prepared containing a 200 mg dose of eplerenone and have the composition shown in Table 11.

**Table 11:**

| **Composition of 200 mg capsules of Example 19** | |
|---|---|
| **Ingredient** | **Amount (mg)** |
| Form L eplerenone | 190.0 |
| Form H eplerenone | 10.0 |
| Lactose, hydrous, NF | 147.8 |
| Microcrystalline cellulose, NF | 29.0 |
| Talc, USP | 10.0 |
| Croscarmellose sodium, NF | 8.0 |
| Sodium lauryl sulfate, NF | 2.0 |
| Colloidal silicon dioxide, NF | 2.0 |
| Magnesium stearate, NF | 1.2 |
| Total capsule fill weight | 400.0 |

### Example 20: Preparation of milled eplerenone

Dried methyl ethyl ketone solvate of eplerenone is first delumped by passing the solvate through a 20 mesh screen on a Fitz mill. The delumped solid is then pin milled using an Alpine Hosakawa stud disk pin mill operating under liquid nitrogen cooling at a feed rate of approximately 250 kg/hour. Pin milling produces milled eplerenone with a D₉₀ particle size of approximately 65-100 µm.

### Example 21: Effect of eplerenone particle size on pharmacokinetic parameters in a dog study

The effect of particle size of Form L eplerenone on eplerenone plasma concentrations and relative bioavailability was studied in a dog model. Four healthy female beagle dogs weighing 8 to 12 kg were intragastrically administered one immediate release capsule (#0, white opaque) containing the formulation described in Table 12 below followed by about 10 ml of water.

**Table 12:**

| **Composition of eplerenone capsule used in Example 21** | | |
|---|---|---|
| Ingredient | Weight % | Amount (mg) |
| Form L eplerenone | 50.00 | 200.00 |
| Lactose, hydrous (Fast-Flo) | 36.95 | 147.80 |
| Microcrystalline cellulose (Avicel™ PH-102) | 7.25 | 29.00 |
| Sodium lauryl sulfate | 0.50 | 2.00 |
| Croscarmellose sodium | 2.00 | 8.00 |
| Talc | 2.50 | 10.00 |
| Colloidal silicon dioxide | 0.50 | 2.00 |
| Magnesium stearate | 0.30 | 1.20 |
| Total | 100.00 | 400.00 |

The dogs were fasted for 15 to 20 hours prior to administration of the capsule and were not fed again until at least 4 hours after dose administration. Blood samples (approximately 3 ml) were collected by venipuncture in chilled tubes containing heparin at 0, 0.5, 1, 2, 3, 4, 6, 8 and 24 hours after dose administration. The blood samples were immediately placed on ice. Separation of plasma from the blood samples was complete after about 15 minutes of centrifugation. The resulting plasma samples were frozen at about -20°C and stored until analyzed. Analysis was performed using an LC/MS/MS procedure.

The same four dogs were used for testing three formulations, each having the composition shown in Table 12 but having different eplerenone particle sizes. The eplerenone starting materials had D₉₀ particle sizes of about 212 µm, about 86 µm and about 36 µm, respectively. A minimum wash-out period of 5 days was allowed between administration of successive formulations. The mean results are reported in Tables 13 and 14 below. Relative bioavailability was calculated from the AUC result, the formulation having D₉₀ of 86 µm being selected as the standard.

**Table 13:**

| **Blood serum eplerenone concentration (µg/ml), Example 21** | | | |
|---|---|---|---|
| **Time (hours)** | **D**_{**90**} **212 µm** | **D**_{**90**} **86 µm** | **D**_{**90**} **36 µm** |
| 0 | 0 | 0 | 0 |
| 0.5 | 1.83 | 3.65 | 1.99 |
| 1 | 2.40 | 6.18 | 5.86 |
| 2 | 3.77 | 6.89 | 6.77 |
| 3 | 2.85 | 5.70 | 6.60 |
| 4 | 2.61 | 4.39 | 5.56 |
| 6 | 1.63 | 3.11 | 3.31 |
| 8 | 1.10 | 1.90 | 2.09 |
| 24 | 0.0252 | 0.032 | 0.0706 |

**Table 14:**

| **Pharmacokinetic (PK) parameters calculated from data of Example 21** | | | |
|---|---|---|---|
| **PK parameter** | **D**_{**90**} **212 µm** | **D**_{**90**} **86 µm** | **D**_{**90**} **36 µm** |
| Cₘₐₓ (µg/ml) | 3.98 | 7.02 | 7.39 |
| Tₘₐₓ (hours) | 1.50 | 1.75 | 2.25 |
| AUC ((µg/ml)hr) | 26.6 | 49.2 | 53.1 |
| Relative bioavailability (%) | 53.25 | 100 | 107.9 |

### Example 22: Effect of eplerenone particle size on pharmacokinetic parameters in a human study

The effect of particle size of Form L eplerenone on eplerenone plasma concentrations and relative bioavailability was studied in a human model, using three pharmaceutical compositions as described in Table 15 below. Subjects received single 100 mg doses of a Form L eplerenone composition as medication on days 1, 8, 15, 22 and 29 according to a randomization schedule. All medication was administered with 180 ml water at 0800 hours. Blood samples for eplerenone pharmacokinetic analyses were collected at -0.5 (pre-dose), 0.5, 1, 2, 3, 4, 6, 8, 10, 12, 16, 24, 36 and 48 hours post-dose.

Plasma concentrations of eplerenone were determined using a validated HPLC method with MS/MS detection. Pharmacokinetic data are reported in Table 16. The particle size distributions of the Form L eplerenone used in preparation of the compositions were determined in the dry powder state using laser light scattering.

**Table 15:**

| **Eplerenone compositions (weight %) used in Example 22** | | | |
|---|---|---|---|
| **Ingredient** | **Capsule A** | **Tablet A** | **Capsule B** |
| Form L eplerenone | | | |
| (D₉₀ 40 µm) | 25 | -- | -- |
| (D₉₀ 82 µm) | -- | 30 | -- |
| (D₉₀ 96 µm) | -- | -- | 25 |
| Lactose monohydrate | -- | 42 | 57.86 |
| Lactose, hydrous | 57.8 | -- | -- |
| Microcrystalline cellulose | | | |
| (Avicel™ PH-101) | 11.4 | 17.5¹ | -- |
| (Avicel™ PH-102) | -- | -- | 11.34 |
| Croscarmellose sodium (Ac-Di-Sol™) | 2 | 5 | 2 |
| HPMC (Pharmacoat™ 603) | -- | 3 | -- |
| Sodium lauryl sulfate | 0.5 | 1 | 0.5 |
| Talc | 2.5 | 1 | 2.5 |
| Magnesium stearate | 0.3 | 0.5 | 0.3 |
| Colloidal silicon dioxide | 0.5 | -- | 0.5 |
| Total | 100 | 100 | 100 |

| | | | |
|---|---|---|---|
| ¹ 7.5% intragranular, 10% extragranular | | | |

**Table 16:**

| **Pharmacokinetic (PK) parameters calculated from data of Example 22** | | | |
|---|---|---|---|
| PK parameter | 100 mg Capsule A (D₉₀ 40 µm) | 100 mg Tablet A (D₉₀ 82 µm) | 100 mg Capsule B (D₉₀ 96 µm) |
| Cₘₐₓ (ng/ml) | 1747 | 1704 | 1669 |
| Tₘₐₓ (hours) | 1.8 | 1.8 | 1.3 |
| AUC ((ng/ml)hr) | 11349 | 11945 | 11981 |

Although this invention has been described with respect to specific embodiments, the details of these embodiments are not to be construed as limitations.

## Claims

1. Eplerenone comprising Form H crystalline eplerenone, wherein said eplerenone has a phase purity from about 90% to about 100% Form H crystalline eplerenone, and wherein said Form H crystalline eplerenone has an orthorhombic crystal system and has an X-ray powder diffraction pattern comprising a peak at 12.0 ± 0.2 degrees 2θ.

2. The eplerenone of Claim 1 wherein said Form H crystalline eplerenone has an X-ray powder diffraction pattern further comprising peaks at 7.0 ± 0.2 and 8.3 ± 0.2 degrees 2θ.

3. The eplerenone of Claim 1 wherein said Form H crystalline eplerenone has an X-ray powder diffraction pattern substantially as shown in Figure 1.

4. The eplerenone of Claim 1 wherein said Form H crystalline eplerenone has a melting point in a range from about 247°C to about 251°C.

5. The eplerenone of Claim 1 wherein said Form H crystalline eplerenone has an IR spectrum comprising a peak at about 1739 cm⁻¹.

6. The eplerenone of Claim 5 wherein said Form H crystalline eplerenone has an IR spectrum comprising a peak at about 1399 cm⁻¹.

7. The eplerenone of Claim 6 wherein said Form H crystalline eplerenone has an IR spectrum comprising a peak at about 1724 cm⁻¹.

8. The eplerenone of Claim 1 wherein said Form H crystalline eplerenone has an IR spectrum substantially as shown in Figure 35.

9. The eplerenone of Claim 1 wherein said Form H crystalline eplerenone has a ¹³C NMR spectrum comprising a carbon resonance at about 24.7 ppm.

10. The eplerenone of Claim 9 wherein said Form H crystalline eplerenone has a ¹³C NMR spectrum comprising carbon resonances at about 24.7 ppm and about 64.8 ppm.

11. The eplerenone of Claim 1 wherein said Form H crystalline eplerenone has a melting point in a range from about 247°C to about 251°C, and an IR spectrum comprising a peak at about 1739 cm⁻¹.

12. The eplerenone of Claim 1 wherein said Form H crystalline eplerenone has a melting point in a range from about 247°C to about 251°C, and an IR spectrum comprising peaks at about 1399 cm⁻¹ and about 1739 cm⁻¹.

13. The eplerenone of Claim 1 wherein said Form H crystalline eplerenone has a melting point in a range from about 247°C to about 251°C, an IR spectrum comprising peaks at about 1399 cm⁻¹ and about 1739 cm⁻¹ and further comprising a ¹³C NMR spectrum comprising a carbon resonance at about 24.7 ppm.

14. The eplerenone of Claim 1 wherein said Form H crystalline eplerenone has a P2₁2₁2₁ space group.

15. The eplerenone of Claim 1 wherein said Form H crystalline eplerenone has unit cell a, b and c values of about 21.22 Å, about 14.50 Å, and about 6.34 Å, respectively.

16. The eplerenone of Claim 1 wherein said Form H crystalline eplerenone has unit cell α, β and γ values that are each about 90°.

17. The eplerenone of Claim 1 wherein said eplerenone is a substantially phase pure form of said Form H crystalline eplerenone.

18. The eplerenone of Claim 1 wherein said eplerenone has a phase purity of at least about 95% Form H crystalline eplerenone.

19. The eplerenone of any one of Claims 1 to 18, further comprising Form L crystalline eplerenone, wherein said Form L crystalline eplerenone has a monoclinic crystal system.

20. The eplerenone of any one of Claims I to 19 further comprising a solvated crystalline form of eplerenone.

21. The eplerenone of Claim 20 wherein said solvated crystalline form of eplerenone is selected from the group consisting of the methyl ethyl ketone, 2-pentanone, acetic acid, acetone, butyl acetate, chloroform, ethanol, isobutanol, isobutyl acetate, methyl acetate, ethyl propionate, n-butanol, n-octanol, n-propanol, isopropanol, propyl acetate, propylene glycol, t-butanol, tetrahydrofuran, toluene and t-butyl acetate crystalline solvates of eplerenone.

22. The eplerenone of Claim 21 wherein said solvated crystalline form of eplerenone is a methyl ethyl ketone crystalline solvate of eplerenone.

23. The eplerenone of any of Claims 1-22 further comprising amorphous eplerenone.

24. The eplerenone of any one of Claims 1 to 23 in a form of particles having a D₉₀ particle size of less than about 400 µm.

25. The eplerenone according to Claim 24 in a form of particles having a D₉₀ particle size of less than about 200 µm.

26. The eplerenone according to Claim 24 in a form of particles having a D₉₀ particle size of less than about 150 µm.

27. The eplerenone according to Claim 24 in a form of particles having a D₉₀ particle size of less than about 100 µm.

28. The eplerenone of any one of Claims 1 to 23 in a form of particles having a D₉₀ particle size of about 25 to about 400 µm.

29. The eplerenone of Claim 28 in a form of particles having a D₉₀ particle size of about 25 to about 200 µm.

30. The eplerenone of Claim 28 in a form of particles having a D₉₀ particle size of about 25 to about 150 µm.

31. The eplerenone of Claim 28 in a form of particles having a D₉₀ particle size of about 25 to about 100 µm.

32. The eplerenone of Claim 28 in a form of particles having a D₉₀ particle size of about 30 to about 50 µm.

33. The eplerenone of any one of Claims 1 to 23 in a form of particles having a D₉₀ particle size of about 50 to about 150 µm.

34. The eplerenone of Claim 33 in a form of particles having a D₉₀ particle size of about 75 to about 125 µm.

35. The eplerenone of Claim 34 in a form of particles having a D₉₀ particle size of about 40 to about 100 µm.

36. The eplerenone of any one of Claims 1 to 23 in a form of particles having a D₉₀ particle size of less than about 15 µm.

37. The eplerenone of Claim 36 in a form of particles having a D₉₀ particle size of less than about 10 µm.

38. The eplerenone of Claim 36 in a form of particles having a D₉₀ particle size of less than about 1 µm.

39. The eplerenone of any one of Claims 1 to 38 further comprising one or more Form H crystal growth promoters.

40. The eplerenone of any one of Claims 1 to 38 further comprising one or more compounds that are substantially crystallographically isostructural to said Form H crystalline eplerenone.

41. The eplerenone of any one of Claims 1 to 38, further comprising one or more compounds selected from the group consisting of (a) 7-methyl hydrogen *4α,5α,9α,11α*-diepoxy-17-hydroxy-3-oxo-17α-pregnane-7α,21-dicarboxylate, γ-lactone; (b) 7-methyl hydrogen 11α, 12α-epoxy-17-hydroxy-3-oxo-17α-pregn-4-ene-7α,21-dicarboxylate, γ-lactone; and (c) 7-methyl hydrogen 17-hydroxy-3-oxo-17α-pregna-4,9(11)-diene-7α,21-dicarboxylate, γ-lactone.

42. The eplerenone of Claim 41 wherein said eplerenone comprises at least about 0.5% by weight of one or more of said compounds.

43. The eplerenone of Claim 42 wherein said eplerenone comprises at least about 1 % by weight of one or more of said compounds.

44. The eplerenone of Claim 42 wherein said eplerenone comprises at least about 2% by weight of one or more of said compounds.

45. A pharmaceutical composition comprising a therapeutically-effective amount of the form of any one of Claims 1 to 44 in association with at least one pharmaceutically-acceptable carrier, adjuvant or diluent.

46. The pharmaceutical composition of Claim 45 comprising eplerenone in an amount from about 10 to about 1000 mg.

47. The composition of Claim 46 wherein said amount is from about 25 to about 200 mg.

48. The composition of Claim 46 or Claim 47 wherein said eplerenone is in a form of particles having a D₉₀ particle size of less than about 400 µm.

49. The composition of Claim 48 wherein said eplerenone is in a form of particles having a D₉₀ particle size of less than about 200 µm.

50. The composition of Claim 48 wherein said eplerenone is in a form of particles having a D₉₀ particle size of less than about 150 µm.

51. The composition of Claim 48 wherein said eplerenone is in a form of particles having a D₉₀ particle size of less than about 100 µm.

52. The composition of Claim 46 or Claim 47 wherein said eplerenone is in a form of particles having a D₉₀ particle size of about 25 to about 400 µm.

53. The composition of Claim 52 wherein said eplerenone is in a form of particles having a D₉₀ particle size of about 25 to about 200 µm.

54. The composition of Claim 52 wherein said eplerenone is in a form of particles having a D₉₀ particle size of about 25 to about 150 µm.

55. The composition of Claim 52 wherein the eplerenone is in a form of particles having a D₉₀ particle size of about 25 to about 100 µm.

56. The composition of Claim 52 wherein said eplerenone is in a form of particles having a D₉₀ particle size of about 30 to about 50 µm.

57. The composition of Claim 52 wherein said eplerenone is in a form of particles having a D₉₀ particle size of about 50 to about 150 µm.

58. The composition of Claim 52 wherein said eplerenone is in a form of particles having a D₉₀ particle size of about 75 to about 125 µm.

59. The composition of Claim 46 or Claim 47 wherein said eplerenone is in a form of particles having a D₉₀ particle size of less than about 15 µm.

60. The composition of Claim 59 wherein said eplerenone is in a form of particles having a D₉₀ particle size of less than about 10 µm.

61. The composition of Claim 59 wherein said eplerenone is in a form of particles having a D₉₀ particle size of less than about 1 µm.

62. Use of the composition of Claim 46 or Claim 47 in the manufacture of a medicament for the treatment or prevention of an aldosterone-mediated condition or disorder.

63. The use of Claim 62 wherein the condition or disorder is selected from the group consisting of hypertension, heart failure, cirrhosis of the liver, excess collagen, fibrosis, benign prostate hypertrophy and depression.

64. The use of Claim 62 wherein the condition or disorder is hypertension.

65. The use of Claim 62 wherein the condition or disorder is heart failure.

66. An eplerenone form according to any one of Claims 1-44 substantially as herein described.

67. An eplerenone or an eplerenone form according to any one of Claims 1-44 substantially as herein described with reference to any one of Examples 1 to 14 and/or Figures 1 to 91.

68. An eplerenone or an eplerenone form substantially as herein described with reference to any one of Examples 6, 7C, 7G, 9, 12 and/or Figures 1,18, 35, 52 and 86-88.

69. A pharmaceutical composition according to any one of Claims 45-61 substantially as herein described.

70. A pharmaceutical composition according to any one of Claims 45-61 substantially as herein described with reference to any one of Examples 1 to 20 and/or Figures 1 to 91.

71. A pharmaceutical composition substantially as herein described with reference to any one of Examples 6, 7C, 7G, 9 and 12 and/or Figures 1, 18, 35, 52 and 86-88.

72. Use according to any one of Claims 62-65 substantially as herein described.

73. Use according to any one of Claims 62-65 substantially as herein described with reference to any one of Examples 1 to 22 and/or Figures 1 to 91.
